# EUROPEAN PATENT APPLICATION

(11) **EP 4 840 887 A1**
(43) Date of publication of application: **18.11.2026**
(21) Application number: 24916658.8
(22) Date of filing: 13.12.2024
(51) Int. Cl.: C07K 19/00, C12N 15/62, A61K 38/17, A61K 39/00, A61P 35/00

(54) **GENETIC ENGINEERING DELIVERY AND USE OF TUMOR ANTIGEN PEPTIDE FUSION PROTEIN AND COMPOSITION THEREOF**

(30) Priority: 12.01.2024 CN 202410053312; 06.02.2024 WO PCT/CN2024/076404
(71) Applicant: Xinniu (Shanghai) Biotechnology Co., Ltd., Shanghai 201100 (CN)
(72) Inventor: HOU, Weizhou, Shanghai 201100 (CN)
(74) Representative: ip21 Ltd
(86) International application number: PCT/CN2024/139143
(87) International publication number: WO 2025/148611

(57) **Abstract**

The present invention relates to genetic engineering delivery and use of a tumor antigen peptide fusion protein and a composition thereof. A tumor antigen peptide fusion protein and a composition thereof have immunogenicity, therapeutic efficacy, and specificity.

## Description

### Technical Field

The present disclosure belongs to the fields of biomedicine and tumor immunology, and provides the genetically engineered delivery and use of a tumor antigen peptide fusion protein and a composition thereof.

### Background Art

Cancer vaccines are categorized into prophylactic and therapeutic types. For cancer-free individuals, vaccination with cancer vaccines can prevent tumorigenesis by activating the ability of the immune system to kill cancer cells. For patients with existing cancer, vaccination with cancer vaccines can activate the immune system to kill cancer cells for the treatment of cancer. Vaccination is one of the effective measures to prevent and treat cancer. However, therapeutic cancer vaccines still encounter many obstacles to maximizing their efficacy, including lack of specific tumor antigens, low immunogenicity of antigens, and poor delivery efficiency.

Therapeutic cancer vaccines are based on tumor antigens and elicit the body's immune response to cancer cells. Tumor-specific antigens are antigens unique to tumor cells. As major histocompatibility complex (MHC)-associated tumor antigens, they can elicit an immune response. In general, tumor-specific antigens are due to acquired genetic variations and are considered to be most prevalent in highly mutated cancers such as melanoma and lung cancer. They can kill cancer cells in the tumor microenvironment with precision through an immune response without the side effects of off-target killing of normal cells. Cancer-testis antigens (CTAs), also known as cancer germline antigens, belong to tumor-specific antigens, and include more than 200 proteins expressed in germline tissues such as testis, ovary, and placenta, expression of which has the following characteristics: (1) they are expressed at different frequencies in various tumor tissues; (2) most CTAs are located on the X chromosome; (3) they usually exist as multiple family members; and (4) the expression of CTAs is often heterogeneous in tumor tissues of diverse origins. Tumor cells create an inhibitory immune microenvironment that promotes the escape of tumor cells from the immune system. Therefore, such tumor antigens can be exploited to develop more precise and effective immunotherapies, making them ideal targets for tumor immunotherapy. Sipuleucel-T (trade name: Provenge; Chinese name: Puliewei), a prostate cancer vaccine, is an autologous cellular immunotherapy based on antigen-presenting cells, and is also the first cellular immunotherapeutic drug for prostate cancer approved by the U.S. Food and Drug Administration (FDA). The median survival of patients in the Sipuleucel-T treatment group was increased by 4.1 months. Although the survival time was extended by several months, considerable efforts are still required to achieve the cure of the cancer.

NAD⁺-dependent 15-hydroxyprostaglandin dehydrogenase (HPGD) is a key enzyme responsible for prostaglandin inactivation. It is widely involved in regulating a variety of cellular pathways such as inflammation, differentiation, and signal transduction, and serves as an important target for pharmacological intervention. In addition, HPGD is ubiquitously expressed in mammalian tissues and localized in the cytoplasm. By degrading prostaglandins (tumor-promoting molecules) in cancer cells, it can inhibit cancer cell proliferation and induce cancer cell differentiation, and HPGD knockout or low expression can increase susceptibility to colon tumors and the spread of pancreatic tumor cells. HPGD is shown to be a tumor suppressor gene and a potential target molecule for cancer control.

Poxviruses are large, structurally complex DNA viruses with a wide variety of species. Among them, orthopoxviruses are the main human pathogens, such as variola virus, vaccinia virus, cowpox virus, monkeypox virus, etc. Poxvirus vectors can express exogenous proteins, which are closer to the native structure and exhibit superior stability as compared to those expressed in bacteria and yeast. Among them, vaccinia virus has been used as a vaccine to prevent smallpox and has the following characteristics: (1) it has a broad host range and can infect mammalian and avian cells; (2) it possesses a large genome and allows the insertion of exogenous protein gene fragments; (3) the virus replicates in the cytoplasm of host cells and does not integrate into the genes of host cells; and (4) some vaccinia virus strains can be used for the development of oncolytic viruses. Therefore, vaccinia virus is an ideal vector for the expression of eukaryotic genes or exogenous viral genes. At present, more common vaccinia virus strains mainly include the Western Reserve (WR) strain, Copenhagen strain, Lister strain, Ankara strain, Modified Vaccinia Ankara strain, New York Vaccinia strain, Tiantan strain, Dryvax strain, Bern strain, Paris strain, Tash Kent strain, IHD-J strain, IHD-W strain, Brighton strain, CVA382 strain, Dairen strain, LC16m8 strain, LC16M0 strain, LIVP strain, ACAM2000 strain, WR65-16 strain, Connaught strain, and EM-63 strain.

Based on the above background, the present disclosure aims to provide a genetically engineered delivery method and use of a tumor antigen peptide fusion protein and a composition thereof.

### Summary of the Invention

An aspect of the present disclosure provides a tumor antigen peptide fusion protein, comprising a signal peptide, human 15-hydroxyprostaglandin dehydrogenase (HPGD), and optionally at least one tumor antigen peptide.

A second aspect of the present disclosure provides a composition, comprising the tumor antigen peptide fusion protein of the present disclosure as described above and a therapeutic agent. The therapeutic agent comprises a therapeutic agent fusion protein, comprising a therapeutic agent and at least one peptide selected from a signal peptide, a transmembrane peptide, and a signal-anchor peptide, wherein the signal peptide, the transmembrane peptide, and/or the signal-anchor peptide are located at the N-terminus or C-terminus of the therapeutic agent fusion protein.

In some embodiments, the composition comprises the tumor antigen peptide fusion protein of the present disclosure as described above and an IL12 p70 fusion protein, wherein the IL12 p70 fusion protein comprises, from the N-terminus to the C-terminus: a signal peptide or signal-anchor peptide, an IL12 p70 protein, and optionally a transmembrane peptide.

A third aspect of the present disclosure provides a nucleic acid construct encoding the tumor antigen peptide fusion protein of the present disclosure as described above, or the composition of the present disclosure as described above.

A fourth aspect of the present disclosure provides a genetically engineered recombinant virus, comprising the nucleic acid construct of the present disclosure as described above.

A fifth aspect of the present disclosure provides an expression system, comprising a host cell and the genetically engineered recombinant virus of the present disclosure as described above.

A sixth aspect of the present disclosure provides a pharmaceutical composition, comprising the tumor antigen peptide fusion protein, composition, nucleic acid construct, or genetically engineered recombinant virus of the present disclosure as described above; and a pharmaceutically acceptable carrier.

A seventh aspect of the present disclosure provides a method for treating or preventing a tumor, comprising administering the tumor antigen peptide fusion protein, composition, nucleic acid construct, genetically engineered recombinant virus, or pharmaceutical composition of the present disclosure as described above.

An eighth aspect of the present disclosure provides the use of the tumor antigen peptide fusion protein, composition, nucleic acid construct, genetically engineered recombinant virus, or pharmaceutical composition in the manufacture of a medicament for treating or preventing a tumor.

A ninth aspect of the present disclosure provides a method for activating or enhancing the function of immune cells, comprising administering the tumor antigen peptide fusion protein, composition, nucleic acid construct, genetically engineered recombinant virus, or pharmaceutical composition of the present disclosure.

The tumor antigen peptide fusion protein, composition, nucleic acid construct, genetically engineered recombinant virus, or pharmaceutical composition of the present disclosure has the following features:
(1) Therapeutic effects: Vaccinia virus exerts a direct lytic effect on tumor cells, and simultaneously activates inflammatory responses to indirectly enhance anti-tumor immunity, thereby exerting an immunopotentiating effect on cancer patients and activating/enhancing the immune system to enter an immune response state. The expression and release of the tumor antigen peptide fusion protein induce the proliferation of tumor-associated specific immune cells, degrade prostaglandin E2, reduce suppressors that inhibit anti-tumor immunity, and enhance the clearance of tumor cells by immune cells.
(2) Vaccine properties: The host may be induced to generate an anti-tumor immune response. Firstly, vaccinia virus itself can trigger a strong anti-viral immune response in the host. Secondly, while the host generates a strong immune response against vaccinia virus, the release of the tumor antigen peptide fusion protein induces a strong anti-tumor immune response and the proliferation of a large number of tumor-associated specific immune cells, thus achieving the effects of treating and preventing cancer.
(3) High specificity and low side effects: Target cells are selectively infected, and a tumor-specific immune response is induced. The proliferated specific immune cells are unable to recognize or attack normal cells. The present disclosure has the advantages of strong immunogenicity, high tumor antigen specificity, mild side effects, and high safety.

### Brief Description of the Drawings

The present disclosure may be more fully understood by reference to the following drawings.

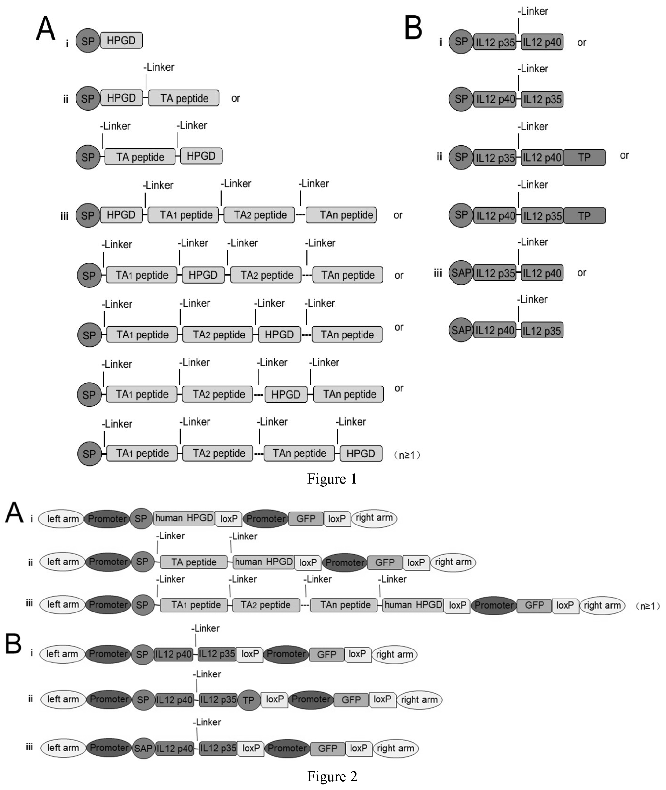

Figure 1 shows schematic diagrams of tumor antigen peptide fusion proteins (A) and IL12 p70 fusion proteins (B). SP denotes signal peptide, TA peptide denotes tumor antigen peptide (TA₁ peptide, TA₂ peptide, ..., and TAₙ peptide may be the same or different), Linker denotes linker peptide, TP denotes transmembrane peptide, and SAP denotes signal-anchor peptide.

Figure 2 shows schematic diagrams of shuttle plasmids. Figure 2A shows a schematic diagram of shuttle plasmids for secreted fusion proteins of tumor antigen peptides, wherein Figure 2Ai shows promoter-driven secreted human HPGD (sHPGD) and green fluorescent protein (GFP); Figure 2Aii shows promoter-driven secreted fusion protein of tumor antigen peptide and human HPGD (hHPGD), i.e., secreted TA peptide + hHPGD (sTAH), and GFP; and Figure 2Aiii shows promoter-driven secreted fusion protein of multiple tumor antigen peptides and human HPGD, i.e., secreted multiple TA peptides + hHPGD (smTAH), and GFP. Figure 2B shows a shuttle plasmid for IL12 p70 fusion proteins, wherein Figure 2Bi shows promoter-driven secreted IL12 p70 (sIL12) and GFP; Figure 2Bii shows promoter-driven type I membrane protein IL12 p70 (mIL12 I) and GFP; and Figure 2Biii shows promoter-driven type II membrane protein IL12 p70 (mIL12 II) and GFP.

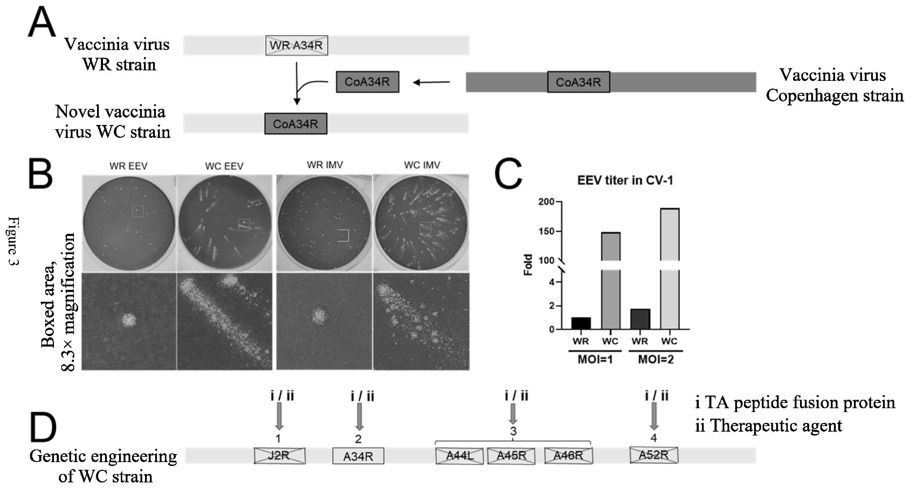

Figure 3 shows the genetic engineering of vaccinia virus. Figure 3A shows a schematic diagram of the construction of the vaccinia virus WC strain, which uses the Western Reserve (WR) strain as the backbone, and replaces the A34R gene sequence of the WR strain (WR A34R) with the A34R gene sequence of the Copenhagen strain (CoA34R). Figure 3B shows the comparison of the plaque morphology of extracellular enveloped viruses (EEVs) and intracellular mature viruses (IMVs) between the WR strain and the WC strain in CV-1 cells. Figure 3C shows the comparison of the EEV titer fold between the WR strain and the WC strain, and the data shown in the figure represent the mean of the results of three experiments. Figure 3D shows the genetic engineering using the vaccinia virus WC strain as the vector, including modifications of six gene sequences: Sequence 1 (J2R), Sequence 2 (A34R), Sequences 3-5 (A44L-A45R-A46R), and Sequence 6 (A52R), as well as knock-in of heterologous nucleic acids encoding tumor antigen peptide fusion proteins and/or therapeutic agents.

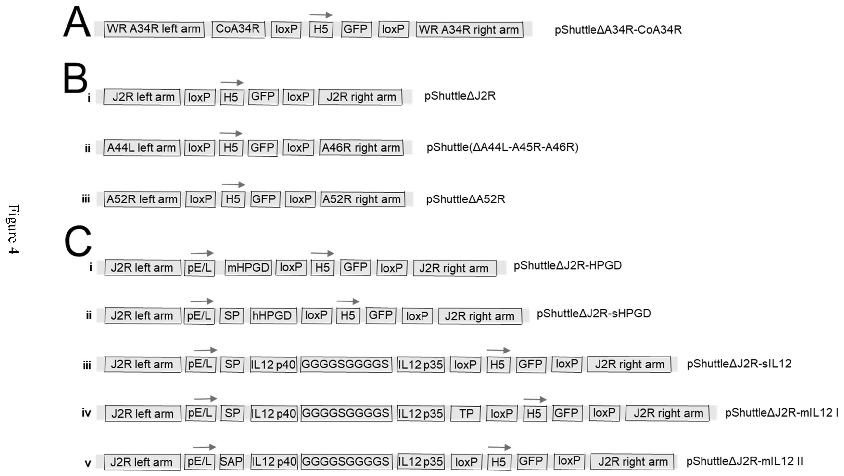

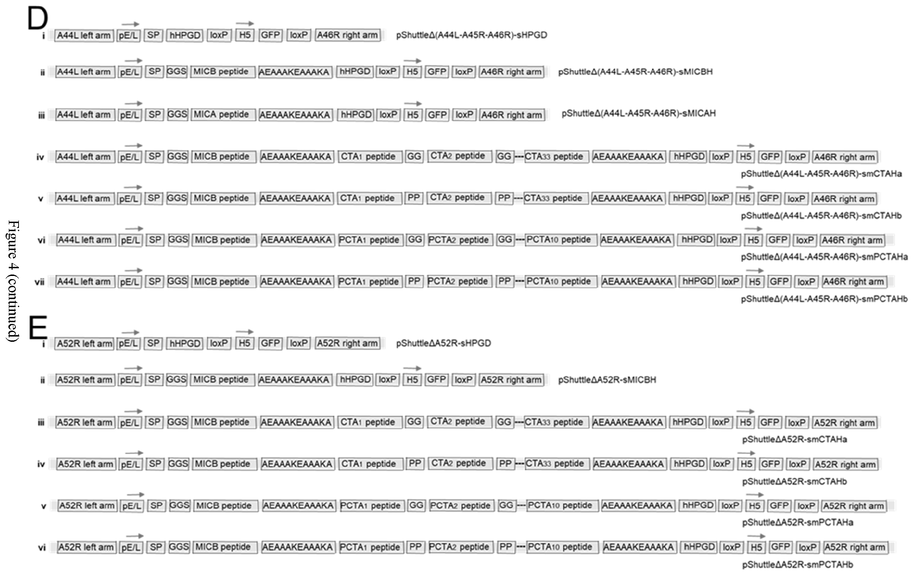

Figure 4 shows shuttle plasmids for constructing genetically engineered recombinant viruses using the novel vaccinia virus WC strain as the vector, wherein Figure 4A shows a shuttle plasmid used for knock-in of heterologous nucleic acid of the Copenhagen strain A34R (CoA34R); Figure 4B shows shuttle plasmids used for gene knockout of J2R (Figure 4Bi), A44L-A45R-A46R (Figure 4Bii), and A52R (Figure 4Biii); Figure 4C shows shuttle plasmids used for simultaneous gene knockout of J2R and knock-in of heterologous nucleic acids encoding mouse HPGD (mHPGD) (Figure 4Ci), secreted human HPGD (sHPGD; Figure 4Cii), sIL12 (Figure 4Ciii), mIL12 I (Figure 4Civ), and mIL12 II (Figure 4Cv); Figure 4D shows shuttle plasmids used for simultaneous gene knockout of A44L-A45R-A46R and knock-in of heterologous nucleic acids encoding sHPGD (Figure 4Di), secreted fusion protein of MHC class I polypeptide-related sequence B (MICB) peptide and human HPGD (hHPGD) (secreted MICB peptide + hHPGD, sMICBH; Figure 4Dii), secreted fusion protein of MHC class I polypeptide-related sequence A (MICA) peptide and hHPGD (secreted MICA peptide + hHPGD, sMICAH; Figure 4Diii), secreted fusion protein of MICB peptide, multiple cancer-testis antigen (CTA) peptides, and hHPGD (secreted MICB peptide + multiple CTA peptides + hHPGD, smCTAH; Figures 4Div-v), and secreted fusion protein of MICB peptide, multiple prostate carcinoma tumor antigen (prostate carcinoma TA, PCTA) peptides, and hHPGD (secreted MICB peptide + multiple PCTA peptides + hHPGD, smPCTAH; Figures 4Dvi-vii); and Figure 4E shows shuttle plasmids used for simultaneous gene knockout of A52R and knock-in of heterologous nucleic acids encoding sHPGD (Figure 4Ei), sMICBH (Figure 4Eii), smCTAH (Figures 4Eiii-iv), and smPCTAH (Figures 4Ev-vi).

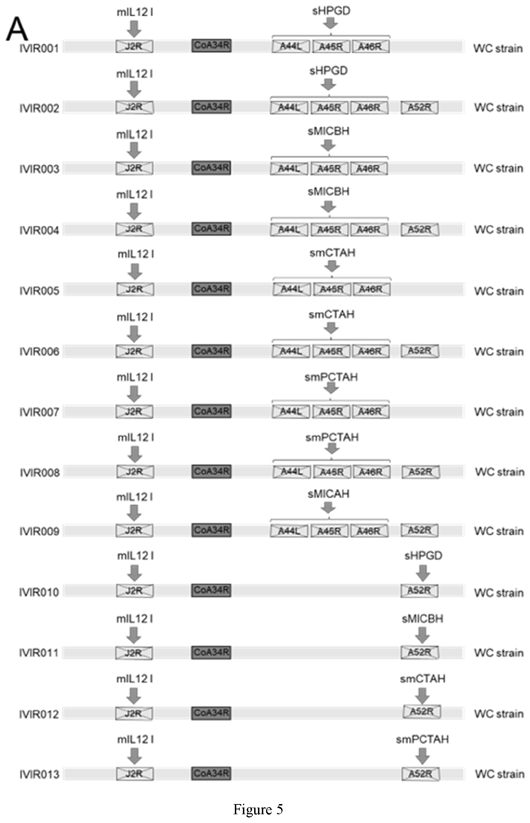

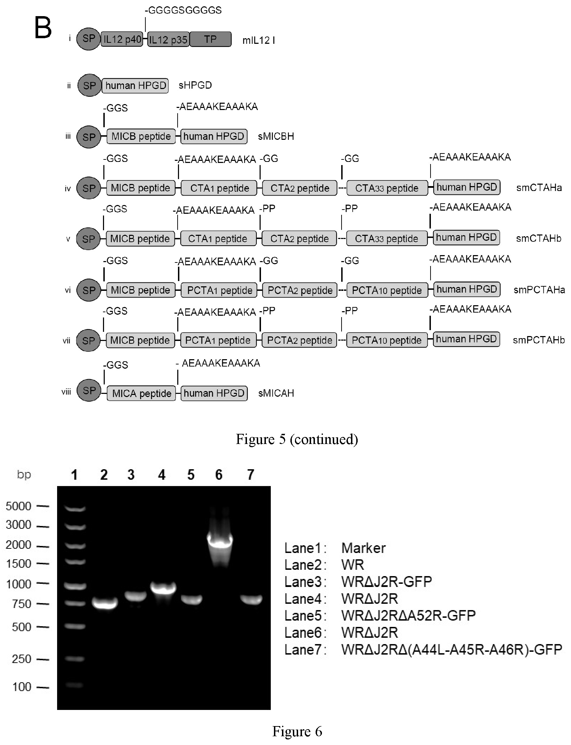

Figure 5 shows genetically engineered recombinant viruses of fused tumor antigen peptides.

Figure 5A shows genetically engineered recombinant viruses IVIR001 to IVIR013 using the novel vaccinia virus WC strain as the vector, respectively, wherein IVIR001, IVIR002, and IVIR010 express type I membrane protein IL12 p70 (mIL12 I) and secreted human HPGD (sHPGD); IVIR003, IVIR004, and IVIR011 express mIL12 I and secreted fusion protein of tumor antigen MICB peptide and hHPGD (sMICBH); IVIR005, IVIR006, and IVIR012 express mIL12 I and secreted fusion protein of tumor antigen MICB peptide, multiple cancer-testis antigen peptides, and hHPGD (smCTAH); IVIR007, IVIR008, and IVIR013 express mIL12 I and secreted fusion protein of tumor antigen MICB peptide, multiple prostate carcinoma tumor antigen peptides, and hHPGD (smPCTAH); and IVIR009 expresses mIL12 I and secreted fusion protein of tumor antigen MICA peptide and hHPGD (sMICAH).

Figure 5B shows fusion proteins delivered by recombinant viruses IVIR001 to IVIR013, respectively, wherein Figure 5Bi shows mIL12 I (type I membrane protein IL12 p70), comprising signal peptide, IL12 p40, (G₄S)₂ linker peptide, IL12 p35, and transmembrane peptide (TP); Figure 5Bii shows sHPGD, comprising signal peptide and human HPGD (hHPGD); Figure 5Biii shows sMICBH, comprising signal peptide, G₂S linker peptide, MICB peptide, AEAAAKEAAAKA linker peptide, and hHPGD; Figure 5Biv shows smCTAHa, comprising signal peptide, G₂S linker peptide, MICB peptide, AEAAAKEAAAKA linker peptide, CTA₁ peptide (cancer-testis antigen peptide 1), CTA₂ peptide, ..., CTA₃₃ peptide, AEAAAKEAAAKA linker peptide, and hHPGD, with GG as the linker peptide between CTA peptides; Figure 5Bv shows smCTAHb, comprising signal peptide, G₂S linker peptide, MICB peptide, AEAAAKEAAAKA linker peptide, CTA₁ peptide (cancer-testis antigen peptide 1), CTA₂ peptide, ..., CTA₃₃ peptide, AEAAAKEAAAKA linker peptide, and hHPGD, with PP as the linker peptide between CTA peptides; Figure 5Bvi shows smPCTAHa, comprising signal peptide, G₂S linker peptide, MICB peptide, AEAAAKEAAAKA linker peptide, PCTA₁ peptide (prostate carcinoma tumor antigen peptide 1), PCTA₂ peptide, ..., PCTA₁₀ peptide, AEAAAKEAAAKA linker peptide, and hHPGD, with GG as the linker peptide between PCTA peptides; Figure 5Bvii shows smPCTAHb, comprising signal peptide, G₂S linker peptide, MICB peptide, AEAAAKEAAAKA linker peptide, PCTA₁ peptide (prostate carcinoma tumor antigen peptide 1), PCTA₂ peptide, ..., PCTA₁₀ peptide, AEAAAKEAAAKA linker peptide, and hHPGD, with PP as the linker peptide between PCTA peptides; and Figure 5Bviii shows sMICAH, comprising signal peptide, G₂S linker peptide, MICA peptide, AEAAAKEAAAKA linker peptide, and hHPGD.

Figure 6 shows the PCR identification results of vaccinia virus gene knockout. Δ indicates gene knockout.

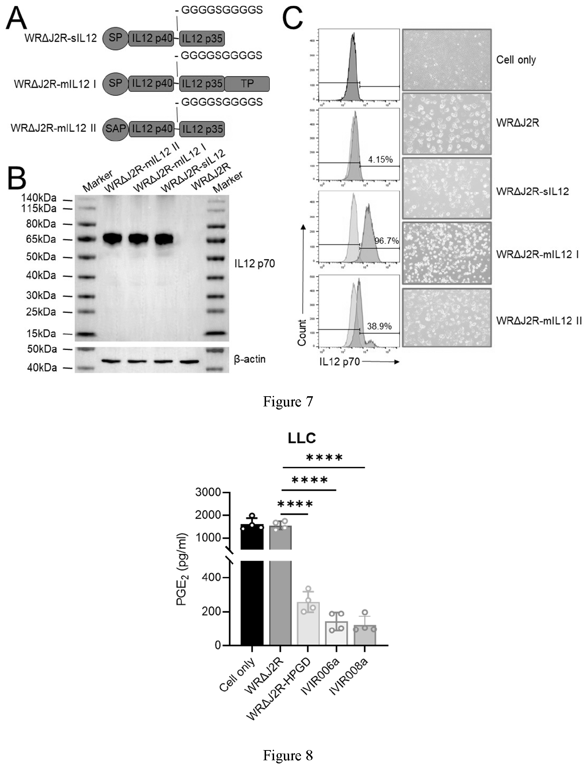

Figure 7 shows the verification of IL12 p70 expression after CV-1 cells are infected with recombinant viruses for 16 h. Figure 7A shows a schematic diagram of IL12 p70 fusion proteins expressed by recombinant viruses WRΔJ2R-sIL12, WRΔJ2R-mIL12 I, and WRΔJ2R-mIL12 II, wherein sIL12 denotes secreted IL12 p70, mIL12 I denotes type I membrane protein IL12 p70, mIL12 II denotes type II membrane protein IL12 p70, SP denotes the secretory signal peptide of sIL12, TP denotes the transmembrane peptide of mIL12 I, and SAP denotes the signal-anchor peptide of mIL12 II. Figure 7B shows the WB results of non-extracellular (intracellular and membrane-bound) IL12 p70 and the internal reference protein β-actin in the infection groups of recombinant viruses WRΔJ2R-sIL12, WRΔJ2R-mIL12 I, WRΔJ2R-mIL12 II, and control virus WRΔJ2R (J2R-knockout WR strain). Figure 7C shows the flow cytometry results of IL12 p70 on the cell membrane in the infection groups of recombinant viruses WRΔJ2R-sIL12, WRΔJ2R-mIL12 I, WRΔJ2R-mIL12 II, and WRΔJ2R, and the virus-uninfected group (Cell only group).

Figure 8 shows the in vitro functional validation of intracellular mouse HPGD and secreted human HPGD expressed by recombinant viruses. The concentration of prostaglandin E2 (PGE₂) in the culture medium after LLC cells were respectively infected with recombinant viruses IVIR006a, IVIR008a, control virus WRΔJ2R (J2R-knockout WR strain), and WRΔJ2R-HPGD (WR strain recombinant virus with J2R knockout and mHPGD knock-in) for 24 h was determined with an ELISA kit. IVIR006a expresses secreted fusion protein of MICB peptide, multiple cancer-testis antigen peptides, and human HPGD (secreted MICB peptide + multiple CTA peptides + hHPGD, smCTAH), and type I membrane protein IL12 p70 (mIL12 I), and IVIR008a expresses secreted fusion protein of MICB peptide, multiple prostate carcinoma tumor antigen peptides, and human HPGD (secreted MICB peptide + multiple PCTA peptides + hHPGD, smPCTAH), and mIL12 I. The data shown in the figure represent the mean ± standard deviation (SD), and the error bars represent ± SD. **** WRΔJ2R vs. WRΔJ2R-HPGD, p-value < 0.0001; **** WRΔJ2R vs. IVIR006a, p-value < 0.0001; and **** WRΔJ2R vs. IVIR008a, p-value < 0.0001.

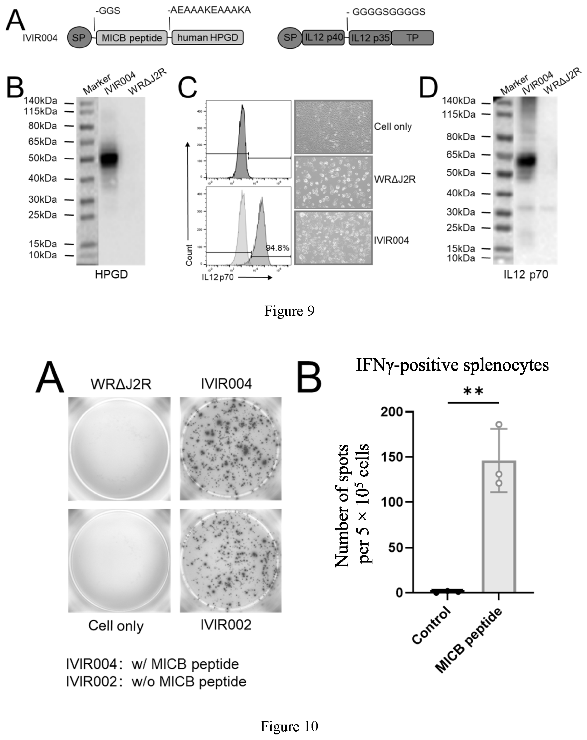

Figure 9 shows the verification of fusion protein expression after CV-1 cells were infected with WC strain recombinant virus IVIR004 delivering tumor antigen MICB peptide fusion proteins for 16 h. Figure 9A shows a schematic diagram of fusion proteins expressed by WC strain recombinant virus IVIR004, which expresses secreted fusion protein of tumor antigen MICB peptide and human HPGD (secreted MICB peptide + hHPGD, sMICBH) and type I membrane protein IL12 p70 (mIL12 I), with the J2R-knockout WR strain (WRΔJ2R) as its control virus. Figure 9B shows the WB results of non-extracellular (intracellular and membrane-bound) HPGD in the infection groups of IVIR004 and control virus WRΔJ2R. Figure 9C shows the flow cytometry results of IL12 p70 expression on the cell membrane in the infection groups of IVIR004 and WRΔJ2R, and the virus-uninfected group (Cell only group). Figure 9D shows the WB results of non-extracellular (intracellular and membrane-bound) IL12 p70 in the infection groups of IVIR004 and WRΔJ2R.

Figure 10 shows the IFNγ ELISpot results of splenocytes from 6-8 week-old female Balb/c mice 14 days after intraperitoneal injection of WC strain recombinant viruses. Figure 10A shows the IFNγ spot images of splenocytes from mice in the infection groups of IVIR002 (not expressing MICB peptide, w/o MICB peptide), IVIR004 (expressing MICB peptide, w/ MICB peptide), and WRΔJ2R (J2R-knockout WR strain), and the virus-uninfected group (Cell only group). Figure 10B shows the number of MICB peptide-specific IFNγ spots (each spot represents one immune cell capable of secreting IFNγ). In the bar chart, the data shown for MICB peptide represent the spot number of the IVIR004 infection group minus the spot number of the IVIR002 infection group in Figure 10A, and the data shown for Control represent the spot number of the WRΔJ2R infection group minus the spot number of the Cell only group in Figure 10A. The data shown in Figure 10B represent the mean ± SD, and the error bars represent ± SD. ** MICB peptide vs. Control, P < 0.01.

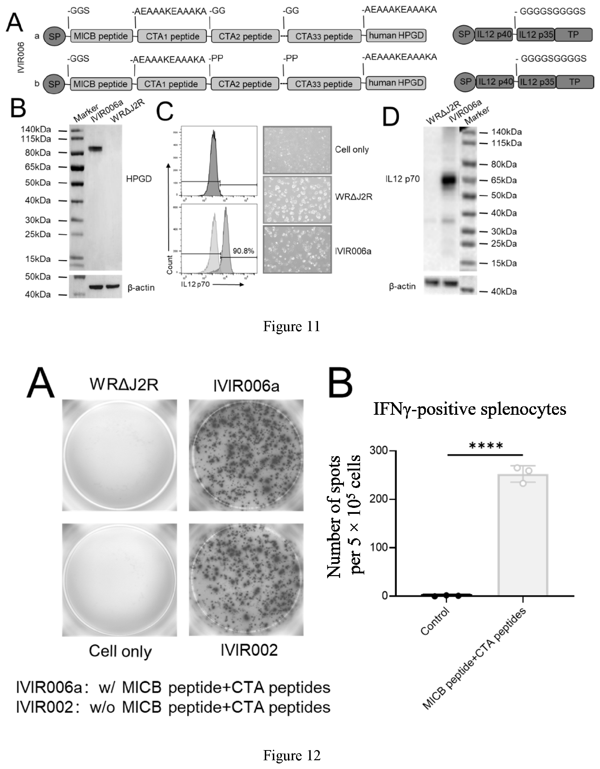

Figure 11 shows the verification of fusion protein expression after CV-1 cells are infected with the WC strain recombinant virus delivering fusion proteins of multiple cancer-testis antigen peptides for 16 h. Figure 11A shows a schematic diagram of fusion proteins expressed by WC strain recombinant virus IVIR006, including two subtypes, a and b. The commonality between the two subtypes is that both express secreted fusion protein of MICB peptide, multiple cancer-testis antigen peptides, and human HPGD (secreted MICB peptide + multiple CTA peptides + hHPGD, smCTAH), and type I membrane protein IL12 p70 (mIL12 I); and the difference lies in the distinct linker peptides between CTA peptides, wherein GG linker peptide is used in IVIR006a, and PP linker peptide is used in IVIR006b. Figure 11B shows the WB results of non-extracellular (intracellular and membrane-bound) HPGD and the internal reference protein β-actin in the infection groups of IVIR006a and its control virus J2R-knockout WR strain (WRΔJ2R). Figure 11C shows the flow cytometry results of IL12 p70 expression on the cell membrane in the infection groups of IVIR006a and WRΔJ2R, and the virus-uninfected group (Cell only group). Figure 11D shows the WB results of non-extracellular (intracellular and membrane-bound) IL12 p70 expression in each group.

Figure 12 shows the IFNγ ELISpot results of splenocytes from 6-8 week-old female C57BL/6N mice 14 days after intraperitoneal injection of WC strain recombinant viruses, including WC strain recombinant viruses IVIR006a (expressing MICB peptide and CTA peptides, w/ MICB peptide + CTA peptides), IVIR002 (not expressing MICB peptide and CTA peptides, w/o MICB peptide + CTA peptides), and control virus WRΔJ2R (J2R-knockout WR strain). Figure 12A shows the IFNγ spot images of splenocytes from mice in each group. Figure 12B shows the number of MICB peptide- and CTA peptide-specific IFNγ spots (each spot represents one immune cell capable of secreting IFNγ). In the bar chart, the data shown for MICB peptide + CTA peptides represent the spot number of the IVIR006a injection group minus the spot number of the IVIR002 injection group in Figure 12A, and the data shown for Control represent the spot number of the WRΔJ2R injection group minus the spot number of the Cell only group in Figure 12A. The data shown in the figure represent the mean ± SD, and the error bars represent ± SD. **** MICB peptide + CTA peptides vs. Control, p-value < 0.0001.

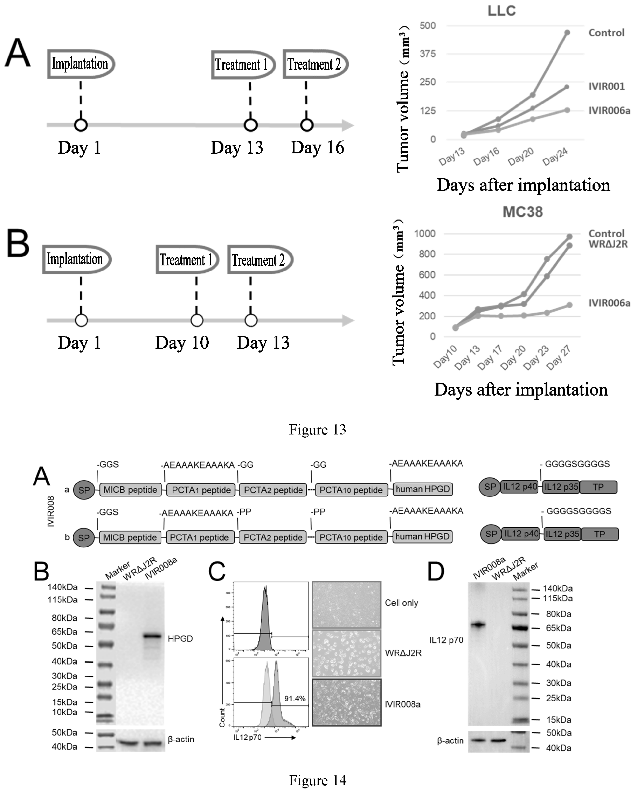

Figure 13 shows the tumor growth curves of lung cancer and colon cancer in mice after treatment with WC strain recombinant viruses delivering fusion proteins of multiple cancer-testis antigen peptides. Figure 13A shows the tumor growth curves of LLC lung cancer cells in mice of the Control group, the IVIR001 treatment group, and the IVIR006a treatment group. Figure 13B shows the tumor growth curves of MC38 colon cancer cells in mice of the Control group, the control virus WRΔJ2R (J2R-knockout WR strain) treatment group, and the IVIR006a treatment group. IVIR001 is a WC strain recombinant virus expressing secreted human HPGD (sHPGD) and type I membrane protein IL12 p70 (mIL12 I), and IVIR006a is a WC strain recombinant virus expressing secreted fusion protein of MICB peptide, multiple cancer-testis antigen peptides, and human HPGD (secreted MICB peptide + multiple CTA peptides + hHPGD, smCTAH), and mIL12 I. Each experimental group contains 9-10 mice, and the data shown in the figure represent the mean tumor volume of each group.

Figure 14 shows the verification of fusion protein expression after CV-1 cells are infected with the WC strain recombinant virus delivering fusion proteins of multiple prostate carcinoma tumor antigen peptides for 16 h. Figure 14A shows a schematic diagram of fusion proteins expressed by WC strain recombinant virus IVIR008, including two subtypes, a and b. The commonality between the two subtypes is that both express secreted fusion protein of MICB peptide, multiple prostate carcinoma tumor antigen peptides, and human HPGD (secreted MICB peptide + multiple PCTA peptides + hHPGD, smPCTAH), and type I membrane protein IL12 p70 (mIL12 I); and the difference lies in the distinct linker peptides between one PCTA peptide and another PCTA peptide, wherein GG linker peptide is used in IVIR008a, and PP linker peptide is used in IVIR008b. Figure 14B shows the WB results of non-extracellular (intracellular and membrane-bound) HPGD and the internal reference protein β-actin in the infection groups of IVIR008a and control virus WRΔJ2R (J2R-knockout WR strain). Figure 14C shows the flow cytometry results of IL12 p70 expression on the cell membrane in the infection groups of IVIR008a and WRΔJ2R, and the virus-uninfected group (Cell only group). Figure 14D shows the WB results of non-extracellular (intracellular and membrane-bound) IL12 p70 expression in the infection groups of IVIR008a and WRΔJ2R.

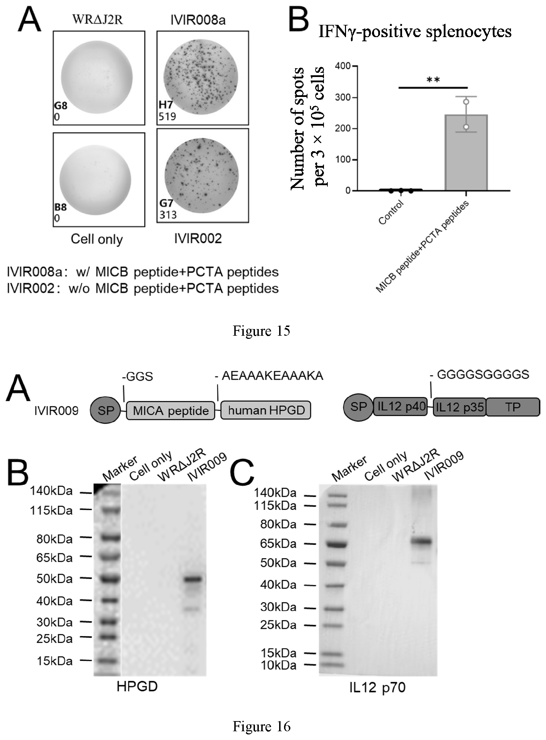

Figure 15 shows the IFNγ ELISpot results of splenocytes from 6-8 week-old female Balb/c mice 14 days after intraperitoneal injection of WC strain recombinant viruses, including IVIR008a (expressing MICB peptide and PCTA peptides, w/ MICB peptide + PCTA peptides), IVIR002 (not expressing MICB peptide and PCTA peptides, w/o MICB peptide + PCTA peptides), and control virus WRΔJ2R (J2R-knockout WR strain). Figure 15A shows the IFNγ spot images of splenocytes from mice in each group. Figure 15B shows the number of MICB peptide- and PCTA peptide-specific IFNγ spots (each spot represents one immune cell capable of secreting IFNγ). In the bar chart, the data shown for MICB peptide + PCTA peptides represent the spot number of the IVIR008a injection group minus the spot number of the IVIR002 injection group in Figure 15A, and the data shown for Control represent the spot number of the WRΔJ2R injection group minus the spot number of the Cell only group in Figure 15A. The data shown in the figure represent the mean ± SD, and the error bars represent ± SD. ** MICB peptide + PCTA peptides vs. Control, p-value < 0.01.

Figure 16 shows the WB verification of fusion protein expression after CV-1 cells were infected with WC strain recombinant virus IVIR009 delivering tumor antigen MICA peptide fusion proteins for 16 h. Figure 16A shows a schematic diagram of fusion proteins expressed by WC strain recombinant virus IVIR009, which expresses secreted fusion protein of tumor antigen MICA peptide and human HPGD (secreted MICA peptide + hHPGD, sMICAH) and type I membrane protein IL12 p70 (mIL12 I), with the J2R-knockout WR strain (WRΔJ2R) as its control virus. Figures 16B-C show the WB results of non-extracellular (intracellular and membrane-bound) HPGD and IL12 p70 in the infection groups of IVIR009 and WRΔJ2R.

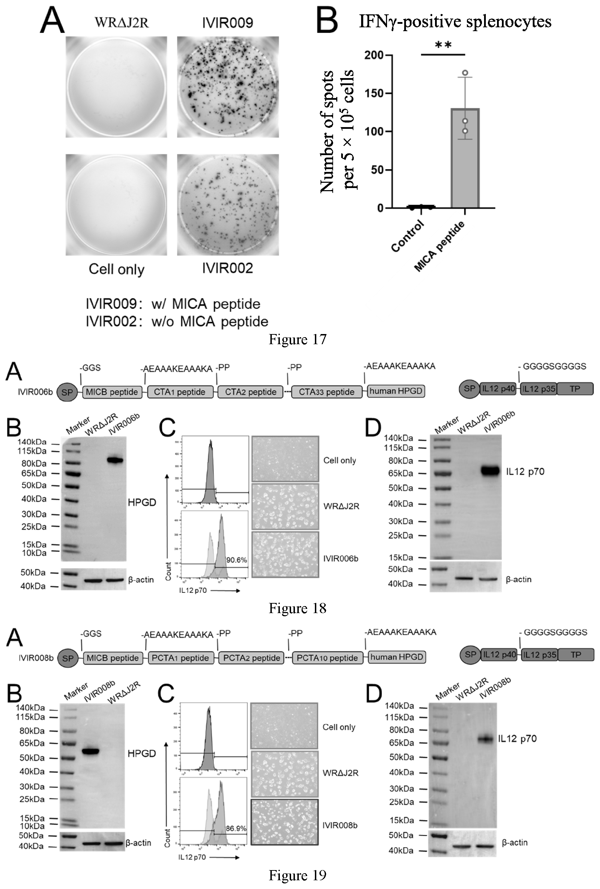

Figure 17 shows the IFNγ ELISpot results of splenocytes from 6-8 week-old female Balb/c mice 14 days after intraperitoneal injection of WC strain recombinant viruses, including IVIR009 (expressing MICA peptide, w/ MICA peptide), IVIR002 (not expressing MICA peptide, w/o MICA peptide), and control virus WRΔJ2R (J2R-knockout WR strain). Figure 17A shows the IFNγ spot images of splenocytes from mice in each group. Figure 17B shows the number of MICA peptide-specific IFNγ spots (each spot represents one immune cell capable of secreting IFNγ). In the bar chart, the data shown for MICA peptide represent the spot number of the IVIR009 injection group minus the spot number of the IVIR002 injection group in Figure 17A, and the data shown for Control represent the spot number of the WRΔJ2R injection group minus the spot number of the Cell only group in Figure 17A. The data shown in the figure represent the mean ± SD, and the error bars represent ± SD. ** MICA peptide vs. Control, p-value < 0.01.

Figure 18 shows the verification of fusion protein expression after CV-1 cells were infected with WC strain recombinant virus IVIR006b delivering fusion proteins of multiple cancer-testis antigen peptides for 16 h. Figure 18A shows a schematic diagram of fusion proteins expressed by WC strain recombinant virus IVIR006b, which expresses secreted fusion protein of tumor antigen MICB peptide, multiple cancer-testis antigen peptides, and human HPGD (secreted MICB peptide + multiple CTA peptides + hHPGD, smCTAH), and type I membrane protein IL12 p70 (mIL12 I), and differs from IVIR006a in that PP linker peptide is used as the linker peptide between one CTA peptide and another CTA peptide. Figure 18B shows the WB results of non-extracellular (intracellular and membrane-bound) HPGD in the infection groups of IVIR006b and control virus WRΔJ2R (J2R-knockout WR strain). Figure 18C shows the flow cytometry results of IL12 p70 expression on the cell membrane in the infection groups of IVIR006b and WRΔJ2R, and the virus-uninfected group (Cell only group). Figure 18D shows the WB results of non-extracellular (intracellular and membrane-bound) IL12 p70 in the infection groups of IVIR006b and WRΔJ2R.

Figure 19 shows the verification of fusion protein expression after CV-1 cells are infected with the WC strain recombinant virus delivering fusion proteins of multiple prostate carcinoma tumor antigen peptides for 16 h. Figure 19A shows a schematic diagram of fusion proteins expressed by WC strain recombinant virus IVIR008b, which expresses secreted fusion protein of MICB peptide, multiple prostate carcinoma tumor antigen peptides, and human HPGD (secreted MICB peptide + multiple PCTA peptides + hHPGD, smPCTAH), and type I membrane protein IL12 p70 (mIL12 I), and differs from IVIR008a in that PP linker peptide is used as the linker peptide between one PCTA peptide and another PCTA peptide. Figure 19B shows the WB results of non-extracellular (intracellular and membrane-bound) HPGD and the internal reference protein β-actin in the infection groups of IVIR008b and control virus WRΔJ2R (J2R-knockout WR strain). Figure 19C shows the flow cytometry results of IL12 p70 expression on the cell membrane in the infection groups of IVIR008b and WRΔJ2R, and the virus-uninfected group (Cell only group). Figure 19D shows the WB results of non-extracellular (intracellular and membrane-bound) IL12 p70 expression in the infection groups of IVIR008b and WRΔJ2R.

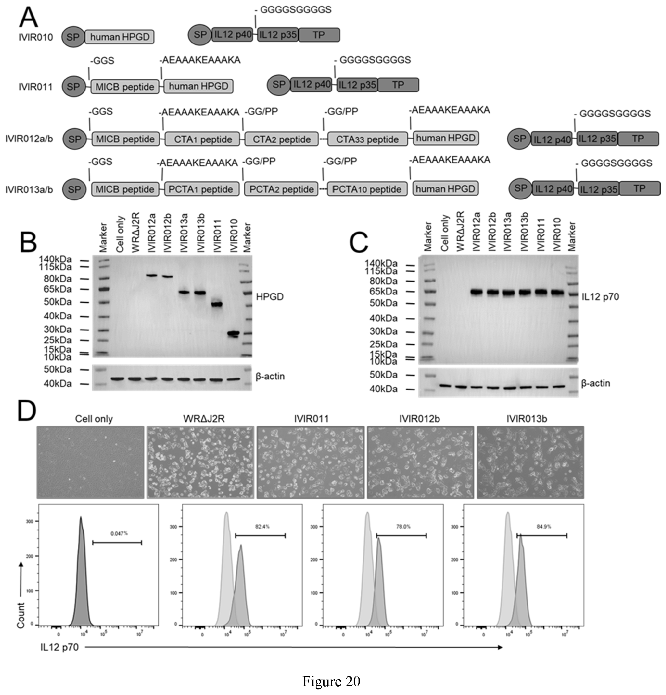

Figure 20 shows the verification of fusion protein expression after CV-1 cells are infected with WC strain recombinant viruses delivering tumor antigen peptide fusion proteins for 16 h. Figure 20A shows a schematic diagram of fusion proteins expressed by WC strain recombinant viruses IVIR010, IVIR011, IVIR012a/b, and IVIR013a/b. Figure 20B shows the WB results of non-extracellular (intracellular and membrane-bound) HPGD and the internal reference protein β-actin in the infection groups of each recombinant virus and control virus WRΔJ2R (J2R-knockout WR strain). Figure 20C shows the WB results of non-extracellular (intracellular and membrane-bound) IL12 p70 and the internal reference protein β-actin expression in the infection groups of each recombinant virus and WRΔJ2R. Figure 20D shows the flow cytometry results of IL12 p70 expression on the cell membrane in the infection groups of WRΔJ2R, IVIR011, IVIR012b, and IVIR013b, and the virus-uninfected group (Cell only group).

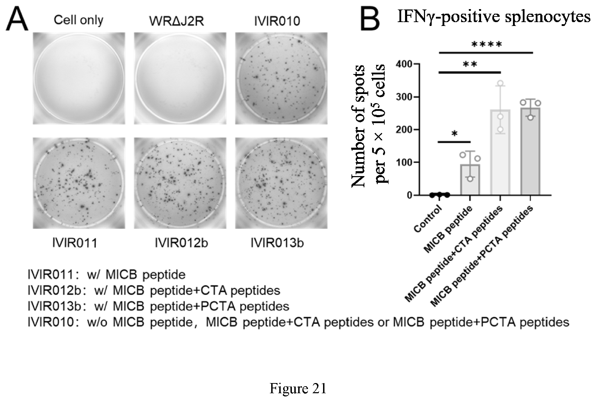

Figure 21 shows the IFNγ ELISpot results of splenocytes from 6-8 week-old female Balb/c mice 7 days after intraperitoneal injection of WC strain recombinant viruses, including IVIR011 (expressing MICB peptide, w/ MICB peptide), IVIR012b (expressing MICB peptide and CTA peptides, w/ MICB peptide + CTA peptides), IVIR013b (expressing MICB peptide and PCTA peptides, w/ MICB peptide + PCTA peptides), IVIR010 (not expressing MICB peptide, MICB peptide and CTA peptides, or MICB peptide and PCTA peptides, w/o MICB peptide, MICB peptide + CTA peptides, or MICB peptide + PCTA peptides), and control virus WRΔJ2R (J2R-knockout WR strain). Figure 21A shows the IFNγ spot images of splenocytes from mice in each group. Figure 21B shows the number of MICB peptide-, MICB peptide + CTA peptide-, and MICB peptide + PCTA peptide-specific IFNγ spots (each spot represents one immune cell capable of secreting IFNγ). In the bar chart, the data shown for MICB peptide, MICB peptide + CTA peptides, and MICB peptide + PCTA peptides represent the spot number of the IVIR011 injection group, the IVIR012b injection group, and the IVIR013b injection group minus the spot number of the IVIR010 injection group in Figure 21A, respectively, and the data shown for Control represent the spot number of the WRΔJ2R injection group minus the spot number of the Cell only group in Figure 21A. The data shown in the figure represent the mean ± SD, and the error bars represent ± SD. * MICB peptide vs. Control, p-value < 0.05; ** MICB peptide + CTA peptides vs. Control, p-value < 0.01; and **** MICB peptide + PCTA peptides vs. Control, p-value < 0.0001.

### Detailed Description of Embodiments

The following description of the present disclosure is merely intended to illustrate various different embodiments of the present disclosure. Therefore, the specific modifications in question should not be construed as limiting the scope of the present disclosure. It is obvious to those skilled in the art that various different equivalents, changes and modifications can be made without departing from the scope of the present disclosure, and it should be understood that such equivalent embodiments are encompassed herein. All references cited herein, including publications, patents, and patent applications, are incorporated by reference in their entirety.

A first aspect of the present disclosure provides a tumor antigen peptide fusion protein, comprising a signal peptide, human 15-hydroxyprostaglandin dehydrogenase (HPGD), and optionally at least one tumor antigen peptide.

In some embodiments, the signal peptide, HPGD, and/or tumor antigen peptide are human peptides or non-human mammalian peptides.

In some embodiments, the tumor antigen peptide may be selected, for example, from an MHC class I polypeptide-related sequence peptide, a cancer-testis antigen (CTA) peptide, and a prostate carcinoma tumor antigen (PCTA) peptide. The MHC class I polypeptide-related sequence is preferably MHC class I polypeptide-related sequence A (MICA) or MHC class I polypeptide-related sequence B (MICB); the CTA is preferably CAMEL (melanoma surface antigen recognized by CTLs; cancer-testis antigen 2; CT6.2), BAGE (melanoma antigen; CT2.1), DAM-6/10 (MAGE-B1/B2; Alzheimer's disease-associated microglia; melanoma antigen family B member 2; CT3.2), GAGE-2 (G antigen 2; CT4.2), GAGE-5 (G antigen 5; CT4.5), GAGE-6 (G antigen 6; CT4.6), GAGE-7 (G antigen 7; CT4.7), GAGE-8 (G antigen 8; CT4.8), GAGE-7B (G antigen 7B; CT4.7), MAGE-A1 (melanoma-associated antigen A1; CT1.1), MAGE-A2 (melanoma-associated antigen A2; CT1.2), MAGE-A3 (melanoma-associated antigen A3; CT1.3), MAGE-A4 (melanoma-associated antigen A4; CT1.4), MAGE-A6 (melanoma-associated antigen A6; CT1.6), MAGE-A10 (melanoma-associated antigen A10; CT1.10), MAGE-A12 (melanoma-associated antigen A12; CT1.12), NY-ESO-1 (human esophageal squamous cell carcinoma antigen 1; cancer-testis antigen 1B; CT6.1), CTAG1A (cancer-testis antigen 1A; CT6.1), or TRAG-3 (taxol resistance-associated gene 3; chondrosarcoma-associated gene family member 2; CT24.2); and the PCTA is preferably prostatic acid phosphatase (PAP). In some embodiments, the sequences of the tumor antigen peptides are shown in Table 5.

In some embodiments, the tumor antigen peptide may be, for example, one or more of the MICB, MICA, CTA, and PAP peptides.

In some embodiments, as shown in Figure 1A, the fusion protein may comprise any one of the polypeptide combinations linked in the following order: (i) a signal peptide, an HPGD, and one or more tumor antigen peptides; (ii) a signal peptide, one or more tumor antigen peptides, and an HPGD; and (iii) a signal peptide, one or more tumor antigen peptides, an HPGD, and one or more tumor antigen peptides. The one or more tumor antigen peptides may be the same or different.

In some embodiments, the HPGD and the tumor antigen peptide, as well as two adjacent tumor antigen peptides among the multiple tumor antigen peptides, may be linked via a linker peptide as needed. The linker peptide may be any linker peptide, for example selected from one or more of GG, G₂S, G₃S, (G₄S)ₙ (n is an integer ≥ 1, preferably 1, 2, 3 or 4), [A(EAAAK)ₙA]ₘ (n = 2, 3, or 4, and m = 1 or 2), (XP)ₙ (n is an integer ≥ 1, and X may be any amino acid), (CWₙ) (n is an integer ≥ 1), GFLG, PLGLWA, and RVLAEA.

In some embodiments, the HPGD may be a human HPGD (Gene ID: 3248), the nucleic acid sequence of which is derived from NM_000860.6, NM_001145816.3, NM_001256301.1, NM_001256305.2, NM_001256306.2, NM_001256307.2, or NM_001363574.2, and the amino acid sequence of which is derived from NP_000851.2, NP_001139288.1, NP_001243230.1, NP_001243234.1, NP_001243235.1, NP_001243236.1, or NP_001350503.1; or the HPGD may be a murine HPGD (Gene ID: 15446), the nucleic acid sequence of which is derived from NM_008278.2, and the amino acid sequence of which is derived from NP_032304.2. Preferably, the amino acid sequence of the human HPGD is SEQ ID NO: 2; and the amino acid sequence of the murine HPGD is SEQ ID NO: 1.

In some embodiments, the MICB may be a human MICB (Gene ID: 4277), the nucleic acid sequence of which is derived from NM_001289160.2, NM_001289161.2, NM_005931.5, XM_054328503.1, XM_054330825.1, or XM_054355464.1, and the amino acid sequence of which is derived from NP_001276089.1, NP_001276090.1, NP_005922.2, XP_054184478.1, XP_054186800.1, or XP_054211439.1. Preferably, the amino acid sequence of the MICB peptide is SEQ ID NO: 28, as shown in Table 5.

In some embodiments, the MICA may be a human MICA (Gene ID: 100507436), the nucleic acid sequence of which is derived from NM_000247.3, NM_001177519.3, NM_001289152.2, NM_001289153.2, or NM_001289154.2, and the amino acid sequence of which is derived from NP_000238.1, NP_001170990.1, NP_001276081.1, NP_001276082.1, or NP_001276083.1. Preferably, the amino acid sequence of the MICA peptide is SEQ ID NO: 27, as shown in Table 5.

In some embodiments, the CTA may be CAMEL (Gene ID: 30848), BAGE (Gene ID: 574), DAM-6 (Gene ID: 4113), DAM-10 (Gene ID: 4112), GAGE-2 (Gene ID: 729447), GAGE-5 (Gene ID: 2577), GAGE-6 (Gene ID: 2578), GAGE-7 (Gene ID: 2579), GAGE-8 (Gene ID: 100101629), GAGE-7B (Gene ID: 26748), MAGE-A1 (Gene ID: 4100), MAGE-A2 (Gene ID: 4101), MAGE-A3 (Gene ID: 4102), MAGE-A4 (Gene ID: 4103), MAGE-A6 (Gene ID: 4105), MAGE-A10 (Gene ID: 4109), MAGE-A12 (Gene ID: 4111), NY-ESO-1 (Gene ID: 1485), CTAG1A (Gene ID: 246100), or TRAG-3 (Gene ID: 102723547). Preferably, the amino acid sequences of the CTA peptides are SEQ ID NOs: 29-61, as shown in Table 5. Preferably, the amino acid sequences of the multiple CTA peptides are SEQ ID NO: 8 or 9, as shown in Table 1.

In some embodiments, the PCTA may be a PAP; and the PAP may be a human PAP (Gene ID: 55), the nucleic acid sequence of which is derived from NM_001134194.2, and the amino acid sequence of which is derived from NP_001127666.1. Preferably, the PCTA peptide is a PAP peptide, the amino acid sequence of which may be SEQ ID NO: 62, as shown in Table 5. Preferably, the amino acid sequences of the multiple PCTA peptides are SEQ ID NO: 10 or 11, as shown in Table 1.

In some embodiments, the amino acid sequences of the signal peptide, HPGD, MICB peptide, MICA peptide, CTA peptide, and PCTA peptide are those as shown in Table 1 or Table 5, or variants thereof.

In some embodiments, as shown in Figure5B, the fusion protein comprises:
(1) sHPGD: signal peptide + HPGD;
(2) sMICBH: signal peptide + MICB peptide + HPGD;
(3) sMICAH: signal peptide + MICA peptide + HPGD;
(4) smCTAH: signal peptide + MICB peptide + CTA₁ peptide + CTA₂ peptide + ... + CTAₙ peptide + HPGD, wherein n is an integer from 1 to 50, preferably 33; and wherein the CTA₁ peptide, CTA₂ peptide, ..., and CTAₙ peptide may be the same or different; or
(5) smPCTAH: signal peptide + MICB peptide + PCTA₁ peptide + PCTA₂ peptide + ... + PCTAₙ peptide + HPGD, wherein n is an integer from 1 to 20, such as 10; and wherein the PCTA₁ peptide, PCTA₂ peptide, ..., and PCTAₙ peptide may be the same or different.

In (4) above, smCTAH comprises n CTA peptides, preferably 33 different CTAs, which are represented by CTA₁ to CTA₃₃ (i.e., SEQ ID NOs: 29-61).

In (5) above, smPCTAH comprises n PCTA peptides, preferably 10 identical prostatic acid phosphatase peptide (PAP peptide) sequences (SEQ ID NO: 62).

In some embodiments, the linker peptide between the MICB peptide or MICA peptide and the signal peptide is GGGGSGGGGS, GGGS, GGS, GG, etc. In some embodiments, the linker peptide between the CTA peptide or PCTA peptide and the MICB peptide is AEAAAKEAAAKA, etc. In some embodiments, the linker peptide between the CTA peptides or between the PCTA peptides is GG, PP, etc.

In some preferred embodiments, the signal peptide comprises SEQ ID NO: 7 or a variant thereof. In some preferred embodiments, the HPGD comprises the amino acid sequence of SEQ ID NO: 1 or 2, or a variant thereof. In some preferred embodiments, the MICB peptide comprises SEQ ID NO: 28 or a variant thereof.

In some preferred embodiments, the tumor antigen peptide fusion protein comprises an amino acid sequence selected from SEQ ID NOs: 12-18, or variants thereof, as shown in Table 2.

A variant of a protein of the present disclosure, such as a signal peptide, HPGD, and/or tumor antigen peptide, refers to a protein that shares at least 75% or more (e.g., 75%, 80%, 85%, 90%, 91%, 92%, 93%, 94%, 95%, 96%, 97%, 98%, 99%, 99.2%, 99.5%, 99.8% or more) sequence identity with the corresponding amino acid sequence; or comprises one or more (e.g., 1, 2, 3, 4, 5, 6, 7, 8, 9, or 10) amino acid deletions, insertions and/or substitutions in the corresponding amino acid sequence; or is a functional domain thereof; wherein the altered sequence substantially retains at least one biological activity of the amino acid sequence from which it is derived. Preferably, the substitution is a conservative substitution.

A second aspect of the present disclosure provides a composition (also referred to as a fusion protein composition), comprising the tumor antigen peptide fusion protein of the first aspect and a therapeutic agent. The therapeutic agent comprises a therapeutic agent fusion protein, comprising a therapeutic agent and at least one peptide selected from a signal peptide, a transmembrane peptide, and a signal-anchor peptide, wherein the signal peptide, the transmembrane peptide, and/or the signal-anchor peptide are located at the N-terminus or C-terminus of the therapeutic agent fusion protein. Thus, the therapeutic agent fusion protein may be secreted (comprising only a signal peptide) or transmembrane (comprising a signal peptide and a transmembrane peptide, or comprising a signal-anchor peptide).

In some embodiments, the therapeutic agent fusion protein is an IL12 p70 fusion protein. The IL12 p70 fusion protein comprises IL12 p35 and IL12 p40, preferably IL12 p40 and IL12 p35 linked via a linker peptide.

In some embodiments, the IL12 p70 may be a human IL12 p70, consisting of two genes IL12 p35 (Gene ID: 3592) and IL12 p40 (Gene ID: 3593), wherein the nucleic acid sequence of IL12 p35 is derived from NM_000882.4, NM_001354582.2, NM_001354583.2, or NM_001397992.1, and the amino acid sequence thereof is derived from NP_000873.2, NP_001341511.1, NP_001341512.1, or NP_001384921.1; and the nucleic acid sequence of IL12 p40 is derived from NM_002187.3, and the amino acid sequence thereof is derived from NP_002178.2. Preferably, the amino acid sequence of the human IL12 p35 is SEQ ID NO: 6, as shown in Table 1, wherein the underlined amino acid sequence is used for the construction of the IL12 p70 fusion protein; and preferably, the amino acid sequence of the human IL12 p40 is SEQ ID NO: 5, as shown in Table 1, wherein the underlined amino acid sequence is used for the construction of the IL12 p70 fusion protein.

In some embodiments, the IL12 p70 may be a murine IL12 p70, consisting of two genes IL12 p35 (Gene ID: 16159) and IL12 p40 (Gene ID: 16160), wherein the nucleic acid sequence of IL12 p35 is derived from NM_001159424.3, NM_001410417.1, NM_001410418.1, NM_001410419.1, NM_001410420.1, or NM_008351.4, and the amino acid sequence thereof is derived from NP_001152896.2, NP_001397346.1, NP_001397347.1, NP_001397348.1, NP_001397349.1, or NP_032377.1; and the nucleic acid sequence of IL12 p40 is derived from NM_001303244.1, and the amino acid sequence thereof is derived from NP_001290173.1. Preferably, the amino acid sequence of the murine IL12 p35 is SEQ ID NO: 4, as shown in Table 1, wherein the underlined amino acid sequence is used for the construction of the IL12 p70 fusion protein; and preferably, the amino acid sequence of the murine IL12 p40 is SEQ ID NO: 3, as shown in Table 1, wherein the underlined amino acid sequence is used for the construction of the IL12 p70 fusion protein.

In some embodiments, as shown in Figure 1B, the IL12 p70 fusion protein may comprise:
(i) a secreted IL12 p70 (sIL12): a signal peptide, IL12 p35, a linker peptide, and IL12 p40 linked in sequence, or a signal peptide, IL12 p40, a linker peptide, and IL12 p35 linked in sequence;
(ii) a type I membrane protein IL12 p70 (mIL12 I): a signal peptide, IL12 p35, a linker peptide, IL12 p40, and a transmembrane peptide linked in sequence, or a signal peptide, IL12 p40, a linker peptide, IL12 p35, and a transmembrane peptide linked in sequence; and/or
(iii) a type II membrane protein IL12 p70 (mIL12 II): a signal-anchor peptide, IL12 p35, a linker peptide, and IL12 p40 linked in sequence, or a signal-anchor peptide, IL12 p40, a linker peptide, and IL12 p35 linked in sequence.

In some embodiments, IL12 p35 comprises an amino acid sequence selected from the underlined portions of SEQ ID NOs: 4 and 6, or variants thereof. In some embodiments, IL12 p40 comprises an amino acid sequence selected from the underlined portions of SEQ ID NOs: 3 and 5, or variants thereof. In some embodiments, the signal peptide comprises SEQ ID NO: 7 or a variant thereof. In some embodiments, the signal-anchor peptide comprises SEQ ID NO: 63 or a variant thereof. In some embodiments, the transmembrane peptide comprises SEQ ID NO: 64 or a variant thereof.

In addition, the same linker peptide as used in the tumor antigen peptide fusion protein may be used in the IL12 p70 fusion protein as needed.

In some embodiments, the IL12 p70 fusion protein comprises an amino acid sequence selected from SEQ ID NOs: 21-26, or variants thereof, as shown in Table 4.

A third aspect of the present disclosure provides a nucleic acid construct encoding the tumor antigen peptide fusion protein of the first aspect, or the composition of the second aspect.

The present disclosure is not particularly limited to the nucleic acid construct, as long as it can encode the relevant fusion protein; for example, it may be a DNA fragment, mRNA, or plasmid. The fusion protein may be expressed by means of a shuttle plasmid, as shown in Figure 2. The shuttle plasmid may be a shuttle plasmid as shown in Figure 4.

A fourth aspect of the present disclosure provides a genetically engineered recombinant virus, comprising the nucleic acid construct of the third aspect.

In some embodiments, the recombinant virus may be a DNA virus, an RNA virus, or a mutant thereof. The DNA virus may be a single-stranded (ss) DNA virus, a double-stranded (ds) DNA virus, or a DNA virus containing ssDNA and dsDNA regions, including the families Adenoviridae, Herpesviridae, and Poxviridae, such as adenovirus, adeno-associated virus, replication-defective retrovirus, herpes simplex virus 1 (HSV1), and vaccinia virus (VV).

In some embodiments, the vaccinia virus may be the Western Reserve (WR) strain, Copenhagen strain, Lister strain, Ankara strain, Modified Vaccinia Ankara strain, New York Vaccinia strain, Tiantan strain, Dryvax strain, Bern strain, Paris strain, Tash Kent strain, IHD-J strain, IHD-W strain, Brighton strain, CVA382 strain, Dairen strain, LC16m8 strain, LC16M0 strain, LIVP strain, ACAM2000 strain, WR65-16 strain, Connaught strain, EM-63 strain, or a derivative strain or genetically engineered strain thereof. The genetically engineered strain may comprise knockout of vaccinia virus genes, as well as knock-in of heterologous nucleic acids (e.g., genes encoding the fusion protein, tumor-targeting genes, tumor suppressor genes, etc.) and optionally other vaccinia virus strain genes. Viral genes that may be knocked out include any one or more regions of A34R, J2R, A44L, A45R, A46R, and/or A52R. In some embodiments, the genetically engineered vaccinia virus comprises knockout of one or two or more genes (J2R, A34R, A44L-A45R-A46R, and A52R) in the vaccinia virus genome, as well as knock-in of exogenous genes and a nucleic acid sequence encoding the fusion protein. The A44L-A45R-A46R gene includes any one of the A44L, A45R, and A46R genes, or a combination of any two or all three thereof, and preferably includes a combination of all three of the A44L, A45R, and A46R genes.

In some embodiments, the genetically engineered vaccinia virus is preferably an extracellular enveloped virus (EEV)-enhanced vaccinia virus, which comprises modification of the A34R gene encoding the vaccinia virus EEV outer membrane glycoprotein, including replacement and/or mutation of the A34R gene. Preferably, the EEV-enhanced vaccinia virus is a vaccinia virus WR strain comprising modification of the A34R gene.

In some embodiments, the modification includes replacement of the WR A34R gene and/or mutation of the WR A34R gene. In some embodiments, the replacement includes replacing the A34R gene of the vaccinia virus WR strain with the A34R gene of the Copenhagen strain, IHD-J strain, or IHD-W strain. In some embodiments, the EEV-enhanced vaccinia virus comprising a mutation may be, for example, the WI strain, the WC strain, or a strain obtained by introducing a point mutation into the A34R gene of the WR strain. The WI strain is a strain that uses the genome of the vaccinia virus WR strain as its backbone, and in which the A34R gene sequence of the WR strain is replaced with the A34R gene sequence of the IHD-J strain. The WC strain is a strain that uses the WR strain genome as its backbone, and in which the A34R gene sequence of the WR strain is replaced with the A34R gene (coA34R) sequence of the Copenhagen strain.

In some embodiments, the EEV-enhanced vaccinia virus comprises the coA34R gene, which encodes an amino acid sequence comprising SEQ ID NO: 19 or a variant thereof.

A fifth aspect of the present disclosure provides an expression system, comprising a host cell and the genetically engineered recombinant virus of the fourth aspect.

In the present disclosure, the host cell may be a prokaryotic cell or a eukaryotic cell, preferably a eukaryotic cell, more preferably a mammalian cell, e.g., HeLa cells, human embryonic kidney 293 (HEK 293) cells, Chinese hamster ovary (CHO) cells, mouse myeloma cells, African green monkey kidney (CV-1 or Vero) cells, etc.

The genetically engineered recombinant virus expressed by the host cell can selectively infect target cells, achieve efficient delivery and release of tumor antigens in vivo, enhance/activate the anti-tumor immune function of the immune system, and promote the proliferation of specific effector cells and the killing of tumor cells, thus achieving the effects of treating a tumor.

For the tumor antigen peptide fusion protein, fusion protein composition, and genetically engineered recombinant virus of the present disclosure, the effector cells thereof are any mammalian cell types capable of promoting the death of target cells. In some embodiments, the effector cells are immune cells, such as T cells, B cells, innate lymphoid cells, natural killer (NK) cells, natural killer T (NKT) cells, granulocytes (e.g., neutrophils, basophils, mast cells, or eosinophils), macrophages, monocytes, or dendritic cells (DCs). Both viral vectors and therapeutic polypeptides can activate or restore effector cells (e.g., T cells) such that they have one or more of the following characteristics: (i) increased proliferation of effector cells; (ii) altered expression or activity of one or more cell surface proteins of effector cells; (iii) altered expression or activity of one or more intracellular proteins expressed by effector cells; (iv) altered amount or properties of factors produced and/or secreted by effector cells (e.g., cytokines, chemokines, or reactive oxygen species); (v) altered morphology of effector cells; (vi) altered chemotactic potential of effector cells, such as increased or decreased expression of one or more chemokine receptors; and (vii) altered functional activity of effector cells, such as increased cytolytic activity and/or increased phagocytic activity. Activation of an effector cell or population of effector cells can be determined by any means known in the art; for example, changes in cell proliferation and protein expression, production, or secretion can be determined by flow cytometry, Western blotting (WB), enzyme-linked immunosorbent assay (ELISA), enzyme-linked immunospot assay (ELISpot), immunohistochemistry, immunoprecipitation, or immunofluorescence.

A sixth aspect of the present disclosure provides a pharmaceutical composition, comprising the tumor antigen peptide fusion protein, composition, nucleic acid construct, or genetically engineered recombinant virus of the present disclosure; and a pharmaceutically acceptable carrier.

A seventh aspect of the present disclosure provides a method for treating or preventing a tumor, comprising administering the tumor antigen peptide fusion protein, composition, nucleic acid construct, genetically engineered recombinant virus, or pharmaceutical composition of the present disclosure.

An eighth aspect of the present disclosure provides the use of the tumor antigen peptide fusion protein, composition, nucleic acid construct, genetically engineered recombinant virus, or pharmaceutical composition of the present disclosure in the manufacture of a medicament for treating or preventing a tumor.

A ninth aspect of the present disclosure provides a method for activating or enhancing the function of immune cells, comprising administering the tumor antigen peptide fusion protein, composition, nucleic acid construct, genetically engineered recombinant virus, or pharmaceutical composition of the present disclosure.

In some embodiments, the dosage form, mode of administration, etc. of the tumor antigen peptide fusion protein, composition, nucleic acid construct, genetically engineered recombinant virus, or pharmaceutical composition of the present disclosure as described above may be specifically selected as needed. The dosage form may be a cream or a lipid composition. The mode of administration may be local or systemic, and the foregoing may be formulated for local intratumoral injection, local intraarterial injection (into tumor-supplying blood vessels), subcutaneous injection, intracavitary injection, intraperitoneal injection, intrathoracic injection, systemic intravenous injection, intramuscular injection, intradermal injection, intrathecal injection, direct intraventricular injection, intracardiac injection, intranasal injection, and other modes of administration. The pharmaceutical composition may be used alone as monotherapy, or in combination with an anticancer agent.

In some specific embodiments, as shown in Figure 5, the present disclosure provides the following recombinant viruses:
(1) IVIR001, IVIR002, and IVIR010 are recombinant viruses expressing secreted human HPGD (sHPGD) and type I membrane protein IL12 p70 (mIL12 I).
(2) IVIR003, IVIR004, and IVIR011 are recombinant viruses expressing mIL12 I and secreted fusion protein of the tumor antigen MICB peptide and human HPGD (sMICBH).
(3) IVIR005, IVIR006, and IVIR012 are recombinant viruses expressing mIL12 I and secreted fusion protein of the MICB peptide, multiple cancer-testis antigen peptides, and human HPGD (smCTAH). They each include two subtypes, a and b, which differ in that the linker peptides between CTA peptides are different, wherein the GG linker peptide is used in subtype a, and the PP linker peptide is used in subtype b.
(4) IVIR007, IVIR008, and IVIR013 are recombinant viruses expressing mIL12 I and secreted fusion protein of the MICB peptide, multiple prostate carcinoma tumor antigen peptides, and human HPGD (smPCTAH). They each include two subtypes, a and b, which differ in that the linker peptides between PCTA peptides are different, wherein the GG linker peptide is used in subtype a, and the PP linker peptide is used in subtype b.
(5) IVIR009 is a recombinant virus expressing mIL12 I and secreted fusion protein of the MICA peptide and human HPGD (sMICAH).

The amino acid sequences of the preferred fusion proteins in (1) to (5) above are shown in Table 2 and Table 4.

### Terms

"Tumor antigen (TA)" refers to a class of major histocompatibility complex (MHC)-associated molecules expressed on the surface of tumor cells, and is key to the preparation of therapeutic cancer vaccines. Vaccine targets are rationally selected and personalized therapeutic regimens are formulated according to the individual tumor conditions of patients to achieve the goal of cancer treatment. Moreover, the first-in-human clinical trials of personalized cancer vaccines have demonstrated the feasibility, safety, and immunotherapeutic activity targeting tumor mutational signatures.

"Tumor antigen peptide (TA peptide)" is a class of tumor markers, which act as antigens to elicit anti-tumor immune responses and as targets for immune cells to attack tumor cells. The core of tumor immunotherapy is the reaction between MHC-presented tumor antigens and T cells. Small molecular peptides produced by the degradation of tumor antigens in cells bind to MHC class I molecules on the surface of antigen-presenting cells, are expressed on the surface of cancer cells, and are presented to T cells.

"Cancer-testis antigen" or "CTA", also known as cancer germline antigen, is derived from genes expressed during fetal development, and has a restricted expression pattern in normal tissues, with its expression generally limited to germline tissues such as testis, ovary, and placenta. The human genome comprises more than 200 CTA genes classified into 44 gene families, some of which include multiple members. Homologs and analogs of CTA genes have been found in various organisms including primates, such as rodents, zebrafish, drosophila, nematodes, and even yeast. Relevant data can be retrieved from the Ludwig Institute for Cancer Research (http://www.cta.lncc.br/index.php). Their expression has the following characteristics: (1) they are expressed at different frequencies in various tumor tissues; (2) most CTAs are located on the X chromosome; (3) they usually exist as multiple family members; and (4) the expression of CTAs is often heterogeneous in tumor tissues of diverse origins.

"Protein" or "polypeptide" refers to a chain of at least two linked amino acids, including post-translational modifications of proteins such as glycosylation, acetylation, phosphorylation, etc., as well as variants obtained by genetic engineering-based modifications such as deletions, substitutions, and insertions of the amino acid sequences of native proteins or polypeptides.

"Modification" refers to the alteration of the amino acid sequences of proteins or polypeptides, including linkage to acetyl groups, carboxyl groups, glycosyl groups, immunoglobulin (Ig) Fc, polyethylene glycol (PEG), streptavidin, and various other molecules (e.g., biotin, radioisotopes, fluorescent agents, enzymes, cytotoxic substances, anti-tumor agents, etc.), as well as linkage to functional groups (e.g., phosphate residues and amino acid residues). After modification, the biochemical characteristics of the proteins are altered, e.g., improved proteolytic resistance, enhanced solubility, etc., or new biochemical characteristics are conferred, e.g., targeting ability, novel antigen recognition epitopes, etc. In the present disclosure, a signal peptide may be coupled to the N-terminus of a tumor antigen peptide to confer the properties of a secreted protein; or a tumor antigen peptide may be concatenated with the tumor suppressor HPGD so that the encoded protein exerts a tumor suppressive effect; or different human tumor antigen peptides may be linked to develop personalized therapeutic cancer vaccines.

"Signal peptide (SP)" refers to a specific sequence at the N-terminus of a secreted protein (not necessarily at the N-terminus in some cases), which can direct the polypeptide and ribosome to the endoplasmic reticulum membrane and the transmembrane translocation of the nascent peptide. The SP is usually cleaved by signal peptidase upon entry into the endoplasmic reticulum. If a secreted protein is synthesized, the entire polypeptide enters the lumen of the endoplasmic reticulum except for the cleaved SP; and if it is a membrane protein, one or more internal stop-transfer signals anchor the protein to the endoplasmic reticulum membrane.

"Transmembrane peptide (TP)" refers to a class of short peptides that anchor proteins or polypeptides to the cell membrane, perform numerous biological functions in cells, and are essential for molecular transport, signal transduction, and membrane fusion.

"Signal-anchor peptide (SAP)" refers to a polypeptide with a function similar to that of the signal peptide (SP), which allows the transmembrane translocation of the peptide chain without being cleaved. It serves as the signal peptide for type II membrane proteins and enables membrane anchoring.

"Linker peptide" refers to a chain of amino acids that links two fusion proteins, with a certain degree of flexibility to allow the proteins on both sides to perform their respective independent functions. It is generally divided into three types, which are flexible linker, rigid linker, and cleavable linker, respectively. As an indispensable component of recombinant fusion proteins, linker peptides play an important role in constructing stable and biologically active fusion proteins.

"Fusion protein" refers to a protein product expressed by purposefully linking two or more genes encoding functional protein molecules using genetic engineering and other techniques. The fusion protein is a novel protein with two or more biological activities and functions. Fusion protein technology is used to improve the properties of proteins, including stability, activity, expression, secretion, and solubility, or confer new functions on proteins. Therefore, fusion protein technology is widely used in clinical practice for the preparation of vaccines, drugs with specific biological activities, thrombolytic agents, antimicrobial peptides, bifunctional enzymes, etc.

"MICA" and "MICB" are two highly similar MHC class I polypeptide-related sequences, widely expressed in mammals, upregulated in virus-infected and tumor-transformed cells, and encode cell surface glycoproteins. As a class of cellular stress antigen molecules, MICA/MICB activate cytotoxic NK cells, T cells, etc. by binding to their receptor G2D natural killer group 2 member D (NKG2D), thereby playing an important role in immune surveillance of the body. Release of soluble MICA/MICB is regarded as a mechanism of tumor immune escape, which can reduce the density of NKG2D ligands on the surface of tumor cells and contribute to the inhibition of immune effector cells; and the levels of soluble MICA/MICB have been shown to be elevated in the serum of patients with various malignant diseases. It has been reported that stabilizing the expression of MICA/MICB may be one of the approaches to activate anti-tumor immune responses.

"15-hydroxyprostaglandin dehydrogenase (HPGD)" is an NAD⁺-dependent prostaglandin-degrading enzyme, which is ubiquitously expressed in mammalian tissues, localized in the cytoplasm, exists as a homodimer, widely regulates a variety of cellular pathways such as inflammation, differentiation, and signal transduction, and serves as an important target for pharmacological intervention. Existing studies have shown that the HPGD-encoding gene has multiple transcript variants responsible for encoding different isoforms. By degrading prostaglandins (tumor-promoting molecules) in cancer cells, HPGD can inhibit cancer cell proliferation and induce cancer cell differentiation, and HPGD knockout or low expression can increase susceptibility to colon tumors and the spread of pancreatic tumor cells. However, there are relatively few studies on the role of HPGD in tumorigenesis and immune responses, and its function remains unclear, which still requires further exploration.

The term "identity" is used to refer to the degree of sequence matching between two polypeptides or two nucleic acids. When a position in both sequences being compared is occupied by the same base or amino acid monomer subunit (e.g., a position in each of two DNA molecules is occupied by adenine, or a position in each of two polypeptides is occupied by lysine), the molecules are identical at that position. The "percent identity" between two sequences is a function of the number of matching positions shared by the two sequences divided by the number of positions compared × 100. For example, if 6 out of 10 positions in two sequences are matched, the two sequences have 60% identity. For example, the DNA sequences CTGACT and CAGGTT share 50% identity (3 out of a total of 6 positions are matched). Generally, comparison is made when two sequences are aligned to give maximum identity. Such alignments can be made by using, for example, the method described in Needleman et al. (1970) J. Mol. Biol. 48: 443-453, which can be conveniently implemented via computer programs such as the Align program (DNAstar, Inc.). The percent identity between two amino acid sequences can also be determined using the algorithm of E. Meyers and W. Miller (Comput. Appl Biosci., 4: 11-17 (1988)), which has been incorporated into the ALIGN program (version 2.0), using a PAM120 weight residue table, a gap length penalty of 12 and a gap penalty of 4. In addition, the percent identity between two amino acid sequences can be determined using the algorithm of Needleman and Wunsch (J MoI Biol. 48: 444-453 (1970)), which has been incorporated into the GAP program in the GCG software package (available at www.gcg.com), using either a Blossum 62 matrix or a PAM250 matrix, and a gap weight of 16, 14, 12, 10, 8, 6, or 4 and a length weight of 1, 2, 3, 4, 5, or 6.

The term "conservative substitution" refers to an amino acid substitution that does not adversely affect or alter the intended properties of the protein/polypeptide comprising the amino acid sequence. For example, conservative substitutions can be introduced by standard techniques known in the art, such as site-directed mutagenesis and PCR-mediated mutagenesis. Conservative amino acid substitutions include the replacement of an amino acid residue with an amino acid residue having a similar side chain, for example, replacement with a residue that is physically or functionally similar to the corresponding amino acid residue (e.g., having similar size, shape, charge, chemical properties, including the ability to form covalent bonds or hydrogen bonds, etc.). Families of amino acid residues having similar side chains have been defined in the art. These families include amino acids with basic side chains (e.g., lysine, arginine, and histidine), acidic side chains (e.g., aspartic acid and glutamic acid), uncharged polar side chains (e.g., glycine, asparagine, glutamine, serine, threonine, tyrosine, cysteine, and tryptophan), nonpolar side chains (e.g., alanine, valine, leucine, isoleucine, proline, phenylalanine, and methionine), β-branched side chains (e.g., threonine, valine, and isoleucine), and aromatic side chains (e.g., tyrosine, phenylalanine, tryptophan, and histidine). Therefore, the corresponding amino acid residue is preferably replaced with another amino acid residue from the same side chain family. Methods for identifying conservative amino acid substitutions are well known in the art.

"Prostaglandin E2 (PGE₂)" is a member of the eicosanoic acid family, which is produced by almost all cells in the human body, is the most abundant prostaglandin in the human body, performs many important physiological functions, and is widely produced under pathophysiological conditions. Cancer cells that secrete PGE₂ have been shown to induce the formation of human regulatory T cells (Tregs). In addition, PGE₂ selectively inhibits the effector functions of macrophages and neutrophils and type I immunity mediated by Th1, CTL, and NK cells, whereas it promotes the responses of Th2, Th17, and Treg cells and local accumulation of myeloid-derived suppressor cells. Targeting the production, degradation, and reactivity of PGE₂ provides tools for modulating immune patterns in a variety of diseases ranging from autoimmunity to cancer.

"Therapeutic agent" refers to an agent that enhances immune responses, including cytokines, immune costimulatory molecules, immune checkpoint inhibitors, antiangiogenic factors, nucleic acid polymers, etc. The cytokines are involved in cellular activities such as cell signal transduction, apoptosis, development, and differentiation, including chemokines, interferons (INF), interleukins (IL), tumor necrosis factors (TNF), etc., and their respective receptors.

"Interleukin (IL)" refers to a class of molecules with basically clear structures and biological functions and important regulatory roles. It was originally named for being produced by leukocytes and acting among leukocytes, belongs to cytokines, and plays important roles in signal transmission, activation and regulation of immune cells, inflammatory responses, and activation, proliferation, and differentiation of immune T and B cells. It includes IL-1, IL-2, IL-3, IL-4, IL-5, IL-6, IL-7, IL-8 (CXCL8), IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-35, and IL-36.

"J2R" is a gene in the genome of vaccinia virus (VV) that is closely related to DNA replication and encodes a thymidine kinase (TK).

"A52R" is an immunomodulatory gene in the VV genome, which encodes the Toll/interleukin-1 receptor (TIR) protein molecule, inhibits intracellular TIR-dependent Toll-like receptor (TLR)/nuclear factor kappa-B (NF-κB) signaling, suppresses innate immune responses, and enables viral immune escape. Deletion of viral genes involved in TLR signaling has been proven to be an effective way to improve VV as an ideal vaccine candidate.

"A46R" is an immunomodulatory gene in the VV genome which targets multiple TIR adaptor proteins in host cells, such as Myd88, TRIF, TRAM, etc., and its knockout results in significantly improved immunomodulation.

"A44L" is one of the immunomodulatory genes in the VV genome, and its encoded hydroxysteroid dehydrogenase plays a key role in steroid hormone synthesis in cells.

"A45R" is a gene expressed in the late stage of VV infection encoding a viral core protein with an amino acid sequence that is partially identical to that of copper-zinc superoxide dismutase (Cu-Zn SOD). This protein is highly conserved among different VV strains and can interact with the late proteins J1R and A44L to contribute to subsequent viral morphogenesis.

"A34R" refers to a gene in VV encoding a C-type lectin-like glycoprotein gp22-24.

"Target cell" refers to a mammalian cell that is to be killed, attacked, destroyed, and/or controlled. In particular, target cells are cells altered in some way as compared to normal cells of the same cell type, including cancer cells and virus-infected cells.

"Gene knockout (KO)" is a widely used genetic engineering technique that involves the targeted removal or inactivation of specific genes in the genome of an organism. This can be done in a variety of ways, including homologous recombination, CRISPR-Cas9, and TALEN. Gene knockout includes both complete gene knockout and conditional gene knockout (also known as incomplete gene knockout). Complete gene knockout refers to the complete elimination of target gene activity by homologous recombination, and conditional gene knockout refers to the gene knockout in a specific space through a site-specific recombination system.

"Gene knock-in (KI)" is a genetic engineering method in molecular cloning and biology in which a DNA sequence of a gene is replaced or inserted with exogenous gene sequence information. The difference between gene knock-in technology and traditional transgenic technology is that gene knock-in involves the insertion of a gene into a specific site and is therefore "targeted" insertion. It is the opposite of gene knockout.

"Genetically engineered recombinant virus" or "recombinant virus" refers to a virus modified by genetic engineering, which can infect one or more cells or tissues and express a protein encoded by a heterologous nucleic acid. Generally, viruses have natural host cell populations that they infect most efficiently. Therefore, modifying a virus to incorporate proteins from different viruses can facilitate viral entry into specific cell or tissue types, and genetic engineering of viruses is performed for the purpose of disease treatment. In some embodiments, the genetically engineered viruses can selectively infect target cell or tissue types, such as tumor cells of cancer patients, as compared to non-genetically engineered viruses.

"Viral genome" refers to the nucleic acid component of a virus that encodes the genome including any protein required for genome replication and/or integration. In some embodiments, the viral genome acts as a viral vector and may comprise a heterologous gene. The viral genome described herein may be based on any viral vector.

"Vector" is a vehicle used in genetic engineering to introduce a gene of interest into a recipient cell. One type of vector is a non-viral vector, which may be mRNA, DNA, plasmid, bacterium, etc. Another type of vector is a viral vector, and other exogenous gene fragments may be ligated into the viral genome to achieve expression of exogenous genes. The viral vector used may be, for example, a poxvirus, such as a vaccinia virus Western Reserve (WR) strain, Copenhagen strain, Lister strain, Ankara strain, Modified Vaccinia Ankara strain, New York Vaccinia strain, Tiantan strain, Dryvax strain, Bern strain, Paris strain, Tash Kent strain, IHD-J strain, IHD-W strain, Brighton strain, CVA382 strain, Dairen strain, LC16m8 strain, LC16M0 strain, LIVP strain, ACAM2000 strain, WR65-16 strain, Connaught strain, and EM-63 strain, as well as adenovirus, herpes simplex virus, etc. In embodiments, the vector is a vaccinia virus vector, and the knock-in nucleic acid sequence is a recombinant nucleic acid sequence encoding a tumor antigen peptide fusion protein and/or a therapeutic agent fusion protein.

"Expression system" refers to host cells for the replication and amplification of expression vectors. The expression vectors in the present disclosure include host cells of genetically engineered viruses, including HeLa cells, Chinese hamster ovary (CHO) cells, human embryonic kidney 293 (HEK 293) cells, African green monkey kidney (CV-1 or Vero) cells, etc.

The term "pharmaceutically acceptable" means approved by a federal regulatory agency or a state government, or listed in the U.S. Pharmacopeia or other generally recognized pharmacopeia for use in animals and humans without side effects, allergies, or other adverse reactions.

The term "carrier" refers to a diluent, adjuvant, protective agent (e.g., bioadhesive, fat, artificial lipid membrane, and liposome), excipient, or vehicle administered with a therapeutic agent. Such pharmaceutical carriers can be sterile liquids, such as water and oils including those of petroleum, animal, vegetable, or synthetic origin, such as peanut oil, soybean oil, mineral oil, sesame oil, etc. Water is a preferred carrier when the pharmaceutical composition is administered intravenously. Saline solutions, aqueous dextrose, and glycerol solutions can also be used as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, glycol, water, ethanol, etc. The composition, if desired, can also comprise minor amounts of wetting or emulsifying agents, or pH buffering agents. These compositions may be in the form of solutions, suspensions, emulsions, tablets, pills, capsules, powders, sustained-release formulations, etc.

The term "treatment" refers to the administration of an effective amount of the fusion protein, composition, nucleic acid construct, recombinant virus, etc. described herein to a subject such that at least one symptom of the disease is reduced or the disease is ameliorated in the subject, e.g., achieving a beneficial or desired clinical outcome. For the purposes of the present disclosure, beneficial or desired clinical outcomes include, but are not limited to, alleviation of one or more symptoms, diminishment of the disease extent, stabilization (i.e., not worsening) of the disease state, delay or slowing of disease progression, amelioration or palliation of the disease state, and remission (whether partial or total), whether detectable or undetectable. Treatment may refer to prolonging survival as compared to expected survival if not receiving treatment. Thus, those skilled in the art realize that treatment may improve the disease condition, but may not be a complete cure for the disease. As used herein, the term "treatment" includes prophylaxis. Alternatively, treatment is "effective" if the progression of the disease is reduced or halted. "Treatment" may also mean prolonging survival as compared to expected survival if not receiving treatment. Patients in need of treatment include those already diagnosed with the relevant disorder and those likely to develop the disorder due to genetic predisposition or other factors.

The terms "patient" and "subject" are used interchangeably, and mean any living organism that can be treated with the fusion protein of the present disclosure. In the present disclosure, the subject or individual receiving therapeutic or prophylactic treatment is preferably a mammal, such as but not limited to humans, non-human primates, livestock (e.g., sheep, cattle, horses, donkeys, and pigs), pets (e.g., dogs and cats), laboratory test animals (e.g., mice, rabbits, rats, guinea pigs, and hamsters), or captive wild animals (e.g., foxes and deer). The subject is preferably a primate. In certain embodiments, the subject is a mammal, preferably the subject is a human. In certain embodiments, the subject is an adult, a child, or an infant.

### Examples

To enable those skilled in the art to better understand solutions of the present disclosure, the technical solutions in the examples of the present disclosure will be described clearly and completely below. Obviously, the described examples are only part of the examples of the present disclosure, rather than all examples.

The meanings of the abbreviations are as follows: "h" means hour, "min" means minute, "s" means second, "µL" means microliter, "mL" means milliliter, "L" means liter, and "mM" means millimolar.

### Experimental methods

(1) Cell culture: African green monkey kidney Vero cells and CV-1 cells (both obtained from the Cell Bank of the Chinese Academy of Sciences) were incubated in DMEM medium or RPMI medium (Gibco) supplemented with 10% fetal bovine serum (FBS; Gibco) and 1% penicillin-streptomycin. Vero cells were used for virus titer determination, and CV-1 cells were used for large-scale virus production and cellular function assay. Cell growth and virus replication were both performed in a 37°C, CO₂ incubator.
(2) PCR technology: Viral genomic DNA was extracted with a viral genome extraction kit (purchased from Biomiga), and tissue DNA was extracted with the DNeasy^{®} Blood & Tissue kit (purchased from Qiagen). The specific operations referred to the instructions for use provided by the manufacturers. Using the extracted DNA as the template, PCR amplification was performed with the following primers using DNA polymerase (TAKARA): primers for J2R: TGTTAGATACATAGATCCTCGTCG and CACTTTCTGGTTCGTAATCTAACTC; primers for A44L-A45R-A46R: GTACTCTTACTGTGTAGTGG and CTCTATACATCTATGAGACTCC; and primers for A52R: TACGTTTACGGTATAGCCTC and TGCCGTAGCCAATGTAGTAATG. The PCR reaction procedure was as follows: pre-denaturation at 94°C for 5 min; 35 cycles of denaturation at 98°C for 10 s, annealing at 58°C for 30 s, and extension at 72°C for 1 min; and final sufficient extension at 72°C for 10 min, hold at 25°C. The PCR reaction was conducted on a PCR instrument (produced by Bio-Rad), and PCR amplification products were analyzed by agarose gel electrophoresis.
(3) Viral plaque assay (Vero cells): Positive control viruses and test viruses were taken out from a -80°C refrigerator and thawed within 5-7 min, respectively. During thawing, the samples were vortexed for 3-5 s and ultrasonically disrupted with a cup-type ultrasonic disruptor for 1 min (4-20°C, 20 s on/20 s off). The viruses were subjected to serial 10-fold gradient dilutions. In a biosafety cabinet, 800 µL of diluted virus samples per well was added to 6-well plates with Vero cells using a 1 mL pipette. Then, the 6-well plates were incubated at 37°C in a 5% CO₂ incubator for 1 h. The inoculum was discarded, and 1.5 mL of medium (DMEM + 10% FBS) and 1.5 mL of 3% carboxymethyl cellulose (CMC) solution were added to each well. The plates were incubated at 37°C in a 5% CO₂ incubator for 40 h. The cells were stained with 0.1% crystal violet staining solution. The 6-well plates were scanned with a scanner, and the number of plaques in each well was recorded (the number of plaques ranging from 30 to 300 per well was regarded as valid). Titer = (number of plaques / 0.8) × dilution factor, expressed in PFU/mL.

### Example 1: Construction and Engineering of Vaccinia Virus WC Strain

Using the genome of the vaccinia virus WR strain (ATCC VR-1354) as the backbone, the A34R gene sequence of the WR strain (WR A34R) was knocked out, and the A34R gene sequence (CoA34R; GenBank: AAA48161.1) of the vaccinia virus Copenhagen strain was knocked in, thereby obtaining a novel vaccinia virus strain, WC strain (Figure 3A). The amino acid sequences encoded by WR A34R and CoA34R are shown in Table 3. The exemplary operations were as follows: The shuttle plasmid pShuttleΔA34R-CoA34R was transfected into CV-1 cells in the presence of the transfection reagent Lipofectamine3000. After 4 h, the transfected CV-1 cells were infected with the vaccinia virus WR strain at 0.01 PFU per cell. After 48 h, the vaccinia viruses carrying CoA34R and expressing green fluorescent protein (WR-CoA34R GFP) were harvested and selected. The above recombinant viruses (with GFP tag) were used to infect CV-1 cells transfected with Cre expression plasmids (Cre expression plasmids were purchased from Sangon Biotech (Shanghai) Co., Ltd.). The GFP gene was removed via the Cre/loxP system, and the recombinant vaccinia virus WC strain with fluorescent tag deleted was thereby obtained. The comet formation assay results showed that the extracellular enveloped viruses (EEVs) and intracellular mature viruses (IMVs) of the WR strain formed round plaques upon infection of CV-1 cells, while the EEVs and IMVs of the WC strain formed comet-shaped plaques upon infection of CV-1 cells (Figure 3B). The titer determination results showed that at multiplicity of infection (MOI) = 1, after CV-1 cells were infected with EEVs of the WC strain for 12 h, the virus titer (1.50 × 10⁵ PFU/mL) was 148 times that of the WR strain (1.01 × 10³ PFU/mL); and at MOI = 2, after CV-1 cells were infected with EEVs of the WC strain for 12 h, the virus titer (1.91 × 10⁵ PFU/mL) was 106 times that of the WR strain (1.79 × 10³ PFU/mL) (Figure 3C).

Figure 3D shows a schematic diagram of the genetic engineering using the vaccinia virus WC strain as the vector, including modifications of six gene sequences: Sequence 1 (J2R), Sequence 2 (A34R), Sequences 3-5 (A44L-A45R-A46R), and Sequence 6 (A52R), as well as knock-in of heterologous nucleic acid sequences encoding fusion proteins.

### Example 2: Construction of Vectors

As shown in Figure 4, the shuttle plasmids include: a shuttle plasmid for gene knock-in of CoA34R (Figure 4A); shuttle plasmids for gene knockout of J2R (Figure 4Bi), A44L-A45R-A46R (Figure 4Bii), and A52R (Figure 4Biii); shuttle plasmids for simultaneous gene knockout of J2R and knock-in of heterologous nucleic acids encoding mouse HPGD (mHPGD) (Figure 4Ci), sHPGD (secreted human HPGD; Figure 4Cii), sIL12 (Figure 4Ciii), mIL12 I (Figure 4Civ), and mIL12 II (Figure 4Cv); shuttle plasmids for simultaneous gene knockout of A44L-A45R-A46R and knock-in of heterologous nucleic acids encoding sHPGD (Figure 4Di), sMICBH (secreted MICB peptide + hHPGD; Figure 4Dii), sMICAH (secreted MICA peptide + hHPGD; Figure 4Diii), smCTAH (secreted MICB peptide + multiple CTA peptides + hHPGD; Figures 4Div-v), and smPCTAH (secreted MICB peptide + multiple PCTA peptides + hHPGD; Figures 4Dvi-vii); and shuttle plasmids for simultaneous gene knockout of A52R and knock-in of heterologous nucleic acids encoding sHPGD (Figure 4Ei), sMICBH (Figure 4Eii), smCTAH (Figures 4Eiii-iv), and smPCTAH (Figures 4Ev-vi). The amino acid sequences of the fusion proteins encoded by the above heterologous nucleic acids are shown in Table 2 and Table 4; and the corresponding nucleotide sequences are shown in Table 6.

### (1) Shuttle plasmids expressing tumor antigen peptide fusion proteins

The following target genes for expression of tumor antigen peptide fusion proteins were designed: A44L left arm-pE/L-SP-hHPGD-loxP-H5-GFP-loxP-A46R right arm (Figure 4Di), A44L left arm-pE/L-SP-GGS-MICB peptide-AEAAAKEAAAKA-hHPGD-loxP-H5-GFP-loxP-A46R right arm (Figure 4Dii), A44L left arm-pE/L-SP-GGS-MICA peptide-AEAAAKEAAAKA-hHPGD-loxP-H5-GFP-loxP-A46R right arm (Figure 4Diii), A44L left arm-pE/L-SP-GGS-MICB peptide-AEAAAKEAAAKA-CTA₁ peptide-GG-CTA₂ peptide-GG...CTA₃₃ peptide-AEAAAKEAAAKA-hHPGD-loxP-H5-GFP-loxP-A46R right arm (Figure 4Div), A44L left arm-pE/L-SP-GGS-MICB peptide-AEAAAKEAAAKA-CTA₁ peptide-PP-CTA₂ peptide-PP...CTA₃₃ peptide-AEAAAKEAAAKA-hHPGD-loxP-H5-GFP-loxP-A46R right arm (Figure 4Dv), A44L left arm-pE/L-SP-GGS-MICB peptide-AEAAAKEAAAKA-PCTA₁ peptide-GG-PCTA₂ peptide-GG...PCTA₁₀ peptide-AEAAAKEAAAKA-hHPGD-loxP-H5-GFP-loxP-A46R right arm (Figure 4Dvi), A44L left arm-pE/L-SP-GGS-MICB peptide-AEAAAKEAAAKA-PCTA₁ peptide-PP-PCTA₂ peptide-PP...PCTA₁₀ peptide-AEAAAKEAAAKA-hHPGD-loxP-H5-GFP-loxP-A46R right arm (Figure 4Dvii), A52R left arm-pE/L-SP-hHPGD-loxP-H5-GFP-loxP-A52R right arm (Figure 4Ei), A52R left arm-pE/L-SP-GGS-MICB peptide-AEAAAKEAAAKA-hHPGD-loxP-H5-GFP-loxP-A52R right arm (Figure 4Eii), A52R left arm-pE/L-SP-GGS-MICB peptide-AEAAAKEAAAKA-CTA₁ peptide-GG-CTA₂ peptide-GG...CTA₃₃ peptide-AEAAAKEAAAKA-hHPGD-loxP-H5-GFP-loxP-A52R right arm (Figure 4Eiii), A52R left arm-pE/L-SP-GGS-MICB peptide-AEAAAKEAAAKA-CTA₁ peptide-PP-CTA₂ peptide-PP...CTA₃₃ peptide-AEAAAKEAAAKA-hHPGD-loxP-H5-GFP-loxP-A52R right arm (Figure 4Eiv), A52R left arm-pE/L-SP-GGS-MICB peptide-AEAAAKEAAAKA-PCTA₁ peptide-GG-PCTA₂ peptide-GG...PCTA₁₀ peptide-AEAAAKEAAAKA-hHPGD-loxP-H5-GFP-loxP-A52R right arm (Figure 4Ev), and A52R left arm-pE/L-SP-GGS-MICB peptide-AEAAAKEAAAKA-PCTA₁ peptide-PP-PCTA₂ peptide-PP...PCTA₁₀ peptide-AEAAAKEAAAKA-hHPGD-loxP-H5-GFP-loxP-A52R right arm (Figure 4Evi). After the target genes were synthesized, they were respectively cloned into pShuttle DNA (Sangon Biotech (Shanghai) Co., Ltd.) to obtain the shuttle plasmids expressing tumor antigen peptide fusion proteins.

### (2) Shuttle plasmids expressing IL12 p70 fusion proteins

The following target genes for expression of IL12 p70 fusion proteins were designed: J2R left arm-pE/L-SP-IL12 p40-GGGGSGGGGS-IL12 p35-loxP-H5-GFP-loxP-J2R right arm (Figure 4Ciii), J2R left arm-pE/L-SP-IL12 p40-GGGGSGGGGS-IL12 p35-transmembrane peptide (TP)-loxP-H5-GFP-loxP-J2R right arm (Figure 4Civ), and J2R left arm-pE/L-signal-anchor peptide (SAP)-IL12 p40-GGGGSGGGGS-IL12 p35-loxP-H5-GFP-loxP-J2R right arm (Figure 4Cv). After the target genes were synthesized, they were respectively cloned between two suitable multiple cloning sites of pShuttle DNA (Sangon Biotech (Shanghai) Co., Ltd.) to obtain the shuttle plasmids expressing IL12 p70 fusion proteins.

### Example 3: Vaccinia Virus Gene Knockout and Heterologous Nucleic Acid Sequence Knock-in via Shuttle Plasmids

(1) J2R gene knockout and heterologous nucleic acid knock-in: The shuttle plasmids obtained in Example 2, such as pShuttleΔJ2R (Figure 4Bi), pShuttleΔJ2R-HPGD (Figure 4Ci), pShuttleΔJ2R-sHPGD (Figure 4Cii), pShuttleΔJ2R-sIL12 (Figure 4Ciii), pShuttleΔJ2R-mIL12 I (Figure 4Civ), or pShuttleΔJ2R-mIL12 II (Figure 4Cv), were transfected into CV-1 cells with Lipofectamine3000. After 4 h, the cells were incubated with the vaccinia virus WR strain or the novel vaccinia virus WC strain constructed in Example 1 at 0.01 PFU per cell. After 48 h, the cells were harvested, and virus-infected cells expressing green fluorescent protein were selected under a fluorescence microscope for recombinant virus selection and small-scale amplification. A series of WR strain or WC strain recombinant viruses (with GFP tag) with J2R gene knockout and heterologous nucleic acid knock-in were obtained, such as WRΔJ2R-GFP, WRΔJ2R-HPGD-GFP, WRΔJ2R-sHPGD-GFP, WRΔJ2R-sIL12-GFP, WRΔJ2R-mIL12 I-GFP, WRΔJ2R-mIL12 II-GFP, and WCΔJ2R-mIL12 I-GFP. Viral genomic DNA was extracted, and J2R gene deletion was identified by PCR. Then, the above recombinant viruses (with GFP tag) were used to infect CV-1 cells transfected with Cre expression plasmids. The GFP gene was removed via the Cre/loxP system, and J2R-knockout recombinant viruses with fluorescent tags deleted were thereby obtained: WRΔJ2R, WRΔJ2R-HPGD, WRΔJ2R-sHPGD, WRΔJ2R-sIL12, WRΔJ2R-mIL12 I, WRΔJ2R-mIL12 II, and WCΔJ2R-mIL12 I.
(2) A44L-A45R-A46R gene knockout and heterologous nucleic acid knock-in: The shuttle plasmids obtained in Example 2, such as pShuttleΔ (A44L-A45R-A46R)-sHPGD (Figure 4Di), pShuttleΔ (A44L-A45R-A46R)-sMICBH (Figure 4Dii), pShuttleΔ (A44L-A45R-A46R)-sMICAH (Figure 4Diii), pShuttleΔ (A44L-A45R-A46R)-smCTAH (Figures 4Div-v), or pShuttleΔ (A44L-A45R-A46R)-smPCTAH (Figures 4Dvi-vii), were transfected into CV-1 cells with Lipofectamine3000. After 4 h, the cells were incubated with the J2R-knockout recombinant virus with fluorescent tags deleted WCΔJ2R-mIL12 I obtained in Step (1) above at 0.01 PFU per cell. After 48 h, the cells were harvested, and virus-infected cells expressing green fluorescent protein were selected under a fluorescence microscope for recombinant virus selection and small-scale amplification. Recombinant viruses (with GFP tag) with J2R and A44L-A45R-A46R gene knockout and heterologous nucleic acid knock-in were obtained. Viral genomic DNA was extracted, and A44L-A45R-A46R gene deletion was identified by PCR. Then, the above recombinant viruses (with GFP tag) were used to infect CV-1 cells transfected with Cre expression plasmids. The GFP gene was removed via the Cre/loxP system, and a series of recombinant viruses with J2R and A44L-A45R-A46R gene knockout and heterologous nucleic acid knock-in and with fluorescent tags deleted were thereby obtained: WCΔJ2RΔ(A44L-A45R-A46R)-mIL12 I/sHPGD (IVIR001), WCΔJ2RΔ(A44L-A45R-A46R)-mIL12 I/sMICBH (IVIR003), WCΔJ2RΔ(A44L-A45R-A46R)-mIL12 I/smCTAH (IVIR005), WCΔJ2RΔ(A44L-A45R-A46R)-mIL12 I/smPCTAH (IVIR007), and WCΔJ2RΔ (A44L-A45R-A46R)-mIL12 I/sMICAH, as shown in Figure 5A.
(3) A52R gene knockout and heterologous nucleic acid knock-in: The shuttle plasmids obtained in Example 2, pShuttleΔA52R (Figure 4Biii), pShuttleΔA52R-sHPGD (Figure 4Ei), pShuttleΔA452R-sMICBH (Figure 4Eii), pShuttleΔA52R-smCTAH (Figures 4Eiii-iv), or pShuttleΔA52R-smPCTAH (Figures 4Ev-vi), were transfected into CV-1 cells with Lipofectamine3000. After 4 h, the cells were incubated with the above recombinant viruses with fluorescent tags deleted, such as WCΔJ2RΔ(A44L-A45R-A46R)-mIL12 I/sHPGD (IVIR001), WCΔJ2RΔ(A44L-A45R-A46R)-mIL12 I/sMICBH (IVIR003), WCΔJ2RΔ(A44L-A45R-A46R)-mIL12 I/smCTAH (IVIR005), WCΔJ2RΔ(A44L-A45R-A46R)-mIL12 I/smPCTAH (IVIR007), or WCΔJ2RΔ(A44L-A45R-A46R)-mIL12 I/sMICAH, at 0.01 PFU per cell. After 48 h, the cells were harvested, and virus-infected cells expressing green fluorescent protein were selected under a fluorescence microscope for recombinant virus selection and small-scale amplification. Recombinant viruses (with GFP tag) with J2R, A44L-A45R-A46R and A52R gene knockout and heterologous nucleic acid knock-in were obtained. Viral genomic DNA was extracted, and A52R gene deletion was identified by PCR. Then, the above recombinant viruses (with GFP tag) with J2R, A44L-A45R-A46R and A52R gene knockout and heterologous nucleic acid knock-in were used to infect CV-1 cells transfected with Cre expression plasmids. The GFP gene was removed via the Cre/loxP system, and a series of recombinant viruses with J2R, A44L-A45R-A46R and A52R gene knockout and heterologous nucleic acid knock-in and with fluorescent tags deleted were thereby obtained: WCΔJ2RΔ (A44L-A45R-A46R)ΔA52R-mIL12 I/sHPGD (IVIR002), WCΔJ2RΔ (A44L-A45R-A46R)ΔA52R-mIL12 I/sMICBH (IVIR004), WCΔJ2RΔ (A44L-A45R-A46R)ΔA52R-mIL12 I/smCTAH (IVIR006), WCΔJ2RΔ (A44L-A45R-A46R)ΔA52R-mIL12 I/smPCTAH (IVIR008), WCΔJ2RΔ (A44L-A45R-A46R)ΔA52R-mIL12 I/sMICAH (IVIR009), WCΔJ2RΔA52R-mIL12 I/sHPGD (IVIR010), WCΔJ2RΔA52R-mIL12 I/sMICBH (IVIR011), WCΔJ2RΔA52R-mIL12 I/smCTAH (IVIR012), and WCΔJ2RΔA52R-mIL12 I/smPCTAH (IVIR013), as shown in Figure 5A.

### Example 4: Selection, Identification, and Amplification of Recombinant Viruses

(1) Selection of recombinant viruses: Virus-infected cells expressing green fluorescent protein were selected and transferred into cryotubes containing 200 µL of cell culture medium. After freezing-thawing, 20 µL of the freeze-thawed samples was diluted with cell culture medium and then added to 6-well plates pre-seeded with CV-1 cells for 24 h. Following another 48 h of incubation, the cells were observed under a microscope, and virus-infected cells expressing green fluorescent protein were selected for the next round of selection, until all infected cells were observed to emit fluorescence under a microscope, thereby obtaining recombinant viruses expressing green fluorescent protein. (For recombinant viruses with the GFP gene deleted, non-fluorescent viruses selected by the same method were the desired recombinant viruses.) Then, the cells were scraped and cryopreserved at -80°C for virus amplification.
(2) Identification of recombinant viruses: 20 µL of virus stock was added to 6-well plates pre-seeded with 5 × 10⁵ CV-1 cells for 24 h, and the plates were incubated for another 16 h. Using whole-cell lysates of CV-1 cells as protein samples, the expression of non-extracellular (intracellular and membrane-bound) IL12 p70 and HPGD proteins was detected by sodium dodecyl sulfate polyacrylamide gel electrophoresis (SDS-PAGE) and Western Blotting (WB) with commercial anti-IL12 p35 antibody (αIL12 p35, ab131039, Abcam) and anti-HPGD antibody (αHPGD, ab187161, Abcam); and the expression of IL12 p70 protein on the cell membrane was detected by flow cytometry with an anti-IL12 antibody (anti-mouse IL12, 560564, BD Biosciences).
(3) Amplification of recombinant viruses: 100 µL of the samples of cells infected with recombinant viruses with fluorescent tags deleted (any one of IVIR001 to IVIR013 obtained in Step (2) and Step (3) of Example 3) was freeze-thawed and then added to 6-well plates containing CV-1 cells. The cells were grown to 80%-90% confluence in 1.5 mL of cell culture medium. After 48 h, the cells and culture medium were scraped and preserved, and the "seed viruses" were thereby obtained. The "seed viruses" were rapidly freeze-thawed three times and diluted to an appropriate concentration. 100 µL of the above virus suspension per flask was added to T225 culture flasks containing CV-1 cells. After 48 h, the cells were harvested by pipetting and centrifuged repeatedly at 1500 rpm (4°C). The pellets were washed with PBS, resuspended in 10 mL of 10 mM Tris-HCl buffer (pH 9), and stored at -80°C for virus concentration.
(4) Concentration of recombinant viruses: The samples of the recombinant viruses amplified in Step (3) above were thawed, then vortexed for several seconds, and centrifuged at 1500 rpm (4°C) for 5 min. Supernatants (containing released viral particles) were collected and diluted with 10 mM Tris-HCl buffer to a total volume of 30 mL. Equal aliquots of 30 mL of the samples were dispensed into four Beckman ultracentrifuge tubes. Then, 17 mL of 36% sucrose solution was slowly added to the virus solution, and the tubes were centrifuged at 13500 rpm (4°C) for 80 min. The final pellets were resuspended in 1-4 mL of virus suspension buffer (PBS; 10% glycerol; 138 mM NaCl; pH 7.4) and stored at -80°C.
(5) WB assay: Cells infected with viruses (any one of IVIR001 to IVIR013 obtained in Step (2) and Step (3) of Example 3, as well as WRΔJ2R, WRΔJ2R-sIL12, WRΔJ2R-mIL12 I, and WRΔJ2R-mIL12 II obtained in Step (1) of Example 3) were harvested with cell scrapers. Total cellular protein was extracted using RIPA lysis buffer (Beyotime Biotech (Shanghai) Co., Ltd.) supplemented with PMSF and protease inhibitor cocktail (purchased from Applygen) via high-speed low-temperature centrifugation (14000 × g, 4°C, 15 min). According to the BCA method, the concentration of the protein samples was determined, and 20 µg of protein per well was subjected to SDS-PAGE. The separated target proteins were transferred from the electrophoresis gel to a PVDF membrane, followed by incubation with primary antibodies αIL12 p35 (ab131039, Abcam), αHPGD (ab187161, Abcam), or anti-β-actin antibody (C1313, Applygen) (35 rpm, 4°C, overnight), and further incubation with secondary antibodies HRP-labeled anti-mouse antibody (BA1050, BOSTER) and HRP-labeled anti-rabbit antibody (ab6721, Abcam) (35 rpm, room temperature, 2 h). Then, ECL luminescent solution (purchased from CWBIO) was dropwise added onto the protein membranes, and exposure and image acquisition were performed with an electronic film pressing imager (purchased from e-blot).
(6) Flow cytometry: CV-1 cells were infected with recombinant viruses at multiplicity of infection (MOI) = 0.5. After 16 h, the virus-infected cells were harvested by the TrypLE digestion method. The harvested cells were incubated with 5 µL of anti-IL12 antibody (anti-mouse IL12, 560564, BD Biosciences) for 30 min at 4°C in the dark. IL12 p70-positive cells were detected and analyzed with a flow cytometer.

Through virus recombination, selection, and identification, it was confirmed that 13 vaccinia virus WC strain recombinant viruses as shown in Figure 5A were obtained: IVIR001, IVIR002, and IVIR010 (all expressing mIL12 I and sHPGD); IVIR003, IVIR004, and IVIR011 (all expressing mIL12 I and sMICBH); IVIR005, IVIR006, and IVIR012 (all expressing mIL12 I and smCTAH); IVIR007, IVIR008, and IVIR013 (all expressing mIL12 I and smPCTAH); and IVIR009 (expressing mIL12 I and sMICAH).

Figure 5B shows fusion proteins expressed by recombinant viruses IVIR001 to IVIR013, wherein Figure 5Bi shows mIL12 I (type I membrane protein IL12 p70), comprising signal peptide, IL12 p40, (G₄S)₂ linker peptide, IL12 p35, and transmembrane peptide (TP); Figure 5Bii shows sHPGD, comprising signal peptide and human HPGD (hHPGD); Figure 5Biii shows sMICBH, comprising signal peptide, G₂S linker peptide, MICB peptide, AEAAAKEAAAKA linker peptide, and hHPGD; Figure 5Biv shows smCTAHa, comprising signal peptide, G₂S linker peptide, MICB peptide, AEAAAKEAAAKA linker peptide, CTA₁ peptide (cancer-testis antigen peptide 1), CTA₂ peptide, ..., CTA₃₃ peptide, AEAAAKEAAAKA linker peptide, and hHPGD, with GG as the linker peptide between CTA peptides; Figure 5Bv shows smCTAHb, comprising signal peptide, G₂S linker peptide, MICB peptide, AEAAAKEAAAKA linker peptide, CTA₁ peptide (cancer-testis antigen peptide 1), CTA₂ peptide, ..., CTA₃₃ peptide, AEAAAKEAAAKA linker peptide, and hHPGD, with PP as the linker peptide between CTA peptides; Figure 5Bvi shows smPCTAHa, comprising signal peptide, G₂S linker peptide, MICB peptide, AEAAAKEAAAKA linker peptide, PCTA₁ peptide (prostate carcinoma tumor antigen peptide 1), PCTA₂ peptide, ..., PCTA₁₀ peptide, AEAAAKEAAAKA linker peptide, and hHPGD, with GG as the linker peptide between PCTA peptides; Figure 5Bvii shows smPCTAHb, comprising signal peptide, G₂S linker peptide, MICB peptide, AEAAAKEAAAKA linker peptide, PCTA₁ peptide (prostate carcinoma tumor antigen peptide 1), PCTA₂ peptide, ..., PCTA₁₀ peptide, AEAAAKEAAAKA linker peptide, and hHPGD, with PP as the linker peptide between PCTA peptides; and Figure 5Bviii shows sMICAH, comprising signal peptide, G₂S linker peptide, MICA peptide, AEAAAKEAAAKA linker peptide, and hHPGD.

### Example 5: Identification of Vaccinia Virus Gene Knockout

At the molecular level, PCR technology was used for the identification of gene knockout of vaccinia virus. As shown in Figure 6, compared with the genome of the wild-type WR strain used as the template, when the genome of the J2R-knockout WR strain with GFP tag (WRΔJ2R-GFP) was used as the template, the electrophoretic position of the PCR product of J2R was higher (the GFP gene fragment is larger than the J2R gene); compared with the genome of the J2R-knockout WR strain (WRΔJ2R) used as the template, when the genome of the WR strain with J2R and A52R knockout and GFP tag (WRΔJ2RΔA52R-GFP) was used as the template, the electrophoretic position of the PCR product of A52R was lower (the GFP gene fragment is smaller than the A52R gene); and compared with the genome of WRΔJ2R used as the template, when the genome of the WR strain with J2R, A52R and A44L-A45R-A46R knockout and GFP tag (WRΔJ2RΔ(A44L-A45R-A46R)-GFP) was used as the template, the electrophoretic position of the PCR product of A44L-A45R-A46R was lower (the GFP gene fragment is smaller than the A44L-A45R-A46R gene).

### Example 6: Identification of IL12 p70 Fusion Protein Expression in Recombinant Viruses

At the molecular level, the expression of IL12 p70 fusion proteins as shown in Figure 7A was identified: secreted IL12 p70 (sIL12), type I membrane protein IL12 p70 (mIL12 I), and type II membrane protein IL12 p70 (mIL12 II). The WB results showed that IL12 p70 proteins were expressed after CV-1 cells were infected with the recombinant viruses WRΔJ2R-sIL12, WRΔJ2R-mIL12 I, and WRΔJ2R-mIL12 II for 16 h, respectively, while no IL12 p70 protein was expressed in cells of the control virus WRΔJ2R infection group (Figure 7B). The flow cytometry assay results indicated that after CV-1 cells were infected with the recombinant viruses WRΔJ2R-sIL12, WRΔJ2R-mIL12 I, and WRΔJ2R-mIL12 II for 16 h, respectively, the proportions of IL12 p70-positive cells were 4.15%, 96.7%, and 38.9%, respectively (Figure 7C).

### Example 7: Functional Validation of Secreted Human HPGD Expressed by Recombinant Viruses

Enzyme-linked immunosorbent assay (ELISA) was used to determine the secretion levels of prostaglandin E2 (PGE₂) in mouse lung cancer LLC cells infected with recombinant viruses IVIR006a, IVIR008a, the WR strain recombinant virus expressing intracellular mouse HPGD prepared in Step (1) of Example 3 (WRΔJ2R-HPGD), and control virus WRΔJ2R, respectively.
(1) Collection of cell culture medium samples: LLC cells were infected with recombinant viruses at multiplicity of infection (MOI) = 1. Cell culture medium was collected at 24 h after infection. 20 mM arachidonic acid (purchased from Sigma-Aldrich) was added for treatment for 4 h at 4 h before the collection of cell culture medium. Any cell debris in the cell culture medium was removed by centrifugation (1500 rpm, 5 min) for later use.
(2) ELISA assay: The concentration of PGE₂ in cell culture medium was determined with a 96-well PGE₂ ELISA kit (purchased from Caymen), and the specific operation procedures referred to the manufacturer's instructions. Both samples and standards were set in quadruplicate. The absorbance value of each well was read at a wavelength of 412 nm, and the concentration of PGE₂ was determined from the standard curve.

The results are shown in Figure 8. The concentrations of PGE₂ in cell culture medium after 24 h were 257 pg/mL in the WRΔJ2R-HPGD infection group, 142 pg/mL in the IVIR006a infection group, and 123 pg/mL in the IVIR008a infection group. The concentrations of PGE₂ in the above three groups were all lower than the concentration of PGE₂ in the WRΔJ2R infection group (1561 pg/mL), and the difference was statistically significant (one-way ANOVA, p-value < 0.0001). In contrast, there was no statistically significant difference in the concentration of PGE₂ between the WRΔJ2R infection group (1561 pg/mL) and the virus-uninfected cell group (Cell only group; 1611 pg/mL) (P = 0.9886). The above results indicate that compared with the intracellular HPGD expressed by WRΔJ2R-HPGD, the secreted HPGD expressed by IVIR006a and IVIR008a exhibits a more obvious degradation effect on PGE₂.

### Example 8: Identification of Fusion Protein Expression in Recombinant Viruses

The fusion proteins expressed after CV-1 cells were infected with recombinant virus IVIR004 (expressing sMICBH and mIL12 I, Figure 9A) for 16 h were detected by WB and flow cytometry assays. The WB results (Figure 9B) showed that HPGD protein was expressed in cells of the IVIR004 infection group, while no HPGD protein was expressed in cells of the control virus WRΔJ2R infection group. The flow cytometry results (Figure 9C) showed that the proportion of IL12 p70-positive cells in cells of the IVIR004 infection group was 94.8%. The WB results (Figure 9D) showed that IL12 p70 protein was expressed in cells of the IVIR004 infection group, while no IL12 p70 protein was expressed in cells of the control virus WRΔJ2R infection group.

### Example 9: Effect of Recombinant Viruses as Therapeutic Cancer Vaccines on Immune Function of Mouse Splenocytes

At the animal level, IFNγ-secreting splenocytes were detected by ELISpot assay in splenocytes derived from mice injected with recombinant viruses.
(1) Acquisition of splenocytes: Female Balb/c or C57BL/6N mice aged 6-8 weeks with normal immunity (purchased from Beijing Vital River Laboratory Animal Technology Co., Ltd.) were selected. At a position about 0.3-0.5 cm on one side of the intersection of the abdominal midline and the line connecting the two thigh roots of mice, 100 µL of recombinant viruses (IVIR002, IVIR004, and control virus WRΔJ2R) was intraperitoneally injected with a syringe needle at a dose of 5 × 10⁶ PFU per mouse, with 6 mice in each group. On Day 7 and Day 14 after injection, 3 mice were selected respectively for spleen harvesting. After the spleens were removed, photographing and splenocyte preparation were performed: the spleens were cut into pieces, ground, and lysed with RBC lysis buffer in sequence, then resuspended in RPMI complete medium + 5% FBS, and 10 µL was taken for cell counting.
(2) Preparation of immunostimulants: CV-1 cells were infected with recombinant viruses at MOI = 1, respectively. After 24 h, the cells were scraped, freeze-thawed three times, and centrifuged at 4000 rpm for 6 min. The supernatants were collected and subjected to inactivation treatment (ultraviolet irradiation for 2.5 min/ultrasonic treatment once for 30 s/ultraviolet irradiation for 2.5 min), and inactivated viruses were thereby obtained for use as stimulants.
(3) ELISpot assay: Mouse splenocytes were seeded in 96-well plates pre-coated with IFNγ (the number of splenocytes was set at 5 × 10⁵ or 3 × 10⁵ cells per well), with 3 replicate wells set for each group. 10 µL of stimulant per well was added to 96-well plates containing splenocytes derived from mice of different injection groups. After incubation in a 37°C incubator for 60 h, the cells were removed, and antibody incubation, color development, and IFNγ spot counting were performed in accordance with the operating procedures in the instructions of the mouse IFNγ ELISpot kit (Beijing Dakewe Biotech Co., Ltd.). As a cytokine secreted by immunocompetent cells, IFNγ plays an important immunomodulatory role in inducing antiviral immunity, including activating T cells, NK cells, phagocytes, etc. Each spot represents one IFNγ-secreting cell. The number of spots reflects the level of immune response of immune cells in the sample, and more spots indicate a higher level of cellular immune response.

IFNγ ELISpot assay was used to detect IFNγ-positive cells in splenocytes of female Balb/c mice at 14 days after intraperitoneal injection of recombinant virus IVIR004 (expressing MICB peptide, w/ MICB peptide), IVIR002 (not expressing MICB peptide, w/o MICB peptide), or control virus WRΔJ2R.

IFNγ ELISpot assay results (Figure 10A) showed that IFNγ spots were detected in splenocytes of mice in the IVIR004 injection group and the IVIR002 injection group. Quantitative analysis (Figure 10B) showed that the number of IFNγ spots formed per 5 × 10⁵ MICB peptide-specific mouse splenocytes was statistically different from that of the control group (unpaired t-test, P < 0.01). The results indicate that IVIR004 expressing MICB peptide can significantly induce the proliferation of MICB peptide-specific antigen-specific immune cells secreting IFNγ. These immune cells can specifically kill tumor cells expressing relevant tumor antigens without affecting normal tissue cells.

### Example 10: Identification of Fusion Protein Expression in Recombinant Viruses

The fusion proteins expressed after CV-1 cells were infected with recombinant virus IVIR006a (expressing smCTAH and mIL12 I, Figure 11A) for 16 h were detected by WB and flow cytometry assays. The WB results (Figure 11B) showed that HPGD protein was expressed in cells of the IVIR006a infection group, while no HPGD protein was expressed in cells of the control virus WRΔJ2R infection group. The flow cytometry results (Figure 11C) showed that the proportion of IL12 p70-positive cells in cells of the IVIR006a infection group was 90.8%. The WB results (Figure 11D) also showed that IL12 p70 protein was expressed in cells of the IVIR006a infection group, while no IL12 p70 protein was expressed in cells of the control virus WRΔJ2R infection group.

### Example 11: Effect of Recombinant Viruses as Therapeutic Cancer Vaccines on Immune Function of Mouse Splenocytes

At the animal level, IFNγ-secreting splenocytes were detected by ELISpot assay in splenocytes derived from mice injected with recombinant viruses. The experimental methods referred to Example 9.

IFNγ ELISpot assay was used to detect IFNγ-positive cells in splenocytes of female C57BL/6N mice at 14 days after intraperitoneal injection of recombinant virus IVIR006a (expressing MICB peptide and CTA peptides, w/ MICB peptide + CTA peptides), IVIR002 (not expressing MICB peptide and CTA peptides, w/o MICB peptide + CTA peptides), or control virus WRΔJ2R.

IFNγ ELISpot assay results (Figure 12A) showed that IFNγ spots were detected in splenocytes of mice in the IVIR006a injection group and the IVIR002 injection group. Quantitative analysis (Figure 12B) showed that the number of IFNγ spots formed per 5 × 10⁵ MICB peptide- and CTA peptide-specific mouse splenocytes was statistically different from that of the control group (unpaired t-test, p-value < 0.0001). The above results indicate that IVIR006a expressing MICB peptide and CTA peptides can significantly induce the proliferation of MICB peptide- and CTA peptide-specific antigen-specific immune cells secreting IFNγ. These immune cells can specifically kill tumor cells expressing relevant tumor antigens without affecting normal tissue cells.

### Example 12: Effect of Recombinant Viruses as Therapeutic Cancer Vaccines on Tumor Growth of Lung Cancer and Colon Cancer in Mice

The growth of xenografted tumor cells in mice and the effect of recombinant viruses as therapeutic cancer vaccines on tumor growth were monitored with a xenograft tumor model.

Female C57BL/6N mice aged 6-8 weeks (purchased from Beijing Vital River Laboratory Animal Technology Co., Ltd.) were selected and raised for one week, and then the inguinal region of the left hind limb was shaved. After 2 days, 100 µL of lung cancer LLC cell suspension (containing 5 × 10⁵ LLC cells) or 50 µL of colon cancer MC38 cell suspension (containing 5 × 10⁵ MC38 cells) was subcutaneously injected into the shaved area to develop a spherical tumor mass. LLC cells were purchased from Wuhan Procell Life Science & Technology Co., Ltd., and MC38 cells were purchased from Shanghai Yaji Biological Co., Ltd. The cell suspension was prepared by suspending tumor cells in PBS. Before subcutaneous implantation, cell counting and mycoplasma detection were performed. Cells suitable for implantation were those in the logarithmic growth phase (70-80% confluence) and free of mycoplasma and bacterial contamination. At 10 or 13 days after implantation, tumor size was measured, and mice with appropriate tumor size were randomly divided into groups, with 9-10 mice in each group.

On Day 13 and Day 16 after LLC cell implantation, recombinant virus IVIR001 (expressing sHPGD and mIL12 I) or IVIR006a (expressing smCTAH and mIL12 I) was used for treating lung cancer tumors, respectively (Figure 13A). 100 µL of virus suspension (containing 1 × 10⁷ PFU of virus) or an equal volume of PBS was administered by intratumoral (IT) injection. Starting from Day 13 after LLC cell implantation, tumor volume was monitored by caliper measurement twice a week. The tumor volume calculated as an ellipsoid was expressed as: tumor volume = π/6 × L (long diameter) × W (short diameter)². Animals were euthanized when the tumor volume was larger than 1400 mm³. In the treatment of the LLC xenograft tumor model, the statistically significant reduction of tumor growth rate indicated the tumor therapeutic advantage of therapeutic cancer vaccines. Survival data were compared with GraphPad software, and the significance of survival differences was determined with the Log-rank (Mantel Cox) test. Significance was determined with the unpaired Student's t-test (*p-value < 0.05). Tumor growth curves were plotted from tumor volume. The results (Figure 13A) showed that the tumor growth curves of the IVIR006a treatment group and the IVIR001 treatment group were lower than that of the control group (equal volume of PBS); and on Day 24 after cell implantation, the tumor volumes of the IVIR006a treatment group and the IVIR001 treatment group were significantly smaller than that of the control group (p-value < 0.05).

On Day 10 and Day 13 after MC38 cell implantation, recombinant virus IVIR006a or control virus WRΔJ2R was used for treating colon cancer tumors, respectively (Figure 13B). 50 µL of virus suspension (containing 5 × 10⁶ PFU of virus) or an equal volume of PBS was administered by intratumoral (IT) injection. Starting from Day 10 after MC38 cell implantation, tumor volume was monitored by caliper measurement twice a week. For the treatment of the MC38 xenograft tumor model, the calculation of tumor volume, plotting of tumor growth curves, and determination of significance (*p-value < 0.05) were all referred to the above treatment of the LLC xenograft tumor model. The results (Figure 13B) showed that the tumor growth curve of the IVIR006a treatment group were lower than those of the control virus WRΔJ2R treatment group and the control group (equal volume of PBS); and on Day 23 and Day 27 after cell implantation, the tumor volume of the IVIR006a treatment group was significantly smaller than that of the control virus WRΔJ2R treatment group (p-value < 0.05).

### Example 13: Identification of Fusion Protein Expression in Recombinant Viruses

The fusion proteins expressed after CV-1 cells were infected with recombinant virus IVIR008a (expressing smPCTAH and mIL12 I, Figure 14A) for 16 h were detected by WB and flow cytometry assays. The WB results (Figure 14B) showed that HPGD protein was expressed in cells of the IVIR008a infection group, while no HPGD protein was expressed in cells of the control virus WRΔJ2R infection group. The flow cytometry results (Figure 14C) showed that the proportion of IL12 p70-positive cells in cells of the IVIR008a infection group was 91.4%. The WB results (Figure 14D) also showed that IL12 p70 protein was expressed in cells of the IVIR008a infection group, while no IL12 p70 protein was expressed in cells of the control virus WRΔJ2R infection group.

### Example 14: Effect of Recombinant Viruses as Therapeutic Cancer Vaccines on Immune Function of Mouse Splenocytes

At the animal level, IFNγ-secreting splenocytes were detected by ELISpot assay in splenocytes derived from mice injected with recombinant viruses. The experimental methods referred to Example 9.

IFNγ ELISpot assay was used to detect IFNγ-positive cells in splenocytes of female Balb/c mice at 14 days after intraperitoneal injection of recombinant virus IVIR008a (expressing MICB peptide and PCTA peptides, w/ MICB peptide + PCTA peptides), IVIR002 (not expressing MICB peptide and PCTA peptides, w/o MICB peptide + PCTA peptides), or control virus WRΔJ2R.

IFNγ ELISpot assay results (Figure 15A) showed that IFNγ spots were detected in splenocytes of mice in the IVIR008a injection group and the IVIR002 injection group. Quantitative analysis (Figure 15B) showed that the number of IFNγ spots formed per 3 × 10⁵ MICB peptide- and PCTA peptide-specific mouse splenocytes was statistically different from that of the control group (unpaired t-test, p-value < 0.01). The results indicate that IVIR008a expressing MICB peptide and PCTA peptides can significantly induce the proliferation of MICB peptide- and PCTA peptide-specific antigen-specific immune cells secreting IFNγ. These immune cells can specifically kill tumor cells expressing relevant tumor antigens without affecting normal tissue cells.

### Example 15: Identification of Fusion Protein Expression in Recombinant Viruses

The fusion proteins expressed after CV-1 cells were infected with recombinant virus IVIR009 (expressing sMICAH and mIL12 I, Figure 16A) for 16 h were detected by WB assay. The WB results (Figure 16B) showed that HPGD protein was expressed in cells of the IVIR009 infection group, while no HPGD protein was expressed in cells of the control virus WRΔJ2R infection group and the virus-uninfected cell group (Cell only group). The WB results (Figure 16C) also showed that IL12 p70 protein was expressed in cells of the IVIR009 infection group, while no IL12 p70 protein was expressed in cells of the control virus WRΔJ2R infection group and the virus-uninfected cell group (Cell only group).

### Example 16: Effect of Recombinant Viruses as Therapeutic Cancer Vaccines on Immune Function of Mouse Splenocytes

At the animal level, IFNγ-secreting splenocytes were detected by ELISpot assay in splenocytes derived from mice injected with recombinant viruses. The experimental methods referred to Example 9.

IFNγ ELISpot assay was used to detect IFNγ-positive cells in splenocytes of female Balb/c mice at 14 days after intraperitoneal injection of IVIR009 (expressing MICA peptide, w/ MICA peptide), IVIR002 (not expressing MICA peptide, w/o MICA peptide), or control virus WRΔJ2R.

IFNγ ELISpot assay results (Figure 17A) showed that IFNγ spots were detected in splenocytes of mice in the IVIR009 injection group and the IVIR002 injection group. Quantitative analysis (Figure 17B) showed that the number of IFNγ spots formed per 5 × 10⁵ MICA peptide-specific mouse splenocytes was statistically different from that of the control group (unpaired t-test, p-value < 0.01). The above results indicate that IVIR009 expressing MICA peptide can significantly induce the proliferation of MICA peptide-specific antigen-specific immune cells secreting IFNγ. These immune cells can specifically kill tumor cells expressing relevant tumor antigens without affecting normal tissue cells.

### Example 17: Identification of Fusion Protein Expression in Recombinant Viruses

IVIR006 includes two subtypes, a and b. The commonality between the two subtypes is that both express secreted fusion protein of MICB peptide, multiple cancer-testis antigen peptides, and human HPGD (secreted MICB peptide + multiple CTA peptides + hHPGD, smCTAH), and type I membrane protein IL12 p70 (mIL12 I); and the difference lies in the distinct linker peptides between CTA peptides, wherein GG linker peptide is used in IVIR006a (Figure 11A), and PP linker peptide is used in IVIR006b (Figure 18A). The fusion proteins expressed after CV-1 cells were infected with recombinant virus IVIR006b for 16 h were detected by WB and flow cytometry assays. The WB results (Figures 18B and 18D) showed that HPGD protein and IL12 p70 protein were expressed in cells of the IVIR006b infection group, while no HPGD protein and IL12 p70 protein were expressed in cells of the control virus WRΔJ2R infection group. The flow cytometry results (Figure 18C) showed that the proportion of IL12 p70-positive cells in cells of the IVIR006b infection group was 90.6%.

IVIR008 includes two subtypes, a and b. The commonality between the two subtypes is that both express secreted fusion protein of MICB peptide, multiple prostate carcinoma tumor antigen peptides, and human HPGD (secreted MICB peptide + multiple PCTA peptides + hHPGD, smPCTAH), and type I membrane protein IL12 p70 (mIL12 I); and the difference lies in the distinct linker peptides between PCTA peptides, wherein GG linker peptide is used in IVIR008a (Figure 14A), and PP linker peptide is used in IVIR008b (Figure 19A). The fusion proteins expressed after CV-1 cells were infected with recombinant virus IVIR008b for 16 h were detected by WB and flow cytometry assays. The WB results (Figures 19B and 19D) showed that HPGD protein and IL12 p70 protein were expressed in cells of the IVIR008b infection group, while no HPGD protein and IL12 p70 protein were expressed in cells of the control virus WRΔJ2R infection group. The flow cytometry results (Figure 19C) showed that the proportion of IL12 p70-positive cells in cells of the IVIR008b infection group was 86.9%.

### Example 18: Identification of Fusion Protein Expression in Recombinant Viruses

The fusion proteins expressed after CV-1 cells were infected with recombinant viruses IVIR010, IVIR011, IVIR012, and IVIR013 (all expressing mIL12 I and expressing sHPGD, sMICBH, smCTAH, and smPCTAH, respectively, Figure 20A) for 16 h were detected by WB and flow cytometry assays. IVIR012 includes two subtypes, a and b. The commonality between the two subtypes is that both express smCTAH and mIL12 I; and the difference lies in the distinct linker peptides between CTA peptides, wherein GG linker peptide is used in IVIR012a, and PP linker peptide is used in IVIR012b. IVIR013 includes two subtypes, a and b. The commonality between the two subtypes is that both express smPCTAH and mIL12 I; and the difference lies in the distinct linker peptides between PCTA peptides, wherein GG linker peptide is used in IVIR013a, and PP linker peptide is used in IVIR013b. The WB results (Figures 20B and 20C) showed that HPGD protein and IL12 p70 protein were expressed in cells of the infection groups of IVIR010, IVIR011, IVIR012a, IVIR012b, IVIR013a, and IVIR013b, while no HPGD protein and IL12 p70 protein were expressed in cells of the control virus WRΔJ2R infection group. The flow cytometry results (Figure 20D) showed that the proportions of IL12 p70-positive cells in cells of the infection groups of IVIR011, IVIR012b, and IVIR013b were 82.4%, 78.0%, and 84.9%, respectively.

### Example 19: Effect of Recombinant Viruses as Therapeutic Cancer Vaccines on Immune Function of Mouse Splenocytes

At the animal level, IFNγ-secreting splenocytes were detected by ELISpot assay in splenocytes derived from mice injected with recombinant viruses. The experimental methods referred to Example 9.

IFNγ ELISpot assay was used to detect IFNγ-positive cells in splenocytes of female Balb/c mice at 7 days after intraperitoneal injection of IVIR011 (expressing MICB peptide, w/ MICB peptide), IVIR012b (expressing MICB peptide and CTA peptides, w/ MICB peptide + CTA peptides), IVIR013b (expressing MICB peptide and PCTA peptides, w/ MICB peptide + PCTA peptides), IVIR010 (not expressing MICB peptide, MICB peptide and CTA peptides, or MICB peptide and PCTA peptides, w/o MICB peptide, MICB peptide + CTA peptides, or MICB peptide + PCTA peptides), or control virus WRΔJ2R.

IFNγ ELISpot assay results (Figure 21A) showed that IFNγ spots were detected in splenocytes of mice in the injection groups of IVIR010, IVIR011, IVIR012b, and IVIR013b. Quantitative analysis (Figure 21B) showed that the numbers of IFNγ spots formed per 5 × 10⁵ MICB peptide-, MICB peptide- and CTA peptide-, and MICB peptide- and PCTA peptide-specific mouse splenocytes were all statistically different from that of the control group (unpaired t-test, p-value < 0.05, p-value < 0.01, and p-value < 0.0001). The above results indicate that IVIR011, IVIR012b, and IVIR013b can significantly induce the proliferation of the corresponding antigen-specific immune cells secreting IFNγ. These immune cells can specifically kill tumor cells expressing relevant tumor antigens without affecting normal tissue cells.

The above results indicate that linker peptides (GG and PP) between tumor antigen peptides can both be used for the design of tumor antigen peptide fusion proteins.

### Incorporation by Reference

The entire contents of each patent and scientific document referred to herein are incorporated herein by reference for all purposes.

### Equivalents

The present disclosure may be embodied in other specific forms without departing from its spirit or essential characteristics. The foregoing embodiments are therefore to be considered in all respects illustrative rather than limiting the invention described herein. Scope of the present disclosure is thus indicated by the appended claims rather than by the foregoing description, and all changes that come within the meaning and range of equivalency of the claims are intended to be embraced therein.

### Sequence Listing

**Table 1. Amino acid sequences**

| Protein | Source | Sequence | **SEQ ID NO.** |
|---|---|---|---|
| HPGD | Mouse | MHVNGKVALVTGAAQGIGKAFAEALLLHGAKVALVDW NLEAGVKCKAALDEQFEPQKTLFVQCDVADQKQLRDTF RKVVDHFGRLDILVNNAGVNNEKNWEQTLQINLVSVISG TYLGLDYMSKQNGGEGGIIINMSSLAGLMPVAQQPVYCA SKHGIIGFTRSAAMAANLMKSGVRLNVICPGFVDTPILESI EKEENMGQYIEYKDQIKAMMKFYGVLHPSTIANGLINLIE DDALNGAIMKITASKGIHFQDYDISPLLVKAPLTS | 1 |
| HPGD | Human | | 2 |
| IL-12 p40 | Mouse | MCPQKLTISWFAIVLLVSPLMAMWELEKDVYVVEVDWT PDAPGETVNLTCDTPEEDDITWTSDQRHGVIGSGKTLTIT VKEFLDAGQYTCHKGGETLSHSHLLLHKKENGIWSTEIL KNFKNKTFLKCEAPNYSGRFTCSWLVQRNMDLKFNIKSS SSSPDSRAVTCGMASLSAEKVTLDQRDYEKYSVSCQEDV TCPTAEETLPIELALEARQQNKYENYSTSFFIRDIIKPDPPK NLQMKPLKNSQVEVSWEYPDSWSTPHSYFSLKFFVRIQR KKEKMKETEEGCNQKGAFLVEKTSTEVQCKGGNVCVQA QDRYYNSSCSKWACVPCRVRS | 3 |
| IL-12 p35 | Mouse | MVSVPTASPSASSSSSQCRSSMCQSRYLLFLATLALLNHL SLARVIPVSGPARCLSQSRNLLKTTDDMVKTAREKLKHY SCTAEDIDHEDITRDQTSTLKTCLPLELHKNESCLATRETS STTRGSCLPPQKTSLMMTLCLGSIYEDLKMYQTEFQAINA ALQNHNHQQIILDKGMLVAIDELMQSLNHNGETLRQKPP VGEADPYRVKMKLCILLHAFSTRVVTINRVMGYLSSA | 4 |
| IL-12 p40 | Human | MCHQQLVISWFSLVFLASPLVAIWELKKDVYVVELDWYP DAPGEMVVLTCDTPEEDGITWTLDQSSEVLGSGKTLTIQV KEFGDAGQYTCHKGGEVLSHSLLLLHKKEDGIWSTDILK DQKEPKNKTFLRCEAKNYSGRFTCWWLTTISTDLTFSVK SSRGSSDPQGVTCGAATLSAERVRGDNKEYEYSVECQED SACPAAEESLPIEVMVDAVHKLKYENYTSSFFIRDIIKPDP PKNLQLKPLKNSRQVEVSWEYPDTWSTPHSYFSLTFCVQ VQGKSKREKKDRVFTDKTSATVICRKNASISVRAQDRYY SSSWSEWASVPCS | 5 |
| IL-12 p35 | Human | MWPPGSASQPPPSPAAATGLHPAARPVSLQCRLSMCPAR SLLLVATLVLLDHLSLARNLPVATPDPGMFPCLHHSQNLL RAVSNMLQKARQTLEFYPCTSEEIDHEDITKDKTSTVEAC LPLELTKNESCLNSRETSFITNGSCLASRKTSFMMALCLSS IYEDLKMYQVEFKTMNAKLLMDPKRQIFLDQNMLAVIDE LMQALNFNSETVPQKSSLEEPDFYKTKIKLCILLHAFRIRA VTIDRVMSYLNAS | 6 |
| Signal peptide | | MTRLTVLALLAGLLASSRA | 7 |
| Signal-anchor peptide | | MYGTIAVIVFFLIGFMIGYLGY | 63 |
| Transmembrane peptide | | FWVLVVVGGVLACYSLLVTVAFIIFWV | 64 |
| Multiple CTA peptides - a | | MLMAQEALAFLGGAARAVFLALGGFLWGPRAYAGGYR PRPRRYGGYYWPRPRRYGGEADPTGHSYGGSLFRAVITK GGNYKHCFPEIGGEVYDGREHSAGGREPVTKAEMLGGD PARYEFLWGGSAFPTTINFGGSAYGEPRKLGGKMVELVH FLGGYLQLVFGIEVGGEYLQLVFGIGGEVDPIGHLYGGFL WGPRALVGGTFPDLESEFGGIMPKAGLLIGGMEVDPIGHL YGGWQYFFPVIFGGGVYDGREHTVGGMVKISGGPRGGE VDPIGHVYGGGLYDGMEHLGGVRIGHLYILGGILLRDAG LVGGSLLMWITQCFLGGSLLMWITQCGGQLSLLMWITGG MPFATPMEAGGASGPGGGAPR | 8 |
| Multiple CTA peptides - b | | MLMAQEALAFLPPAARAVFLALPPFLWGPRAYAPPYRP RPRRYPPYYWPRPRRYPPEADPTGHSYPPSLFRAVITKPP NYKHCFPEIPPEVYDGREHSAPPREPVTKAEMLPPDPAR YEFLWPPSAFPTTINFPPSAYGEPRKLPPKMVELVHFLPP | 9 |
| | | YLQLVFGIEVPPEYLQLVFGIPPEVDPIGHLYPPFLWGPRA LVPPTFPDLESEFPPIMPKAGLLIPPMEVDPIGHLYPPWQY FFPVIFPPGVYDGREHTVPPMVKISGGPRPPEVDPIGHVY PPGLYDGMEHLPPVRIGHLYILPPILLRDAGLVPPSLLMW ITQCFLPPSLLMWITQCPPQLSLLMWITPPMPFATPMEAP PASGPGGGAPR | |
| Multiple PCTA peptides - a | | ILLWQPIPVGGILLWQPIPVGGILLWQPIPVGGILLWQPIPV GGILLWQPIPVGGILLWQPIPVGGILLWQPIPVGGILLWQPI PVGGILLWQPIPVGGILLWQPIPV | 10 |
| Multiple PCTA peptides - b | | ILLWQPIPVPPILLWQPIPVPPILLWQPIPVPPILLWQPIPVP PILLWQPIPVPPILLWQPIPVPPILLWQPIPVPPILLWQPIPV PPILLWQPIPVPPILLWQPIPV | 11 |

| | | | |
|---|---|---|---|
| Notes: The underlined regions are peptide fragments for preparing fusion proteins, and the italicized GG and PP are linker peptides. | | | |

**Table 2. Amino acid sequences of tumor antigen peptide fusion proteins**

| Fusion protein | Sequence | **SEQ ID NO.** |
|---|---|---|
| sHPGD (human) | MTRLTVLALLAGLLASSRAMHVNGKVALVTGAAQGIGRAFAEALLL KGAKVALVDWNLEAGVQCKAALDEQFEPQKTLFIQCDVADQQQ LRDTFRKVVDHFGRLDILVNNAGVNNEKNWEKTLQINLVSVISGT YLGLDYMSKQNGGEGGIIINMSSLAGLMPVAQQPVYCASKHGIV GFTRSAALAANLMNSGVRLNAICPGFVNTAILESIEKEENMGQYIE YKDHIKDMIKYYGILDPPLIANGLITLIEDDALNGAIMKITTSKGIH FQDYDTTPFQAKTQ | 12 |
| sMICAH | MTRLTVLALLAGLLASSRAGGSRTVPPMVNVTRSEASEGNITVTCRA SGFYPWNITLSWRQDGVSLSHDTQQWGDVLPDGNGTYQTWVAT RICQGEEQRFTCYMEHSGNHSTHPVPSGKVLVLQSHWAEAAAKE AAAKAMHVNGKVALVTGAAQGIGRAFAEALLLKGAKVALVDW NLEAGVQCKAALDEQFEPQKTLFIQCDVADQQQLRDTFRKVVDH FGRLDILVNNAGVNNEKNWEKTLQINLVSVISGTYLGLDYMSKQ NGGEGGIIINMSSLAGLMPVAQQPVYCASKHGIVGFTRSAALAAN LMNSGVRLNAICPGFVNTAILESIEKEENMGQYIEYKDHIKDMIKY YGILDPPLIANGLITLIEDDALNGAIMKITTSKGIHFQDYDTTPFQA KTQ | 13 |
| sMICBH | MTRLTVLALLAGLLASSRAGGSRTVPPMVNVTCSEVSEGNITVTCRA SSFYPRNITLTWRQDGVSLSHNTQQWGDVLPDGNGTYQTWVAT RIRQGEEQRFTCYMEHSGNHGTHPVPSGKALVLQSQRTDAEAAA KEAAAKAMHVNGKVALVTGAAQGIGRAFAEALLLKGAKVALV DWNLEAGVQCKAALDEQFEPQKTLFIQCDVADQQQLRDTFRKV VDHFGRLDILVNNAGVNNEKNWEKTLQINLVSVISGTYLGLDYM SKQNGGEGGIIINMSSLAGLMPVAQQPVYCASKHGIVGFTRSAAL AANLMNSGVRLNAICPGFVNTAILESIEKEENMGQYIEYKDHIKD MIKYYGILDPPLIANGLITLIEDDALNGAIMKITTSKGIHFQDYDTT PFQAKTQ | 14 |
| smPCTAHa | MTRLTVLALLAGLLASSRAGGSRTVPPMVNVTCSEVSEGNITVTCRA SSFYPRNITLTWRQDGVSLSHNTQQWGDVLPDGNGTYQTWVAT RIRQGEEQRFTCYMEHSGNHGTHPVPSGKALVLQSQRTDAEAAA KEAAAKAILLWQPIPVGGILLWQPIPVGGILLWQPIPVGGILLWQPI PVGGILLWQPIPVGGILLWQPIPVGGILLWQPIPVGGILLWQPIPVG GILLWQPIPVGGILLWQPIPVAEAAAKEAAAKAMHVNGKVALVT GAAQGIGRAFAEALLLKGAKVALVDWNLEAGVQCKAALDEQFE PQKTLFIQCDVADQQQLRDTFRKVVDHFGRLDILVNNAGVNNEK NWEKTLQINLVSVISGTYLGLDYMSKQNGGEGGIIINMSSLAGLM PVAQQPVYCASKHGIVGFTRSAALAANLMNSGVRLNAICPGFVN TAILESIEKEENMGQYIEYKDHIKDMIKYYGILDPPLIANGLITLIED DALNGAIMKITTSKGIHFQDYDTTPFQAKTQ | 15 |
| smPCTAHb | MTRLTVLALLAGLLASSRAGGSRTVPPMVNVTCSEVSEGNITVTCRA SSFYPRNITLTWRQDGVSLSHNTQQWGDVLPDGNGTYQTWVAT RIRQGEEQRFTCYMEHSGNHGTHPVPSGKALVLQSQRTDAEAAA KEAAAKAILLWQPIPVPPILLWQPIPVPPILLWQPIPVPPILLWQPIP VPPILLWQPIPVPPILLWQPIPVPPILLWQPIPVPPILLWQPIPVPPILL WQPIPVPPILLWQPIPVAEAAAKEAAAKAMHVNGKVALVTGAAQ GIGRAFAEALLLKGAKVALVDWNLEAGVQCKAALDEQFEPQKTL FIQCDVADQQQLRDTFRKVVDHFGRLDILVNNAGVNNEKNWEK TLQINLVSVISGTYLGLDYMSKQNGGEGGIIINMSSLAGLMPVAQ QPVYCASKHGIVGFTRSAALAANLMNSGVRLNAICPGFVNTAILE SIEKEENMGQYIEYKDHIKDMIKYYGILDPPLIANGLITLIEDDALN GAIMKITTSKGIHFQDYDTTPFQAKTQ | 16 |
| smCTAHa | MTRLTVLALLAGLLASSRAGGSRTVPPMVNVTCSEVSEGNITVTCRA SSFYPRNITLTWRQDGVSLSHNTQQWGDVLPDGNGTYQTWVAT | 17 |
| | RIRQGEEQRFTCYMEHSGNHGTHPVPSGKALVLQSQRTDAEAAA KEAAAKAMLMAQEALAFLGGAARAVFLALGGFLWGPRAYAGG YRPRPRRYGGYYWPRPRRYGGEADPTGHSYGGSLFRAVITKGGN YKHCFPEIGGEVYDGREHSAGGREPVTKAEMLGGDPARYEFLWG GSAFPTTINFGGSAYGEPRKLGGKMVELVHFLGGYLQLVFGIEVG GEYLQLVFGIGGEVDPIGHLYGGFLWGPRALVGGTFPDLESEFGGI MPKAGLLIGGMEVDPIGHLYGGWQYFFPVIFGGGVYDGREHTVG GMVKISGGPRGGEVDPIGHVYGGGLYDGMEHLGGVRIGHLYILG GILLRDAGLVGGSLLMWITQCFLGGSLLMWITQCGGQLSLLMWI TGGMPFATPMEAGGASGPGGGAPRAEAAAKEAAAKAMHVNGK VALVTGAAQGIGRAFAEALLLKGAKVALVDWNLEAGVQCKAAL DEQFEPQKTLFIQCDVADQQQLRDTFRKVVDHFGRLDILVNNAG VNNEKNWEKTLQINLVSVISGTYLGLDYMSKQNGGEGGIIINMSS LAGLMPVAQQPVYCASKHGIVGFTRSAALAANLMNSGVRLNAIC PGFVNTAILESIEKEENMGQYIEYKDHIKDMIKYYGILDPPLIANGL ITLIEDDALNGAIMKITTSKGIHFQDYDTTPFQAKTQ | |
| smCTAHb | MTRLTVLALLAGLLASSRAGGSRTVPPMVNVTCSEVSEGNITVTCRA SSFYPRNITLTWRQDGVSLSHNTQQWGDVLPDGNGTYQTWVAT RIRQGEEQRFTCYMEHSGNHGTHPVPSGKALVLQSQRTDAEAAA KEAAAKAMLMAQEALAFLPPAARAVFLALPPFLWGPRAYAPPYR PRPRRYPPYYWPRPRRYPPEADPTGHSYPPSLFRAVITKPPNYKHC FPEIPPEVYDGREHSAPPREPVTKAEMLPPDPARYEFLWPPSAFPT TINFPPSAYGEPRKLPPKMVELVHFLPPYLQLVFGIEVPPEYLQLVF GIPPEVDPIGHLYPPFLWGPRALVPPTFPDLESEFPPIMPKAGLLIPP MEVDPIGHLYPPWQYFFPVIFPPGVYDGREHTVPPMVKISGGPRPP EVDPIGHVYPPGLYDGMEHLPPVRIGHLYILPPILLRDAGLVPPSLL MWITQCFLPPSLLMWITQCPPQLSLLMWITPPMPFATPMEAPPAS GPGGGAPRAEAAAKEAAAKAMHVNGKVALVTGAAQGIGRAFAE ALLLKGAKVALVDWNLEAGVQCKAALDEQFEPQKTLFIQCDVA DQQQLRDTFRKVVDHFGRLDILVNNAGVNNEKNWEKTLQINLVS VISGTYLGLDYMSKQNGGEGGIIINMSSLAGLMPVAQQPVYCASK HGIVGFTRSAALAANLMNSGVRLNAICPGFVNTAILESIEKEENM GQYIEYKDHIKDMIKYYGILDPPLIANGLITLIEDDALNGAIMKITT SKGIHFQDYDTTPFQAKTQ | 18 |

| | | |
|---|---|---|
| Notes: The underlined regions are linker peptides, and the italicized regions are signal peptides. | | |

**Table 3. Amino acid sequences encoded by A34R**

| Protein | Source | Sequence | **SEQ ID NO.** |
|---|---|---|---|
| CoA34R | Vaccinia virus Copenhagen strain | MKSLNRQTVSMFKKLSVPAAIMMILSTIISGIGTFL HYKEELMPSACANGWIQYDKHCYLDTNIKMSTD NAVYQCRKLRARLPRPDTRHLRVLFSIFYKDYWV SLKKTNNKWLDINNDKDIDISKLTNFKQLNSTTDA EACYIYKSGKLVKTVCKSTQSVLCVKKFYK | 19 |
| WR A34R | Vaccinia virus WR strain | MKSLNRQTVSRFKKLSVPAAIMMILSTIISGIGTFL HYKEELMPSACANGWIQYDKHCYLDTNIKMSTD NAVYQCRKLRARLPRPDTRHLRVLFSIFYKDYWV SLKKTNDKWLDINNDKDIDISKLTNFKQLNSTTDA EACYIYKSGKLVKTVCKSTQSVLCVKKFYK | 20 |

**Table 4. Amino acid sequences of IL12 p70 fusion proteins**

| Fusion protein | Sequence | **SEQ ID NO.** |
|---|---|---|
| sIL12 (mouse) | MTRLTVLALLAGLLASSRAMWELEKDVYVVEVDWTPDAPGETV NLTCDTPEEDDITWTSDQRHGVIGSGKTLTITVKEFLDAGQYTC HKGGETLSHSHLLLHKKENGIWSTEILKNFKNKTFLKCEAPNYS GRFTCSWLVQRNMDLKFNIKSSSSSPDSRAVTCGMASLSAEKV TLDQRDYEKYSVSCQEDVTCPTAEETLPIELALEARQQNKYEN YSTSFFIRDIIKPDPPKNLQMKPLKNSQVEVSWEYPDSWSTPHSY FSLKFFVRIQRKKEKMKETEEGCNQKGAFLVEKTSTEVQCKGG NVCVQAQDRYYNSSCSKWACVPCRVRSGGGGSGGGGSRVIPV SGPARCLSQSRNLLKTTDDMVKTAREKLKHYSCTAEDIDHEDIT RDQTSTLKTCLPLELHKNESCLATRETSSTTRGSCLPPQKTSLM MTLCLGSIYEDLKMYQTEFQAINAALQNHNHQQIILDKGMLVA IDELMQSLNHNGETLRQKPPVGEADPYRVKMKLCILLHAFSTR VVTINRVMGYLSSA | 21 |
| mIL-12 I (mouse) | MTRLTVLALLAGLLASSRAMWELEKDVYVVEVDWTPDAPGETV NLTCDTPEEDDITWTSDQRHGVIGSGKTLTITVKEFLDAGQYTC HKGGETLSHSHLLLHKKENGIWSTEILKNFKNKTFLKCEAPNYS GRFTCSWLVQRNMDLKFNIKSSSSSPDSRAVTCGMASLSAEKV TLDQRDYEKYSVSCQEDVTCPTAEETLPIELALEARQQNKYEN | 22 |
| | YSTSFFIRDIIKPDPPKNLQMKPLKNSQVEVSWEYPDSWSTPHSY FSLKFFVRIQRKKEKMKETEEGCNQKGAFLVEKTSTEVQCKGG NVCVQAQDRYYNSSCSKWACVPCRVRSGGGGSGGGGSRVIPV SGPARCLSQSRNLLKTTDDMVKTAREKLKHYSCTAEDIDHEDIT RDQTSTLKTCLPLELHKNESCLATRETSSTTRGSCLPPQKTSLM MTLCLGSIYEDLKMYQTEFQAINAALQNHNHQQIILDKGMLVA IDELMQSLNHNGETLRQKPPVGEADPYRVKMKLCILLHAFSTR VVTINRVMGYLSSAFWVLVVVGGVLACYSLLVTVAFIIFWV | |
| mIL-12 II (mouse) | MYGTIAVIVFFLIGFMIGYLGYMWELEKDVYVVEVDWTPDAPGE TVNLTCDTPEEDDITWTSDQRHGVIGSGKTLTITVKEFLDAGQY TCHKGGETLSHSHLLLHKKENGIWSTEILKNFKNKTFLKCEAPN YSGRFTCSWLVQRNMDLKFNIKSSSSSPDSRAVTCGMASLSAE KVTLDQRDYEKYSVSCQEDVTCPTAEETLPIELALEARQQNKY ENYSTSFFIRDIIKPDPPKNLQMKPLKNSQVEVSWEYPDSWSTP HSYFSLKFFVRIQRKKEKMKETEEGCNQKGAFLVEKTSTEVQC KGGNVCVQAQDRYYNSSCSKWACVPCRVRSGGGGSGGGGSR VIPVSGPARCLSQSRNLLKTTDDMVKTAREKLKHYSCTAEDID HEDITRDQTSTLKTCLPLELHKNESCLATRETSSTTRGSCLPPQK TSLMMTLCLGSIYEDLKMYQTEFQAINAALQNHNHQQIILDKG MLVAIDELMQSLNHNGETLRQKPPVGEADPYRVKMKLCILLHA FSTRVVTINRVMGYLSSA | 23 |
| sIL12 (human) | MTRLTVLALLAGLLASSRAIWELKKDVYVVELDWYPDAPGEMVV LTCDTPEEDGITWTLDQSSEVLGSGKTLTIQVKEFGDAGQYTCH KGGEVLSHSLLLLHKKEDGIWSTDILKDQKEPKNKTFLRCEAK NYSGRFTCWWLTTISTDLTFSVKSSRGSSDPQGVTCGAATLSAE RVRGDNKEYEYSVECQEDSACPAAEESLPIEVMVDAVHKLKYE NYTSSFFIRDIIKPDPPKNLQLKPLKNSRQVEVSWEYPDTWSTPH SYFSLTFCVQVQGKSKREKKDRVFTDKTSATVICRKNASISVRA QDRYYSSSWSEWASVPCSGGGGSGGGGSRNLPVATPDPGMFP CLHHSQNLLRAVSNMLQKARQTLEFYPCTSEEIDHEDITKDKTS TVEACLPLELTKNESCLNSRETSFITNGSCLASRKTSFMMALCLS SIYEDLKMYQVEFKTMNAKLLMDPKRQIFLDQNMLAVIDELM QALNFNSETVPQKSSLEEPDFYKTKIKLCILLHAFRIRAVTIDRV MSYLNAS | 24 |
| mIL-12 I (human) | MTRLTVLALLAGLLASSRAIWELKKDVYVVELDWYPDAPGEMVV LTCDTPEEDGITWTLDQSSEVLGSGKTLTIQVKEFGDAGQYTCH | 25 |
| | KGGEVLSHSLLLLHKKEDGIWSTDILKDQKEPKNKTFLRCEAK NYSGRFTCWWLTTISTDLTFSVKSSRGSSDPQGVTCGAATLSAE RVRGDNKEYEYSVECQEDSACPAAEESLPIEVMVDAVHKLKYE NYTSSFFIRDIIKPDPPKNLQLKPLKNSRQVEVSWEYPDTWSTPH SYFSLTFCVQVQGKSKREKKDRVFTDKTSATVICRKNASISVRA QDRYYSSSWSEWASVPCSGGGGSGGGGSRNLPVATPDPGMFP CLHHSQNLLRAVSNMLQKARQTLEFYPCTSEEIDHEDITKDKTS TVEACLPLELTKNESCLNSRETSFITNGSCLASRKTSFMMALCLS SIYEDLKMYQVEFKTMNAKLLMDPKRQIFLDQNMLAVIDELM QALNFNSETVPQKSSLEEPDFYKTKIKLCILLHAFRIRAVTIDRV MSYLNASFWVLVVVGGVLACYSLLVTVAFIIFWV | |
| mIL-12 II (human) | MYGTIAVIVFFLIGFMIGYLGYIWELKKDVYVVELDWYPDAPGE MVVLTCDTPEEDGITWTLDQSSEVLGSGKTLTIQVKEFGDAGQ YTCHKGGEVLSHSLLLLHKKEDGIWSTDILKDQKEPKNKTFLR CEAKNYSGRFTCWWLTTISTDLTFSVKSSRGSSDPQGVTCGAA TLSAERVRGDNKEYEYSVECQEDSACPAAEESLPIEVMVDAVH KLKYENYTSSFFIRDIIKPDPPKNLQLKPLKNSRQVEVSWEYPDT WSTPHSYFSLTFCVQVQGKSKREKKDRVFTDKTSATVICRKNA SISVRAQDRYYSSSWSEWASVPCSGGGGSGGGGSRNLPVATPD PGMFPCLHHSQNLLRAVSNMLQKARQTLEFYPCTSEEIDHEDIT KDKTSTVEACLPLELTKNESCLNSRETSFITNGSCLASRKTSFM MALCLSSIYEDLKMYQVEFKTMNAKLLMDPKRQIFLDQNMLA VIDELMQALNFNSETVPQKSSLEEPDFYKTKIKLCILLHAFRIRA VTIDRVMSYLNAS | 26 |

| | | |
|---|---|---|
| Notes: The underlined regions are linker peptides, and the italicized regions are signal peptides, signal-anchor peptides, or transmembrane peptides. | | |

**Table 5. Amino acid sequences of tumor antigen peptides**

| Protein | Antigen peptide sequence | **SEQ ID NO.** |
|---|---|---|
| MICA | MICA peptide: | 27 |
| | RTVPPMVNVTRSEASEGNITVTCRASGFYPWNITLSWRQDG VSLSHDTQQWGDVLPDGNGTYQTWVATRICQGEEQRFTCY MEHSGNHSTHPVPSGKVLVLQSHW | |
| MICB | MICB peptide: | 28 |
| | RTVPPMVNVTCSEVSEGNITVTCRASSFYPRNITLTWRQDGV SLSHNTQQWGDVLPDGNGTYQTWVATRIRQGEEQRFTCYM EHSGNHGTHPVPSGKALVLQSQRTD | |
| CAMEL | A2 peptide: MLMAQEALAFL | 29 |
| BAGE | Cw1601 peptide: AARAVFLAL | 30 |
| DAM-6/10 | A2 peptide: FLWGPRAYA | 31 |
| GAGE-2/8 | Cw6 peptide: YRPRPRRY | 32 |
| GAGE-5/6/7/7B | A29 peptide: YYWPRPRRY | 33 |
| MAGE-A1 | A1 peptide: EADPTGHSY | 34 |
| | A3 peptide: SLFRAVITK | 35 |
| | A24 peptide: NYKHCFPEI | 36 |
| | A28 peptide: EVYDGREHSA | 37 |
| | B37 peptide: REPVTKAEML | 38 |
| | B53 peptide: DPARYEFLW | 39 |
| | Cw2 peptide: SAFPTTINF | 40 |
| | Cw3/16 peptide: SAYGEPRKL | 41 |
| MAGE-A2 | A2 peptide: KMVELVHFL | 42 |
| | A2 peptide: YLQLVFGIEV | 43 |
| | A24 peptide: EYLQLVFGI | 44 |
| MAGE-A3 | B3501 peptide: EVDPIGHLY | 45 |
| | A2 peptide: FLWGPRALV | 46 |
| | A24 peptide: TFPDLESEF | 47 |
| | A24 peptide: IMPKAGLL | 48 |
| | B44 peptide: MEVDPIGHLY | 49 |
| | B52 peptide: WQYFFPVIF | 50 |
| MAGE-A4 | A2 peptide: GVYDGREHTV | 51 |
| MAGE-A6 | A34 peptide: MVKISGGPR | 52 |
| | B3501 peptide: EVDPIGHVY | 53 |
| MAGE-A10 | A2 peptide: GLYDGMEHL | 54 |
| MAGE-A12 | Cw7 peptide: VRIGHLYIL | 55 |
| TRAG-3 | A0201 peptide: ILLRDAGLV | 56 |
| NY-ESO-1 | A2 peptide: SLLMWITQCFL | 57 |
| | A2 peptide: SLLMWITQC | 58 |
| | A2 peptide: QLSLLMWIT | 59 |
| | B3501 peptide: MPFATPMEA | 60 |
| CTAG1A | A31 peptide: ASGPGGGAPR | 61 |
| PAP | A2 peptide: ILLWQPIPV | 62 |

**Table 6. Nucleotide sequences for expression of fusion proteins**

| Fusion protein | Nucleotide sequence | **SEQ ID NO.** |
|---|---|---|
| sHPGD | ATGACCAGGCTGACAGTGCTGGCCCTGCTGGCCGGCCTCCTGGCCAGCTCCAGGGCC ATGCACGTCAACGGAAGGTGGCCCTGGTGACAGGGGCCGCCCAGGGCATCGGGAG AGCCTTCGCCGAGGCCCTGCTGCTGAAGGGGGCCAAGGTGGCCCTGGTGGATTGGA ACCTGGAGGCCGGCGTGCAGTGCAAGGCCGCCCTGGATGAGCAGTTCGAGCCCCAG AAAACCCTCTTCATCCAGTGCGATGTGGCCGATCAGCAGCAGCTGCGGATACCTTT AGGAAGGTGGTGGACCACTTCGGCAGGCTGGACATCCTGGTGAACAACGCCGGGGT GAACAACGAGAAGAACTGGGAAAAGACTCTGCAGATTAACCTGGTGTCCGTGATCT CCGGGACCTACCTGGGGCTGGACTACATGAGCAAGCAGAACGGGGGCGAGGGGGG GATCATCATTAACATGTCCAGCCTGGCCGGGCTGATGCCCGTGGCCCAGCAGCCCGT GTACTGCGCCAGCAAGCACGGCATCGTGGGGTTCACCCGGAGCGCCGCCCTCGCCG CCAACCTGATGAACAGCGGGGTGCGGCTGAACGCCATCTGCCCAGGGTTTGTGAAC ACCGCCATCCTCGAGAGCATCGAGAAGGAGGAGAACATGGGCCAGTACATTGAGTA CAAGGATCACATCAAGGATATGATCAAGTACTACGGCATCCTCGATCCCCCCCTGAT TGCCAACGGGCTGATCACCCTGATTGAGGACGACGCCCTGAACGGGGCCATCATGA AGATCACAACCTCCAAGGGCATCCACTTCCAGGACTACGACACAACACCCTTCCAG GCCAAGACACAG | 65 |
| sMICAH | ATGACAAGGCTGACAGTGCTGGCCCTGCTGGCCGGCCTGCTGGCCTCCAGCCGGGCC GGGGGCAGCAGGACCGTGCCCCCAATGGTGAACGTGACAAGGTCCGAGGCCAGCGA GGGCAACATCACCGTGACATGCCGGGCCTCCGGGTTCTACCCCTGGAACATCACCCT GAGCTGGCGGCAGGATGGCGTGAGCCTGTCCCACGACACACAGCAGTGGGGGGATG TGCTGCCCGATGGCAACGGCACATACCAGACTTGGGTGGCCACCCGGATCTGCCAG | 66 |
| | GGGGAGGAGCAGCGGTTTACATGCTACATGGAGCACTCCGGCAACCACAGCACTCA CCCCGTGCCCAGCGGGAAGGTGCTGGTGCTCCAGAGCCACTGGGCTGAGGCCGCCG CTAAGGAGGCCGCCGCCAAGGCCATGCACGTCAACGGGAAGGTGGCCCTGGTGACA GGGGCCGCCCAGGGCATCGGGAGAGCCTTCGCCGAGGCCCTGCTGCTGAAGGGGGC CAAGGTGGCCCTGGTGGATTGGAACCTGGAGGCCGGCGTGCAGTGCAAGGCCGCCC TGGATGAGCAGTTCGAGCCCCAGAAAACCCTCTTCATCCAGTGCGATGTGGCCGATC AGCAGCAGCTGCGGGATACCTTTAGGAAGGTGGTGGACCACTTCGGCAGGCTGGAC ATCCTGGTGAACAACGCCGGGGTGAACAACGAGAAGAACTGGGAAAAGACTCTGCA GATTAACCTGGTGTCCGTGATCTCCGGGACCTACCTGGGGCTGGACTACATGAGCAA GCAGAACGGGGGCGAGGGGGGATCATCATTAACATGTCCAGCCTGGCCGGGCTGA TGCCCGTGGCCCAGCAGCCCGTGTACTGCGCCAGCAAGCACGGCATCGTGGGGTTC ACCCGGAGCGCCGCCCTCGCCGCCAACCTGATGAACAGCGGGGTGCGGCTGAACGC CATCTGCCCAGGGTTTGTGAACACCGCCATCCTCGAGAGCATCGAGAAGGAGGAGA ACATGGGCCAGTACATTGAGTACAAGGATCACATCAAGGATATGATCAAGTACTAC GGCATCCTCGATCCCCCCCTGATTGCCAACGGGCTGATCACCCTGATTGAGGACGAC GCCCTGAACGGGGCCATCATGAAGATCACAACCTCCAAGGGCATCCACTTCCAGGA CTACGACACAACACCCTTCCAGGCCAAGACACAG | |
| sMICBH | ATGACCAGGCTGACAGTGCTGGCCCTGCTGGCCGGCCTCCTGGCCAGCTCCAGGGCC GGGGGCAGCCGGACCGTGCCCCCCATGGTGAACGTGACATGCAGCGAGGTCAGCGA GGGCAACATCACAGTGACCTGCAGAGCCTCCAGCTTCTACCCCAGGAACATCACACT GACTTGGAGGCAGGATGGGGTGAGCCTGAGCCACAACACACAGCAGTGGGGGGAT GTGCTGCCTGACGGGAACGGCACATACCAGACATGGGTGGCCACAAGGATCCGGCA GGGCGAGGAGCAGCGCTTCACCTGCTACATGGAGCACAGCGGGAACCACGGACAC ACCCCGTGCCTAGCGGCAAGGCCCTCGTGCTGCAGAGCCAGCGGACCGACGCTGAG GCCGCCGCTAAGGAGGCCGCCGCCAAGGCCATGCACGTCAACGGAAGGTGGCCCT GGTGACAGGGGCCGCCCAGGGCATCGGAGAGCCTTCGCCGAGGCCCTGCTGCTGA AGGGGGCCAAGGTGGCCCTGGTGGATTGGAACCTGGAGGCCGGCGTGCAGTGCAAG GCCGCCCTGGATGAGCAGTTCGAGCCCCAGAAAACCCTCTTCATCCAGTGCGATGTG GCCGATCAGCAGCAGCTGCGGGATACCTTTAGGAAGGTGGTGGACCACTTCGGCAG GCTGGACATCCTGGTGAACAACGCCGGGGTGAACAACGAGAAGAACTGGGAAAAG ACTCTGCAGATTAACCTGGTGTCCGTGATCTCCGGGACCTACCTGGGGCTGGACTAC ATGAGCAAGCAGAACGGGGGCGAGGGGGGATCATCATTAACATGTCCAGCCTGGC CGGGCTGATGCCCGTGGCCCAGCAGCCCGTGTACTGCGCCAGCAAGCACGGCATCG TGGGGTTCACCCGGAGCGCCGCCCTCGCCGCCAACCTGATGAACAGCGGGGTGCGG CTGAACGCCATCTGCCCAGGGTTTGTGAACACCGCCATCCTCGAGAGCATCGAGAA GGAGGAGAACATGGGCCAGTACATTGAGTACAAGGATCACATCAAGGATATGATCA | 67 |
| | AGTACTACGGCATCCTCGATCCCCCCCTGATTGCCAACGGGCTGATCACCCTGATTG AGGACGACGCCCTGAACGGGGCCATCATGAAGATCACAACCTCCAAGGGCATCCAC TTCCAGGACTACGACACAACACCCTTCCAGGCCAAGACACAG | |
| smPCTAHa | ATGACCAGGCTGACAGTGCTGGCCCTGCTGGCCGGCCTCCTGGCCAGCTCCAGGGCC GGGGGCAGCCGGACCGTGCCCCCATGGTGAACGTGACATGCAGCGAGGTCAGCGA GGGCAACATCACAGTGACCTGCAGAGCCTCCAGCTTCTACCCCAGGAACATCACACT GACTTGGAGGCAGGATGGGGTGAGCCTGAGCCACAACACACAGCAGTGGGGGGAT GTGCTGCCTGACGGGAACGGCACATACCAGACATGGGTGGCCACAAGGATCCGGCA GGGCGAGGAGCAGCGCTTCACCTGCTACATGGAGCACAGCGGGAACCACGGACAC ACCCCGTGCCTAGCGGCAAGGCCCTCGTGCTGCAGAGCCAGCGGACCGACGCCGAG GCCGCCGCCAAGGAGGCCGCCGCCAAGGCCATCCTGCTGTGGCAGCCCATTCCCGT GGGCGGGATCCTGCTGTGGCAGCCCATCCCCGTGGGCGGCATCCTGCTCTGGCAGCC CATCCCCGTCGGCGGGATCCTCCTCTGGCAGCCAATCCCCGTCGGGGGCATCCTCCT GTGGCAGCCAATCCCCGTGGGGGGCATCCTGTTGTGGCAGCCTATTCCCGTGGGGGG GATCCTCCTCTGGCAGCCTATCCCCGTCGGGGGCATTCTGCTGTGGCAACCCATCCC CGTGGGCGGCATCCTCCTGTGGCAGCCTATCCCCGTGGGGGGGATCCTGCTCTGGCA GCCCATTCCCGTGGCCGAAGCTGCTGCTAAGGAGGCCGCCGCTAAGGCCATGCACG TCAACGGGAAGGTGGCCCTGGTGACAGGGGCCGCCCAGGGCATCGGGAGAGCCTTC GCCGAGGCCCTGCTGCTGAAGGGGGCCAAGGTGGCCCTGGTGGATTGGAACCTGGA GGCCGGCGTGCAGTGCAAGGCCGCCCTGGATGAGCAGTTCGAGCCCCAGAAAACCC TCTTCATCCAGTGCGATGTGGCCGATCAGCAGCAGCTGCGGGATACCTTTAGGAAGG TGGTGGACCACTTCGGCAGGCTGGACATCCTGGTGAACAACGCCGGGGTGAACAAC GAGAAGAACTGGGAAAAGACTCTGCAGATTAACCTGGTGTCCGTGATCTCCGGGAC CTACCTGGGGCTGGACTACATGAGCAAGCAGAACGGGGGCGAGGGGGGGATCATCA TTAACATGTCCAGCCTGGCCGGGCTGATGCCCGTGGCCCAGCAGCCCGTGTACTGCG CCAGCAAGCACGGCATCGTGGGGTTCACCCGGAGCGCCGCCCTCGCCGCCAACCTG ATGAACAGCGGGGTGCGGCTGAACGCCATCTGCCCAGGGTTTGTGAACACCGCCAT CCTCGAGAGCATCGAGAAGGAGGAGAACATGGGCCAGTACATTGAGTACAAGGATC ACATCAAGGATATGATCAAGTACTACGGCATCCTCGATCCCCCCCTGATTGCCAACG GGCTGATCACCCTGATTGAGGACGACGCCCTGAACGGGGCCATCATGAAGATCACA ACCTCCAAGGGCATCCACTTCCAGGACTACGACACAACACCCTTCCAGGCCAAGAC ACAG | 68 |
| smPCTAHb | ATGACCAGGCTGACAGTGCTGGCCCTGCTGGCCGGCCTCCTGGCCAGCTCCAGGGCC GGGGGCAGCCGGACCGTGCCCCCCATGGTGAACGTGACATGCAGCGAGGTCAGCGA GGGCAACATCACAGTGACCTGCAGAGCCTCCAGCTTCTACCCCAGGAACATCACACT GACTTGGAGGCAGGATGGGGTGAGCCTGAGCCACAACACACAGCAGTGGGGGGAT | 69 |
| | GTGCTGCCTGACGGGAACGGCACATACCAGACATGGGTGGCCACAAGGATCCGGCA GGGCGAGGAGCAGCGCTTCACCTGCTACATGGAGCACAGCGGGAACCACGGACAC ACCCCGTGCCTAGCGGCAAGGCCCTCGTGCTGCAGAGCCAGCGGACCGACGCTGAG GCCGCCGCTAAGGAGGCCGCCGCCAAGGCCATCCTGCTGTGGCAGCCCATTCCCGTG CCCCCTATCCTGCTCTGGCAGCCAATCCCCGTCCCCCCCATCCTGCTCTGGCAGCCCA TCCCCGTCCCCCCCATCCTCCTGTGGCAGCCAATCCCCGTGCCACCCATTCTGCTGTG GCAGCCTATCCCTGTGCCCCCAATCCTGCTTTGGCAGCCTATCCCCGTGCCACCCATC CTCCTCTGGCAGCCCATTCCCGTGCCTCCCATCCTGTTGTGGCAGCCCATCCCCGTGC CCCCTATCCTGTTGTGGCAGCCAATTCCCGTGCCCCCCATCCTGCTGTGGCAACCCAT CCCCGTGGCCGAGGCCGCCGCAAAGGAGGCCGCCGCCAAGGCCATGCACGTCAACG GGAAGGTGGCCCTGGTGACAGGGGCCGCCCAGGGCATCGGGAGAGCCTTCGCCGAG GCCCTGCTGCTGAAGGGGGCCAAGGTGGCCCTGGTGGATTGGAACCTGGAGGCCGG CGTGCAGTGCAAGGCCGCCCTGGATGAGCAGTTCGAGCCCCAGAAAACCCTCTTCAT CCAGTGCGATGTGGCCGATCAGCAGCAGCTGCGGGATACCTTTAGGAAGGTGGTGG ACCACTTCGGCAGGCTGGACATCCTGGTGAACAACGCCGGGGTGAACAACGAGAAG AACTGGGAAAAGACTCTGCAGATTAACCTGGTGTCCGTGATCTCCGGGACCTACCTG GGGCTGGACTACATGAGCAAGCAGAACGGGGGCGAGGGGGGGATCATCATTAACAT GTCCAGCCTGGCCGGGCTGATGCCCGTGGCCCAGCAGCCCGTGTACTGCGCCAGCA AGCACGGCATCGTGGGGTTCACCCGGAGCGCCGCCCTCGCCGCCAACCTGATGAAC AGCGGGGTGCGGCTGAACGCCATCTGCCCAGGGTTTGTGAACACCGCCATCCTCGA GAGCATCGAGAAGGAGGAGAACATGGGCCAGTACATTGAGTACAAGGATCACATCA AGGATATGATCAAGTACTACGGCATCCTCGATCCCCCCCTGATTGCCAACGGGCTGA TCACCCTGATTGAGGACGACGCCCTGAACGGGGCCATCATGAAGATCACAACCTCC AAGGGCATCCACTTCCAGGACTACGACACAACACCCTTCCAGGCCAAGACACAG | |
| smCTAHa | ATGACCAGGCTGACAGTGCTGGCCCTGCTGGCCGGCCTCCTGGCCAGCTCCAGGGCC GGGGGCAGCCGGACCGTGCCCCCATGGTGAACGTGACATGCAGCGAGGTCAGCGA GGGCAACATCACAGTGACCTGCAGAGCCTCCAGCTTCTACCCCAGGAACATCACACT GACTTGGAGGCAGGATGGGGTGAGCCTGAGCCACAACACACAGCAGTGGGGGGAT GTGCTGCCTGACGGGAACGGCACATACCAGACATGGGTGGCCACAAGGATCCGGCA GGGCGAGGAGCAGCGCTTCACCTGCTACATGGAGCACAGCGGGAACCACGGACAC ACCCCGTGCCTAGCGGCAAGGCCCTCGTGCTGCAGAGCCAGCGGACCGACGCCGAG GCCGCCGCCAAGGAGGCCGCCGCCAAGGCCATGCTGATGGCCCAGGAGGCCCTGGC CTTCCTGGGCGGCGCCGCCAGGGCCGTGTTTCTGGCCCTCGGCGGGTTTCTCTGGGG CCCCAGAGCCTACGCCGGGGGGTACAGGCCTAGGCCCAGGAGGTACGGGGGCTACT ACTGGCCCCGGCCCAGAAGGTACGGGGGCGAGGCCGACCCCACCGGGCACAGCTAT GGCGGGAGCCTGTTCAGGGCCGTGATCACAAAGGGCGGGAACTACAAGCACTGCTT | 70 |
| | TCCCGAGATTGGCGGGGAGGTGTACGACGGGCGGAGCACTCCGCCGGGGGGCGGG AGCCCGTCACAAAGGCTGAGATGCTGGGGGGGATCCCGCCCGGTACGAGTTCCTG TGGGGGGGAGCGCCTTCCCAACAACCATCAACTTCGGGGGCTCCGCCTACGGGGA GCCCAGGAAGCTGGGGGGCAAGATGGTGGAGCTCGTGCACTTCCTGGGGGGGTACC TGCAGCTCGTGTTCGGGATTGAGGTGGGCGGCGAGTACCTCCAGCTGGTCTTTGGCA TCGGCGGGGAGGCAGATCCTATTGGGCACCTGTACGGCGGGTTCCTGTGGGGGCCC AGGGCCCTGGTCGGGGGCACTTTCCCCGACCTGGAGAGCGAGTTCGGGGGCATCAT GCCCAAGGCCGGCCTGCTGATTGGCGGAATGGAGGTGGACCCCATCGGCCACCTGT ACGGCGGCTGGCAGTACTTCTTTCCAGTGATCTTCGGGGGCGGGGTGTACGATGGGC GGGAGCACACAGTGGGCGGGATGGTGAAGATTAGCGGGGGGCCCAGGGGCGGGGA GGTGGATCCTATCGGCCACGTGTACGGCGGGGGCCTGTACGACGGGATGGAGCACC TGGGCGGCGTCCGGATTGGGCACCTGTACATTCTGGGCGGCATCCTGCTGCGGGACG CCGGCCTGGTGGGCGGCTCCCTGCTGATGTGGATCACCCAGTGCTTCCTGGGCGGGA GCCTGCTCATGTGGATCACCCAGTGCGGCGGCCAGCTGAGCCTGCTGATGTGGATCA CAGGCGGCATGCCCTTCGCCACCCCCATGGAGGCCGGGGGCGCCTCCGGGCCTGGG GGCGGCGCCCCCAGGGCCGAAGCTGCTGCTAAGGAGGCCGCCGCTAAGGCCATGCA CGTCAACGGGAAGGTGGCCCTGGTGACAGGGGCCGCCCAGGGCATCGGGAGAGCCT TCGCCGAGGCCCTGCTGCTGAAGGGGGCCAAGGTGGCCCTGGTGGATTGGAACCTG GAGGCCGGCGTGCAGTGCAAGGCCGCCCTGGATGAGCAGTTCGAGCCCCAGAAAAC CCTCTTCATCCAGTGCGATGTGGCCGATCAGCAGCAGCTGCGGGATACCTTTAGGAA GGTGGTGGACCACTTCGGCAGGCTGGACATCCTGGTGAACAACGCCGGGGTGAACA ACGAGAAGAACTGGGAAAAGACTCTGCAGATTAACCTGGTGTCCGTGATCTCCGGG ACCTACCTGGGGCTGGACTACATGAGCAAGCAGAACGGGGGCGAGGGGGGGATCAT CATTAACATGTCCAGCCTGGCCGGGCTGATGCCCGTGGCCCAGCAGCCCGTGTACTG CGCCAGCAAGCACGGCATCGTGGGGTTCACCCGGAGCGCCGCCCTCGCCGCCAACC TGATGAACAGCGGGGTGCGGCTGAACGCCATCTGCCCAGGGTTTGTGAACACCGCC ATCCTCGAGAGCATCGAGAAGGAGGAGAACATGGGCCAGTACATTGAGTACAAGGA TCACATCAAGGATATGATCAAGTACTACGGCATCCTCGATCCCCCCCTGATTGCCAA CGGGCTGATCACCCTGATTGAGGACGACGCCCTGAACGGGGCCATCATGAAGATCA CAACCTCCAAGGGCATCCACTTCCAGGACTACGACACAACACCCTTCCAGGCCAAG ACACAG | |
| smCTAHb | ATGACCAGGCTGACAGTGCTGGCCCTGCTGGCCGGCCTCCTGGCCAGCTCCAGGGCC GGGGGCAGCCGGACCGTGCCCCCCATGGTGAACGTGACATGCAGCGAGGTCAGCGA GGGCAACATCACAGTGACCTGCAGAGCCTCCAGCTTCTACCCCAGGAACATCACACT GACTTGGAGGCAGGATGGGGTGAGCCTGAGCCACAACACACAGCAGTGGGGGGAT GTGCTGCCTGACGGGAACGGCACATACCAGACATGGGTGGCCACAAGGATCCGGCA | 71 |
| | GGGCGAGGAGCAGCGCTTCACCTGCTACATGGAGCACAGCGGAACCACGGACAC ACCCCGTGCCTAGCGGCAAGGCCCTCGTGCTGCAGAGCCAGCGGACCGACGCTGAG GCCGCCGCTAAGGAGGCCGCCGCCAAGGCCATGCTGATGGCCCAGGAGGCCCTGGC CTTTCTGCCACCCGCCGCAAGGGCCGTGTTCCTCGCTCTTCCTCCCTTTCTGTGGGGG CCCCGCGCCTACGCACCTCCATATCGGCCACGGCCTAGGAGGTACCCTCCATACTAC TGGCCCCGGCCTAGGCGGTACCCCCCGAGGCCGACCCCACAGGCCACAGCTACCC CCCCAGCCTGTTCCGGGCCGTGATCACTAAGCCACCTAACTACAAGCACTGCTTCCC TGAGATCCCTCCCGAGGTGTACGATGGCAGGGAGCACTCCGCCCCCCCAGGGAGC CCGTGACCAAGGCCGAGATGCTGCCCCCGATCCCGCCCGGTACGAGTTCCTGTGGC CACCTTCCGCCTTTCCTACCACCATCAACTTCCCCCCAGCGCCTACGGCGAGCCTA GGAAGCTGCCTCCAAAGATGGTCGAGCTGGTGCACTTCCTGCCACCCTACCTGCAGC TGGTCTTTGGGATTGAGGTGCCCCCCGAGTACCTGCAGCTGGTGTTCGGAATTCCCC CCGAGGCCGATCCCATCGGGCACCTGTACCCCCCTTCCTGTGGGGACCCAGGGCCC TGGTGCCCCCAACATTCCCCGACCTCGAGTCCGAGTTCCCCCTATCATGCCCAAGG CCGGGCTGCTCATCCCCCCAATGGAGGTGGACCCCATCGGGCACCTGTACCCCCCT GGCAGTACTTCTTTCCCGTGATCTTCCCCCCAGGGGTGTACGACGGCGGGAGCACA CAGTGCCACCCATGGTGAAGATAAGCGGCGGCCCTAGGCCCCCCGAGGTGGACCCT ATCGGCCACGTGTACCCCCCGGCCTGTACGATGGCATGGAGCACCTGCCCCCCGTG CGGATTGGCCACCTGTACATCCTGCCCCCCATTCTGCTGCGGATGCCGGCCTGGTG CCCCCCAGCCTGCTGATGTGGATTACACAGTGCTTTCTGCCCCCCAGCCTCCTGATGT GGATTACCCAGTGCCCCCCCCAGCTGTCCCTCCTGATGTGGATCACCCCCCCCATGC CCTTCGCCACCCCCATGGAGGCCCCCCCGCCAGCGGACCCGGCGGGGGGCCCCT CGGGCCGAGGCCGCCGCAAAGGAGGCCGCCGCCAAGGCCATGCACGTCAACGGGA AGGTGGCCCTGGTGACAGGGGCCGCCCAGGGCATCGGGAGAGCCTTCGCCGAGGCC CTGCTGCTGAAGGGGGCCAAGGTGGCCCTGGTGGATTGGAACCTGGAGGCCGGCGT GCAGTGCAAGGCCGCCCTGGATGAGCAGTTCGAGCCCCAGAAAACCCTCTTCATCC AGTGCGATGTGGCCGATCAGCAGCAGCTGCGGGATACCTTTAGGAAGGTGGTGGAC CACTTCGGCAGGCTGGACATCCTGGTGAACAACGCCGGGGTGAACAACGAGAAGAA CTGGGAAAAGACTCTGCAGATTAACCTGGTGTCCGTGATCTCCGGGACCTACCTGGG GCTGGACTACATGAGCAAGCAGAACGGGGGCGAGGGGGGGATCATCATTAACATGT CCAGCCTGGCCGGGCTGATGCCCGTGGCCCAGCAGCCCGTGTACTGCGCCAGCAAG CACGGCATCGTGGGGTTCACCCGGAGCGCCGCCCTCGCCGCCAACCTGATGAACAG CGGGGTGCGGCTGAACGCCATCTGCCCAGGGTTTGTGAACACCGCCATCCTCGAGA GCATCGAGAAGGAGGAGAACATGGGCCAGTACATTGAGTACAAGGATCACATCAAG GATATGATCAAGTACTACGGCATCCTCGATCCCCCCCTGATTGCCAACGGGCTGATC | |
| | ACCCTGATTGAGGACGACGCCCTGAACGGGGCCATCATGAAGATCACAACCTCCAA GGGCATCCACTTCCAGGACTACGACACAACACCCTTCCAGGCCAAGACACAG | |
| sIL12 (human) | ATGACCCGGCTCACAGTGCTGGCCCTGCTCGCCGGCCTGCTGGCCAGCTCCAGGGCC ATATGGGAACTGAAGAAAGATGTTTATGTCGTAGAATTGGATTGGTATCCGGATGCC CCTGGAGAAATGGTGGTCCTCACCTGTGACACCCCTGAAGAAGATGGTATCACCTGG ACCTTGGACCAGAGCAGTGAGGTCTTAGGCTCTGGCAAAACCCTGACCATCCAAGTC AAAGAGTTTGGAGATGCTGGCCAGTACACCTGTCACAAAGGAGGCGAGGTTCTAAG CCATTCGCTCCTGCTGCTTCACAAAAAGGAAGATGGAATTTGGTCCACTGATATTTT AAAGGACCAGAAAGAACCCAAAAATAAGACCTTTCTAAGATGCGAGGCCAAGAATT ATTCTGGACGTTTCACCTGCTGGTGGCTGACGACAATCAGTACTGATTTGACATTCA GTGTCAAAAGCAGCAGAGGCTCTTCTGACCCCCAAGGGGTGACGTGCGGAGCTGCT ACACTCTCTGCAGAGAGAGTCAGAGGGGACAACAAGGAGTATGAGTACTCAGTGGA GTGCCAGGAGGACAGTGCCTGCCCAGCTGCTGAGGAGAGTCTGCCCATTGAGGTCA TGGTGGATGCCGTTCACAAGCTCAAGTATGAAAACTACACCAGCAGCTTCTTCATCA GGGACATCATCAAACCTGACCCACCCAAGAACTTGCAGCTGAAGCCATTAAAGAAT TCTCGGCAGGTGGAGGTCAGCTGGGAGTACCCTGACACCTGGAGTACTCCACATTCC TACTTCTCCCTGACATTCTGCGTTCAGGTCCAGGGCAAGAGCAAGAGAGAAAAGAA AGATAGAGTCTTCACGGACAAGACCTCAGCCACGGTCATCTGCCGCAAAAATGCCA GCATTAGCGTGCGGGCCCAGGACCGCTACTATAGCTCATCTTGGAGCGAATGGGCAT CTGTGCCCTGCAGTGGCGGCGGCGGCAGCGGCGGCGGCGGCAGCAGAAACCTCCCC GTGGCCACTCCAGACCCAGGAATGTTCCCATGCCTTCACCACTCCCAAAACCTGCTG AGGGCCGTCAGCAACATGCTCCAGAAGGCCAGACAAACTCTAGAATTTTACCCTTGC ACTTCTGAAGAGATTGATCATGAAGATATCACAAAAGATAAAACCAGCACAGTGGA GGCCTGTTTACCATTGGAATTAACCAAGAATGAGAGTTGCCTAAATTCCAGAGAGAC CTCTTTCATAACTAATGGGAGTTGCCTGGCCTCCAGAAAGACCTCTTTTATGATGGC CCTGTGCCTTAGTAGTATTTATGAAGACTTGAAGATGTACCAGGTGGAGTTCAAGAC CATGAATGCAAAGCTTCTGATGGATCCTAAGAGGCAGATCTTTCTAGATCAAAACAT GCTGGCAGTTATTGATGAGCTGATGCAGGCCCTGAATTTCAACAGTGAGACTGTGCC ACAAAAATCCTCCCTTGAAGAACCGGATTTTTATAAAACTAAAATCAAGCTCTGCAT ACTTCTTCATGCTTTCAGAATTCGGGCAGTGACTATTGATAGAGTGATGAGCTATCT GAATGCTTCC | 72 |
| mIL-12 I (human) | ATGACCCGGCTCACAGTGCTGGCCCTGCTCGCCGGCCTGCTGGCCAGCTCCAGGGCC ATATGGGAACTGAAGAAAGATGTTTATGTCGTAGAATTGGATTGGTATCCGGATGCC CCTGGAGAAATGGTGGTCCTCACCTGTGACACCCCTGAAGAAGATGGTATCACCTGG ACCTTGGACCAGAGCAGTGAGGTCTTAGGCTCTGGCAAAACCCTGACCATCCAAGTC AAAGAGTTTGGAGATGCTGGCCAGTACACCTGTCACAAAGGAGGCGAGGTTCTAAG | 73 |
| | CCATTCGCTCCTGCTGCTTCACAAAAGGAAGATGGAATTTGGTCCACTGATATTTT AAAGGACCAGAAAGAACCCAAAAATAAGACCTTTCTAAGATGCGAGGCCAAGAATT ATTCTGGACGTTTCACCTGCTGGTGGCTGACGACAATCAGTACTGATTTGACATTCA GTGTCAAAAGCAGCAGAGGCTCTTCTGACCCCCAAGGGGTGACGTGCGGAGCTGCT ACACTCTCTGCAGAGAGAGTCAGAGGGGACAACAAGGAGTATGAGTACTCAGTGGA GTGCCAGGAGGACAGTGCCTGCCCAGCTGCTGAGGAGAGTCTGCCCATTGAGGTCA TGGTGGATGCCGTTCACAAGCTCAAGTATGAAAACTACACCAGCAGCTTCTTCATCA GGGACATCATCAAACCTGACCCACCCAAGAACTTGCAGCTGAAGCCATTAAAGAAT TCTCGGCAGGTGGAGGTCAGCTGGGAGTACCCTGACACCTGGAGTACTCCACATTCC TACTTCTCCCTGACATTCTGCGTTCAGGTCCAGGGCAAGAGCAAGAGAGAAAAGAA AGATAGAGTCTTCACGGACAAGACCTCAGCCACGGTCATCTGCCGCAAAAATGCCA GCATTAGCGTGCGGGCCCAGGACCGCTACTATAGCTCATCTTGGAGCGAATGGGCAT CTGTGCCCTGCAGTGGCGGCGGCGGCAGCGGCGGCGGCGGCAGCAGAAACCTCCCC GTGGCCACTCCAGACCCAGGAATGTTCCCATGCCTTCACCACTCCCAAAACCTGCTG AGGGCCGTCAGCAACATGCTCCAGAAGGCCAGACAAACTCTAGAATTTTACCCTTGC ACTTCTGAAGAGATTGATCATGAAGATATCACAAAAGATAAAACCAGCACAGTGGA GGCCTGTTTACCATTGGAATTAACCAAGAATGAGAGTTGCCTAAATTCCAGAGAGAC CTCTTTCATAACTAATGGGAGTTGCCTGGCCTCCAGAAAGACCTCTTTTATGATGGC CCTGTGCCTTAGTAGTATTTATGAAGACTTGAAGATGTACCAGGTGGAGTTCAAGAC CATGAATGCAAAGCTTCTGATGGATCCTAAGAGGCAGATCTTTCTAGATCAAAACAT GCTGGCAGTTATTGATGAGCTGATGCAGGCCCTGAATTTCAACAGTGAGACTGTGCC ACAAAAATCCTCCCTTGAAGAACCGGATTTTTATAAAACTAAAATCAAGCTCTGCAT ACTTCTTCATGCTTTCAGAATTCGGGCAGTGACTATTGATAGAGTGATGAGCTATCT GAATGCTTCCTTTTGGGTGCTGGTGGTGGTTGGTGGAGTCCTGGCTTGCTATAGCTTG CTAGTAACAGTGGCCTTTATTATTTTCTGGGTG | |
| mIL-12 II (human) | ATGTATGGCACCATTGCGGTGATTGTGTTTTTCTGATTGGCTTTATGATTGGCTATC TGGGCTATATATGGGAACTGAAGAAAGATGTTATGTCGTAGAATTGGATTGGTATC CGGATGCCCCTGGAGAAATGGTGGTCCTCACCTGTGACACCCCTGAAGAAGATGGT ATCACCTGGACCTTGGACCAGAGCAGTGAGGTCTTAGGCTCTGGCAAAACCCTGACC ATCCAAGTCAAAGAGTTTGGAGATGCTGGCCAGTACACCTGTCACAAAGGAGGCGA GGTTCTAAGCCATTCGCTCCTGCTGCTTCACAAAAAGGAAGATGGAATTTGGTCCAC TGATATTTTAAAGGACCAGAAAGAACCCAAAAATAAGACCTTTCTAAGATGCGAGG CCAAGAATTATTCTGGACGTTTCACCTGCTGGTGGCTGACGACAATCAGTACTGATT TGACATTCAGTGTCAAAAGCAGCAGAGGCTCTTCTGACCCCCAAGGGGTGACGTGC GGAGCTGCTACACTCTCTGCAGAGAGAGTCAGAGGGGACAACAAGGAGTATGAGTA CTCAGTGGAGTGCCAGGAGGACAGTGCCTGCCCAGCTGCTGAGGAGAGTCTGCCCA | 74 |
| | TTGAGGTCATGGTGGATGCCGTTCACAAGCTCAAGTATGAAAACTACACCAGCAGCT TCTTCATCAGGGACATCATCAAACCTGACCCACCCAAGAACTTGCAGCTGAAGCCAT TAAAGAATTCTCGGCAGGTGGAGGTCAGCTGGGAGTACCCTGACACCTGGAGTACT CCACATTCCTACTTCTCCCTGACATTCTGCGTTCAGGTCCAGGGCAAGAGCAAGAGA GAAAAGAAAGATAGAGTCTTCACGGACAAGACCTCAGCCACGGTCATCTGCCGCAA AAATGCCAGCATTAGCGTGCGGGCCCAGGACCGCTACTATAGCTCATCTTGGAGCG AATGGGCATCTGTGCCCTGCAGTGGCGGCGGCAGCGGCGGCGGCAGCAGA AACCTCCCCGTGGCCACTCCAGACCCAGGAATGTTCCCATGCCTTCACCACTCCCAA AACCTGCTGAGGGCCGTCAGCAACATGCTCCAGAAGGCCAGACAAACTCTAGAATT TTACCCTTGCACTTCTGAAGAGATTGATCATGAAGATATCACAAAAGATAAAACCAG CACAGTGGAGGCCTGTTTACCATTGGAATTAACCAAGAATGAGAGTTGCCTAAATTC CAGAGAGACCTCTTTCATAACTAATGGGAGTTGCCTGGCCTCCAGAAAGACCTCTTT TATGATGGCCCTGTGCCTTAGTAGTATTTATGAAGACTTGAAGATGTACCAGGTGGA GTTCAAGACCATGAATGCAAAGCTTCTGATGGATCCTAAGAGGCAGATCTTTCTAGA TCAAAACATGCTGGCAGTTATTGATGAGCTGATGCAGGCCCTGAATTTCAACAGTGA GACTGTGCCACAAAAATCCTCCCTTGAAGAACCGGATTTTTATAAAACTAAAATCAA GCTCTGCATACTTCTTCATGCTTTCAGAATTCGGGCAGTGACTATTGATAGAGTGAT GAGCTATCTGAATGCTTCC | |

## Claims

1. A tumor antigen peptide fusion protein, comprising a signal peptide, human 15-hydroxyprostaglandin dehydrogenase (HPGD), and optionally at least one tumor antigen peptide.

2. The tumor antigen peptide fusion protein according to claim 1, wherein the tumor antigen peptide fusion protein comprises, from the N-terminus to the C-terminus, any one of the polypeptide combinations selected from (i)-(iii) and linked in the following order:
(i) a signal peptide, an HPGD, and one or more tumor antigen peptides;
(ii) a signal peptide, one or more tumor antigen peptides, and an HPGD; and
(iii) a signal peptide, one or more tumor antigen peptides, an HPGD, and one or more tumor antigen peptides.

3. The tumor antigen peptide fusion protein according to claim 1 or 2, wherein the HPGD and the tumor antigen peptide, as well as two adjacent tumor antigen peptides among the multiple tumor antigen peptides, are linked via a linker peptide; and preferably, the linker peptide is selected from one or more of GG, G₂S, G₃S, (G₄S)ₙ (n ≥ 1, preferably 1, 2, 3 or 4), [A(EAAAK)ₙA]ₘ (n = 2, 3, or 4, and m = 1 or 2), (XP)ₙ (n ≥ 1, and X is any amino acid), (CW)ₙ (n ≥ 1), GFLG, PLGLWA, and RVLAEA.

4. The tumor antigen peptide fusion protein according to any one of claims 1-3, wherein the tumor antigen peptide is selected from MHC class I polypeptide-related sequence B (MICB) peptide and MHC class I polypeptide-related sequence A (MICA) peptide.

5. The tumor antigen peptide fusion protein according to claim 4, wherein the tumor antigen peptide also comprises a tumor antigen peptide selected from prostate carcinoma tumor antigen (PCTA) peptide and cancer-testis antigen (CTA) peptide.

6. The tumor antigen peptide fusion protein according to any one of claims 1-5, wherein the signal peptide comprises SEQ ID NO: 7, or an amino acid sequence having 75%, 80%, 85%, 90%, 91%, 92%, 93%, 94%, 95%, 96%, 97%, 98%, 99%, 99.2%, 99.5%, 99.8%, or more sequence identity to SEQ ID NO: 7; and/or the HPGD comprises SEQ ID NO: 1 or 2, or an amino acid sequence having 75%, 80%, 85%, 90%, 91%, 92%, 93%, 94%, 95%, 96%, 97%, 98%, 99%, 99.2%, 99.5%, 99.8%, or more sequence identity to SEQ ID NO: 1 or 2; and/or the MICB peptide comprises SEQ ID NO: 28, or an amino acid sequence having 75%, 80%, 85%, 90%, 91%, 92%, 93%, 94%, 95%, 96%, 97%, 98%, 99%, 99.2%, 99.5%, 99.8%, or more sequence identity to SEQ ID NO: 28.

7. The tumor antigen peptide fusion protein according to any one of claims 2-6, wherein the fusion protein comprises any one of the polypeptide combinations selected from the following groups (1)-(5):
(1) sHPGD: signal peptide + HPGD;
(2) sMICBH: signal peptide + MICB peptide + HPGD;
(3) sMICAH: signal peptide + MICA peptide + HPGD;
(4) smCTAH: signal peptide + MICB peptide + CTA₁ peptide + CTA₂ peptide + ... + CTA₃₃ peptide + HPGD, wherein the CTA₁ peptide, CTA₂ peptide, ..., and CTA₃₃ peptide are different; and
(5) smPCTAH: signal peptide + MICB peptide + PCTA₁ peptide + PCTA₂ peptide + ... + PCTA₁₀ peptide + HPGD, wherein the PCTA₁ peptide, PCTA₂ peptide, ..., and PCTA₁₀ peptide are the same.

8. The tumor antigen peptide fusion protein according to any one of claims 1-7, wherein the tumor antigen peptide fusion protein further comprises an IL12 p70 protein after the signal peptide at the N-terminus.

9. The tumor antigen peptide fusion protein according to any one of claims 1-8, wherein the tumor antigen peptide fusion protein comprises an amino acid sequence selected from SEQ ID NOs: 12-18, or an amino acid sequence having 75%, 80%, 85%, 90%, 91%, 92%, 93%, 94%, 95%, 96%, 97%, 98%, 99%, 99.2%, 99.5%, 99.8%, or more sequence identity to the amino acid sequence selected from SEQ ID NOs: 12-18.

10. A composition, comprising the tumor antigen peptide fusion protein according to any one of claims 1-9 and an IL12 p70 fusion protein, wherein the IL12 p70 fusion protein comprises, from the N-terminus to the C-terminus: a signal peptide or signal-anchor peptide, an IL12 p70 protein, and optionally a transmembrane peptide.

11. The composition according to claim 10, wherein the IL12 p70 protein comprises IL12 p40 and IL12 p35 linked via a linker peptide.

12. The composition according to claim 10 or 11, wherein the IL12 p70 fusion protein comprises any one of the polypeptide combinations selected from (i)-(iii):
(i) a signal peptide, IL12, a linker peptide, and IL12 linked in sequence;
(ii) a signal peptide, IL12, a linker peptide, IL12, and a transmembrane peptide linked in sequence; and
(iii) a signal-anchor peptide, IL12, a linker peptide, and IL12 linked in sequence;
wherein two IL12 in (i)-(iii) above are IL12 p35 or IL12 p40, respectively; and preferably, the linker peptide is selected from one or more of GG, G₂S, G₃S, (G₄S)ₙ (n ≥ 1, preferably 1, 2, 3 or 4), [A(EAAAK)ₙA]ₘ (n = 2, 3, or 4, and m = 1 or 2), (XP)ₙ (n ≥ 1, and X is any amino acid), (CW)ₙ (n ≥ 1), GFLG, PLGLWA, and RVLAEA.

13. The composition according to claim 12, wherein the IL12 p40 is a murine or human IL12 p40 fragment, and the IL12 p35 is a murine or human IL12 p35 fragment.

14. The composition according to any one of claims 10-12, wherein the IL12 p70 fusion protein comprises an amino acid sequence selected from SEQ ID NOs: 21-26, or an amino acid sequence having 75%, 80%, 85%, 90%, 91%, 92%, 93%, 94%, 95%, 96%, 97%, 98%, 99%, 99.2%, 99.5%, 99.8%, or more sequence identity to the amino acid sequence selected from SEQ ID NOs: 21-26.

15. A nucleic acid construct encoding the tumor antigen peptide fusion protein according to any one of claims 1-9 or the composition according to any one of claims 10-14; wherein preferably, the nucleic acid construct is a DNA fragment, mRNA, or plasmid.

16. A genetically engineered recombinant virus, comprising the nucleic acid construct according to claim 15, which is a DNA fragment.

17. The recombinant virus according to claim 16, wherein the vector of the recombinant virus is selected from a poxvirus, an adenovirus, and a herpes simplex virus; and wherein the poxvirus is selected from a vaccinia virus Western Reserve (WR) strain, Copenhagen strain, Lister strain, Ankara strain, Modified Vaccinia Ankara strain, New York Vaccinia strain, Tiantan strain, Dryvax strain, Bern strain, Paris strain, Tash Kent strain, IHD-J strain, IHD-W strain, Brighton strain, CVA382 strain, Dairen strain, LC16m8 strain, LC16M0 strain, LIVP strain, ACAM2000 strain, WR65-16 strain, Connaught strain, EM-63 strain, and derivative strains and genetically engineered strains thereof.

18. The recombinant virus according to claim 17, wherein the genetically engineered strain comprises knockout of one or more of the following gene sequences: J2R, A34R, A44L, A45R, A46R, and A52R.

19. The recombinant virus according to claim 18, wherein the genetically engineered strain comprises knockout of one or more of the gene sequences J2R, A34R, A44L-A45R-A46R, and A52R, and knock-in of exogenous genes; and preferably, the A44L-A45R-A46R gene comprises any one of the A44L, A45R, and A46R genes, or a combination of any two or all three thereof, and preferably comprises a combination of all three of the A44L, A45R, and A46R genes.

20. The recombinant virus according to claim 19, wherein the exogenous gene knock-in is tumor-targeting gene knock-in, therapeutic agent-encoding gene knock-in, and/or tumor suppressor gene knock-in.

21. The recombinant virus according to claim 20, wherein the therapeutic agent comprises one or more of a cytokine, an immune costimulatory molecule, an immune checkpoint inhibitor, an antiangiogenic factor, and a nucleic acid polymer; and wherein the cytokine is preferably one or more of chemokines, interferons (INF), interleukins (IL), and tumor necrosis factors (TNF).

22. The recombinant virus according to claim 20, wherein the tumor suppressor gene is a gene encoding HPGD, a homolog thereof, or a fusion protein thereof.

23. The recombinant virus according to claim 17, wherein the vector of the recombinant virus is an extracellular enveloped virus-enhanced vaccinia virus, which is a vaccinia virus WR strain comprising modification of the A34R gene; preferably, the modification comprises replacement of the A34R gene and/or mutation of the A34R gene, and preferably the modification comprises replacing the A34R gene of the vaccinia virus WR strain with the A34R gene of the vaccinia virus Copenhagen strain, IHD-J strain, or IHD-W strain; and preferably, the extracellular enveloped virus-enhanced vaccinia virus comprises the A34R gene of the Copenhagen strain, which encodes an amino acid sequence comprising SEQ ID NO: 19 or a variant thereof.

24. An expression system, comprising a host cell and the genetically engineered recombinant virus according to any one of claims 16-23.

25. A pharmaceutical composition, comprising the tumor antigen peptide fusion protein according to any one of claims 1-9, the composition according to any one of claims 10-14, the nucleic acid construct according to claim 15, or the genetically engineered recombinant virus according to any one of claims 16-23; and a pharmaceutically acceptable carrier.

26. The pharmaceutical composition according to claim 25, further comprising use in combination with an anticancer agent.

27. A method for treating or preventing a tumor, comprising administering the tumor antigen peptide fusion protein according to any one of claims 1-9, the composition according to any one of claims 10-14, the nucleic acid construct according to claim 15, the genetically engineered recombinant virus according to any one of claims 16-23, or the pharmaceutical composition according to claim 25 or 26.

28. Use of the tumor antigen peptide fusion protein according to any one of claims 1-9, the composition according to any one of claims 10-14, the nucleic acid construct according to claim 15, the genetically engineered recombinant virus according to any one of claims 16-23, or the pharmaceutical composition according to claim 25 or 26 in the manufacture of a medicament for treating or preventing a tumor.

29. The method according to claim 27 or the use according to claim 28, wherein the tumor is a solid tumor or a non-solid tumor; and preferably, the solid tumor is selected from the group consisting of: breast cancer, lung cancer, colon cancer, ovarian cancer, melanoma, renal cancer, liver cancer, gastric cancer, glioma, pancreatic cancer, osteosarcoma, prostate cancer, bladder cancer, rectal cancer, mesothelioma, cervical cancer, endometrial cancer, lymphoma, and nasopharyngeal carcinoma.

30. A method for activating or enhancing the function of immune cells, comprising administering the tumor antigen peptide fusion protein according to any one of claims 1-9, the composition according to any one of claims 10-14, the nucleic acid construct according to claim 15, the genetically engineered recombinant virus according to any one of claims 16-23, or the pharmaceutical composition according to claim 25 or 26.
